(19) Europäisches Patentamt
European Patent Office
Office européen des brevets

(11) EP 1 466 901 A1

# (12) EUROPEAN PATENT APPLICATION
published in accordance with Art. 158(3) EPC

(43) Date of publication:
**13.10.2004 Bulletin 2004/42**

(21) Application number: **02790747.6**

(22) Date of filing: **12.12.2002**

(51) Int Cl.[7]: **C07D 201/00**

(86) International application number:
**PCT/JP2002/013043**

(87) International publication number:
**WO 2003/053928 (03.07.2003 Gazette 2003/27)**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **13.12.2001 JP 2001379598**
**28.12.2001 JP 2001399453**
**16.01.2002 JP 2002007140**

(71) Applicant: **Sumitomo Pharmaceuticals Company, Limited**
**Osaka-shi, Osaka 541-8510 (JP)**

(72) Inventors:
- **KODO, Toru**
**Takarazuka-shi, Hyogo 665-0056 (JP)**
- **YAGI, Hideki**
**Nishinomiya-shi, Hyogo 662-0831 (JP)**
- **DAN, Akihito**
**Funabashi-shi, Chiba 273-0039 (JP)**
- **MASUMOTO, Shuji**
**Bunkyo-ku, Tokyo 113-0034 (JP)**
- **KINOMURA, Naoya**
**Nada-ku, Kobe-shi, Hyogo 657-0024 (JP)**
- **KOYAMA, Koji**
**Nishinomiya-shi, Osaka 561-0802 (JP)**

(74) Representative: **Cresswell, Thomas Anthony**
**J.A. KEMP & CO.**
**14 South Square**
**Gray's Inn**
**London WC1R 5JJ (GB)**

## (54) SEROTONINE REUPTAKE INHIBITOR

(57) There is provided a selective serotonin reuptake inhibitor having affinity for serotonin 1A receptors which comprises a cyclic amine represented by the formula:

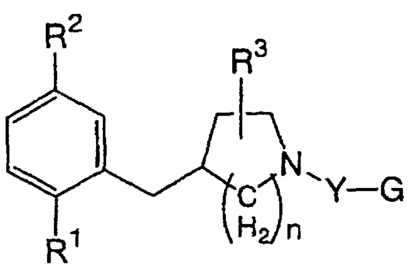

(1)

wherein G represents a formula (2):



Printed by Jouve, 75001 PARIS (FR)

(Cont. next page)

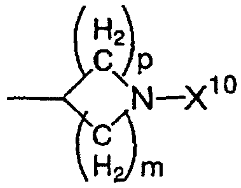

(a) 、 $-Z^2-X^{20}$ (b) 、 or $-Z^3$ (c) (2)

a prodrug of said cyclic amine, or a pharmaceutically acceptable salt of said cyclic amine or prodrug, as an active ingredient.

## Description

### TECHNICAL FIELD

The present invention relates to a serotonin reuptake inhibitor. The serotonin reuptake inhibitor of the present invention is a selective serotonin reuptake inhibitor that has affinity for serotonin 1A receptors, has a small inhibitory effect on the reuptake of dopamine and noradrenaline, and strongly inhibits serotonin reuptake. In addition, the present invention relates to novel compounds having such a selective inhibitory effect on serotonin reuptake. Furthermore, the present invention relates to novel benzylpiperidine derivatives that are intermediates for synthesizing such compounds, and a process for the production of the derivatives.

### BACKGROUND ART

Depression is a chronic disease that affects persons of all ages. Of various antidepressants used at present, the most successful antidepressants are selective serotonin reuptake inhibitors (hereinafter abbreviated as SSRI in some cases). SSRI has serotonin reuptake inhibitory effect more selective than dopamine and noradrenaline reuptake inhibitory effect. The first drug put on the market as SSRI is zimelidine. Other SSRIs that have been put on the market or are under development include, for example, fluoxetine, fluvoxamine, citalopram, cericlamine, femoxetine, ifoxetine, cyanodothiepin, sertraline, paroxetine and litoxetine.

Although SSRIs are the most successful as antidepressants at present, several problems in them are pointed out. Of these problems, the following two problems are typical: about one-third of all melancholiacs are so difficult to cure that SSRIs are not sufficiently effective, and the exhibition of a sufficient clinical effect of SSRI requires 3 to 8 weeks. In particular, this slow exhibition of antidepressant effect causes the following problems. Since SSRIs exhibit side effects immediately though they exhibit antidepressant effect slowly, there is a vulnerable period in which the patient undergoes only the side effects without obtaining the therapeutic effect of the drug. Therefore, persuading the patient into continuing the same treatment also in this period is often a heavy burden for a doctor in charge. In addition, because of the gradual beginning of experience of the effect, a patient who tends to commit suicide resumes initiative before experiencing sufficient improvement of depressive condition. Therefore, there are, for example, a risk of suicide and a necessity for frequent admission into a hospital. Accordingly, the development of an antidepressant capable of exhibiting its effect rapidly is desired.

The reason why the exhibition of the antidepressant effect of SSRI requires several weeks is as follows.

SSRI inhibits rapid serotonin reuptake in serotonin metabolic turnover. Since this action takes place at the nerve ending of each serotonergic neuron, neurotransmission due to serotonin is enhanced. The inhibition of rapid serotonin reuptake by SSRI, however, takes place also in serotonergic neuron cell bodies and dendrites, which are present in raphe nucleus. Therefore, the firing auto-inhibition (the negative feedback reaction) of the serotonergic neuron through 5-HT1A auto-receptor is also enhanced in the raphe nucleus. As a result, the neurotransmission in the serotonergic neuron is not enhanced to an expected degree as a whole by initial administration of SSRI. On the other hand, in the course of continuous taking of SSRI for several weeks, serotonin 1A auto-receptors present on serotonergic neuronal cell bodies and dendrites in the raphe nucleus are desensitized, so that the negative feed back reaction is abolished. As a result, the firing inhibition of the serotonergic neuron is broken down, so that the enhancement of the activity of the serotonergic neuron and the inhibition of serotonin uptake at the nerve ending succeed cooperatively. Therefore, the serotonin neurotransmission is enhanced, resulting in exhibition of the antidepressant effect.

Accordingly, the reduction of a period required for the exhibition of the effect of SSRI or the enhancement of the antidepressant effect may be achieved either by stopping the negative feedback reaction of serotonin by inhibiting the serotonin 1A auto-receptor by the use of a serotonin 1A receptor antagonist, or by reducing a period required for the desensitization by positively stimulating the serotonin 1A auto-receptor by the use of a serotonin 1A receptor agonist. That is, a compound having affinity for serotonin 1A receptors and a selective inhibitory effect on serotonin reuptake has a marked antidepressant effect and hence may be used as a therapeutic agent for psychiatric diseases which exhibits its effect after a reduced period. In fact, it has been reported that, for example, pindolol having a high affinity for serotonin 1A receptors increases the effect of a serotonin reuptake inhibitor in a melancholiac and reduces a period required for the onset of the effect (Arch, Gen. Psychiatry, (1994), 51, 248-251).

There are many references concerning derivatives containing two nitrogen-containing saturated heterocyclic rings represented by a piperidine ring in the molecule. Among these derivatives, as bispiperidine derivatives having two piperidine rings bonded to each other through an alkylene chain at the 4-potision and 1-position, there have been disclosed, for example, benzoylpiperidine derivatives as therapeutic agents for Parkinson's disease in GB1250719. In addition, EP512901 discloses 1-aralkyl-4-aryl-4-piperidinoalkylpiperidine derivatives as substance P antagonists and neurokinin. WO97/09308 discloses indole derivatives as neuropeptide Y antagonists. WO98/27085 discloses cyclic carboxyamide derivatives as tachykinin receptors antagonists. WO2000/023438 discloses imidazolylalkyl-piperidine

derivatives as H3 agonists. WO2000/035877 discloses heterocyclic piperidine derivatives as chemokine receptor regulators. Further, DE19908483 discloses pyridine derivatives as inhibitors of nicotinamide mononucleotide production. None of these prior art references, however, report that the compounds disclosed therein have inhibitory effect on serotonin reuptake. In addition, JP-A-2001-131149 discloses bispiperidine derivatives having inhibitory effect on serotonin reuptake, but it reports that these derivatives should have two other ring structures besides piperidine rings.

As to cycloalkyl derivatives having a 4-benzylpiperidinoalkyl group as a substituent, FR7031, for example, discloses phenylcyclohexane derivatives having anticonvulsant and analgesic effects. In addition, DE3614907 discloses N-substituted piperidine derivatives as fungicides. WO9418172 discloses imidazolylbenzene derivatives as NMDA antagonists. WO00/35449 and WO00/35452 disclose piperidine derivatives as chemokine receptor regulators. None of these prior art references, however, report that the compounds disclosed therein have inhibitory effect on serotonin reuptake.

Furthermore, as to non-aromatic heterocyclic derivatives having a 4-benzylpiperidinoalkyl group as a substituent on the carbon atom of the derivative, DE19531321, for example, discloses N-phenyl-oxazolidinone derivatives having effects on central nervous system. In addition, DE4037426 discloses benzoxazione derivatives as anti-inflammatories. None of these prior art references, however, report that the compounds disclosed therein have inhibitory effect on serotonin reuptake.

DISCLOSURE OF THE INVENTION

The present invention relates to the following items (1) to (40).

(1) A serotonin reuptake inhibitor comprising a cyclic amine represented by the formula:

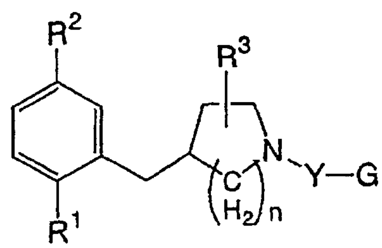

(1)

wherein G represents a formula (2):

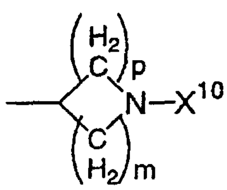

(a) 、 $-Z^2-X^{20}$ (b) 、 $-Z^3$ (c) (2)

wherein

$R^1$ is a halogen atom or an alkyl group;
$R^2$ is a hydrogen atom, a halogen atom, a hydroxyl group, an alkyl group, a substituted alkyl group, an alkoxy group, a substituted alkoxy group, an alkylthio group or a substituted alkylthio group;
$R^3$ is a hydrogen atom or a lower alkyl group;
Y is a substituted or unsubstituted alkylene group;
n is an integer of 1, 2 or 3;
m is an integer of 0, 1, 2 or 3;
p is an integer of 1, 2, 3 or 4;
$X^{10}$ is a hydrogen atom, an alkyl group, a substituted alkyl group, a cycloalkyl group, an alkanoyl group, a

substituted alkanoyl group, an alkanesulfonyl group, a substituted alkanesulfonyl group, a carbamoyl group, an alkylcarbamoyl group, a substituted alkylcarbamoyl group, an alkylsulfamoyl group, a substituted alkylsulfamoyl group, an alkoxycarbonyl group, a substituted alkoxycarbonyl group, an amidino group or a substituted amidino group;
$X^{20}$ is an alkyl group, a substituted alkyl group, an amino group, a substituted amino group, a hydroxyl group, an alkoxy group, a substituted alkoxy group, a carbamoyloxy group, an alkylcarbamoyloxy group or a substituted alkylcarbamoyloxy group;
$Z^2$ is a cycloalkane ring;
$Z^3$ represents a formula:

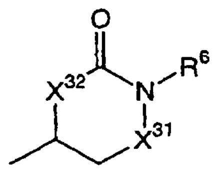

(A)

wherein

$X^{31}$ is a bond, a methylene group or a carbonyl group;
$X^{32}$ is an oxygen atom, a sulfur atom or a group: $-N(R^7)-$;
$R^6$ is a hydrogen atom, an alkyl group, a substituted alkyl group, a cycloalkyl group, a substituted cycloalkyl group, an aryl group, a substituted aryl group, a heteroaryl group or a substituted heteroaryl group;
$R^7$ is a hydrogen atom or an alkyl group, or a formula:

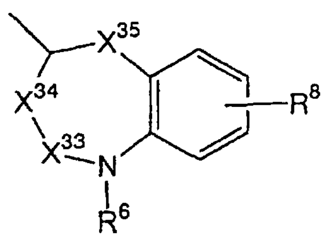

(B)

wherein

$X^{33}$ is a bond, a methylene group or a carbonyl group;
$X^{34}$ is a bond or a methylene group, and $X^{35}$ is a bond, a methylene group, an oxygen atom, a sulfur atom or a group: $-N(R^7)-$, provided that $X^{34}$ and $X^{35}$ are not bonds at the same time;
$R^6$ and $R^7$ are as defined above; and
$R^8$ is a hydrogen atom, a halogen atom, an alkyl group, a hydroxyl group, an alkoxy group, a substituted alkoxy group, a carbamoyloxy group, an alkylcarbamoyloxy group or a substituted alkylcarbamoyloxy group,

a prodrug of said cyclic amine, or a pharmaceutically acceptable salt of said cyclic amine or prodrug, as an active ingredient.
(2) A serotonin reuptake inhibitor according to the above item (1), wherein G represents a formula:

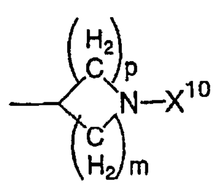

(3) A serotonin reuptake inhibitor according to the above item (2), wherein the alkylene group for Y is an ethylene

group or a trimethylene group.
(4) A serotonin reuptake inhibitor according to the above item (2) or (3), wherein n is 1 or 2.
(5) A serotonin reuptake inhibitor according to the above item (2), (3) or (4), wherein each of p and m is 2.
(6) A serotonin reuptake inhibitor according to the above item (2), (3) or (4), wherein one of p and m is 1 and the other is 3.
(7) A serotonin reuptake inhibitor according to any one of the above items (2) to (6), wherein $X^{10}$ is a hydrogen atom, a lower alkyl group, a hydroxyalkyl group of 4 or less carbon atoms, an alkanoyl group, a hydroxy-substituted alkanoyl group, an alkanesulfonyl group or an alkylsulfamoyl group.
(8) A cyclic amine represented by the formula:

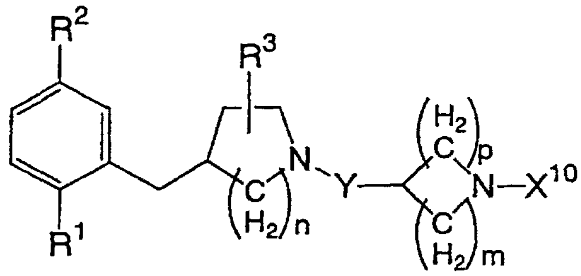

wherein

$R^1$ is a halogen atom or an alkyl group;
$R^2$ is a hydrogen atom, a halogen atom, a hydroxyl group, an alkyl group, a substituted alkyl group, an alkoxy group, a substituted alkoxy group, an alkylthio group or a substituted alkylthio group;
$R^3$ is a hydrogen atom or a lower alkyl group;
Y is a substituted or unsubstituted alkylene group;
$X^{10}$ is a hydrogen atom, an alkyl group, a substituted alkyl group, a cycloalkyl group, an alkanoyl group, a substituted alkanoyl group, an alkanesulfonyl group, a substituted alkanesulfonyl group, a carbamoyl group, an alkylcarbamoyl group, a substituted alkylcarbamoyl group, an alkylsulfamoyl group, a substituted alkylsulfamoyl group, an alkoxycarbonyl group, a substituted alkoxycarbonyl group, an amidino group or a substituted amidino group;
n is an integer of 1, 2 or 3;
m is an integer of 0, 1, 2 or 3; and
p is an integer of 1, 2, 3 or 4,

a prodrug of said cyclic amine, or a pharmaceutically acceptable salt or solvate of said cyclic amine or prodrug.
(9) A compound according to the above item (8), wherein $R^2$ is a hydroxyl group, a lower alkoxy group, a halogen-substituted alkoxy group or a halogen atom, or $R^2$ is a hydrogen atom only in the case of $R^1$ being a bromine atom; and Y is a substituted or unsubstituted alkylene group of 2 or 3 carbon atoms.
(10) A compound according to the above item (8) or (9), wherein n is 1 or 2.
(11) A compound according to any one of the above items (8) to (10), wherein Y is an unsubstituted alkylene group.
(12) A compound according to any one of the above items (8) to (11), wherein each of p and m is 2.
(13) A compound according to any one of the above items (8) to (12), wherein $R^1$ is a bromine atom, a chlorine atom, a methyl group or an ethyl group.
(14) A compound according to any one of the above items (8) to (13), wherein $R^2$ is a lower alkoxy group or a halogen atom.
(15) A compound according to any one of the above items (8) to (14), wherein $X^{10}$ is a hydrogen atom, a lower alkyl group, a hydroxyalkyl group of 4 or less carbon atoms, an alkanoyl group, a hydroxy-substituted alkanoyl group, an alkanesulfonyl group or an alkylsulfamoyl group.
(16) A serotonin reuptake inhibitor according to the above item (1), wherein G represents a formula:

$$-Z^2-X^{20}$$

(17) A serotonin reuptake inhibitor according to the above item (16), wherein the alkylene group for Y is an ethylene group or a trimethylene group.
(18) A serotonin reuptake inhibitor according to the above item (16) or (17), wherein n is 1 or 2.
(19) A serotonin reuptake inhibitor according to the above item (16), (17) or (18), wherein the cycloalkane ring is a cyclohexane ring.
(20) A serotonin reuptake inhibitor according to the above item (19), wherein the substitution positions of the cyclohexane ring are the 1-position and the 4-position.
(21) A serotonin reuptake inhibitor according to any one of the above items (16) to (20), wherein X is an amino group, a substituted amino group, a hydroxyl group, an alkoxy group or a carbamoyloxy group, and the substituent of the aforesaid substituted amino group is a lower alkyl group, a hydroxyalkyl group of 4 or less carbon atoms, an alkanoyl group, a hydroxyalkanoyl group, an alkanesulfonyl group or an alkylsulfamoyl group.
(22) A cyclic amine represented by the formula:

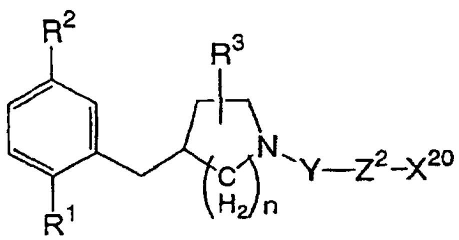

wherein

$R^1$ is a halogen atom or an alkyl group;
$R^2$ is a hydrogen atom, a halogen atom, a hydroxyl group, an alkoxy group, a substituted alkoxy group, an alkylthio group or a substituted alkylthio group;
$R^3$ is a hydrogen atom or a lower alkyl group;
$Z^2$ is a cycloalkane ring;
$X^{20}$ is an alkyl group, a substituted alkyl group, an amino group, a substituted amino group, a hydroxyl group, an alkoxy group, a substituted alkoxy group, a carbamoyloxy group, an alkylcarbamoyloxy group or a substituted alkylcarbamoyloxy group;
Y is a substituted or unsubstituted alkylene group; and
n is an integer of 1, 2 or 3,

a prodrug of said cyclic amine, or a pharmaceutically acceptable salt of said cyclic amine or prodrug.
(23) A compound according to the above item (22), wherein $R^2$ is a hydroxyl group, a lower alkoxy group, a halogen-substituted alkoxy group or a halogen atom, or $R^2$ is a hydrogen atom only in the case of $R^1$ being a bromine atom; and Y is a substituted or unsubstituted alkylene group of 2 or 3 carbon atoms.
(24) A compound according to the above item (22) or (23), wherein n is 1 or 2.
(25) A compound according to any one of the above items (22) to (24), wherein Y is an unsubstituted alkylene group.
(26) A compound according to any one of the above items (22) to (25), wherein Z is a cyclohexane ring.
(27) A compound according to the above item (26), wherein the substitution positions of the cyclohexane ring are the 1-position and the 4-position.
(28) A compound according to any one of the above items (22) to (27), wherein $R^1$ is a bromine atom, a chlorine atom, a methyl group or an ethyl group.
(29) A compound according to any one of the above items (22) to (28), wherein $R^2$ is a lower alkoxy group or a halogen atom.
(30) A compound according to any one of the above items (22) to (29), wherein X is an amino group, a substituted amino group, a hydroxyl group, an alkoxy group or a carbamoyloxy group, and the substituent of the aforesaid substituted amino group is a lower alkyl group, a hydroxyalkyl group of 4 or less carbon atoms, an alkanoyl group, a hydroxyalkanoyl group, an alkanesulfonyl group, an alkylsulfamoyl group or a substituted alkylsulfamoyl group.
(31) A serotonin reuptake inhibitor according to the above item (1), wherein G represents a formula:

$$-Z^3$$

(32) A serotonin reuptake inhibitor according to the above item (31), wherein the alkylene group for Y is a methylene group, an ethylene group or a trimethylene group.
(33) A serotonin reuptake inhibitor according to the above item (31) or (32), wherein n is 1 or 2.
(34) A serotonin reuptake inhibitor according to the above item (31), (32) or (33), wherein $R^2$ is a halogen atom or an alkoxy group.
(35) A cyclic amine represented by the formula:

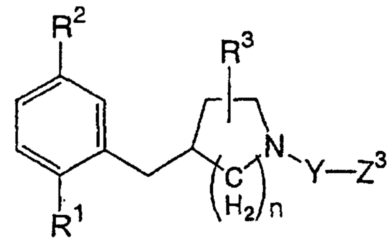

wherein

$R^1$ is a halogen atom or an alkyl group;
$R^2$ is a hydrogen atom, a halogen atom, a hydroxyl group, an alkyl group, a substituted alkyl group, an alkoxy group, a substituted alkoxy group, an alkylthio group or a substituted alkylthio group;
$R^3$ is a hydrogen atom or a lower alkyl group;
Y is a substituted or unsubstituted alkylene group;
$Z^3$ represents a formula:

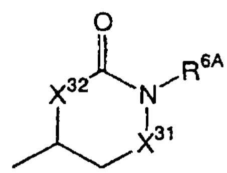

(A')

wherein

$X^{31}$ is a bond, a methylene group or a carbonyl group;
$X^{32}$ is an oxygen atom, a sulfur atom or a group: $-N(R^7)-$;
$R^{6A}$ is a hydrogen atom, an alkyl group, a substituted alkyl group, a cycloalkyl group, a substituted cycloalkyl group, a heteroaryl group or a substituted heteroaryl group;
$R^7$ is a hydrogen atom or an alkyl group, or a formula:

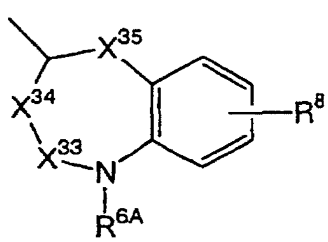

(B')

wherein

$X^{33}$ is a bond, a methylene group or a carbonyl group;
$X^{34}$ is a bond or a methylene group, and $X^{35}$ is a bond, a methylene group, an oxygen atom, a sulfur atom or

a group: $-N(R^7)-$, provided that $X^{34}$ and $X^{35}$ are not bonds at the same time;
$R^{6A}$ and $R^7$ are as defined above;
$R^8$ is a hydrogen atom, a halogen atom, an alkyl group, a hydroxyl group, an alkoxy group, a substituted alkoxy group, a carbamoyloxy group, an alkylcarbamoyloxy group or a substituted alkylcarbamoyloxy group; and
n is an integer of 1, 2 or 3,

a prodrug of said cyclic amine, or a pharmaceutically acceptable salt of said cyclic amine or prodrug.
(36) A compound according to the above item (35), wherein the alkylene group for Y is a methylene group, an ethylene group or a trimethylene group.
(37) A compound according to the above item (35) or (36), wherein n is 1 or 2.
(38) A compound according to the above item (35), (36) or (37), wherein $R^2$ is a halogen atom or an alkoxy group.

BEST MODE FOR CARRYING OUT THE INVENTION

The present invention is more concretely explained below.
The halogen atom includes, for example, bromine atom, chlorine atom and fluorine atom.
The alkyl group includes, for example, alkyl groups of 10 or less carbon atom(s), such as methyl, ethyl, propyl, isopropyl, butyl, isobutyl, sec-butyl, tert-butyl, pentyl, 1-methylbutyl, 2-methylbutyl, 3-methylbutyl, 1-ethylpropyl, hexyl, heptyl, octyl, etc. Preferable examples of the alkyl group are the lower alkyl groups described below. More preferable examples thereof are alkyl groups of 4 or less carbon atom(s), such as methyl, ethyl, propyl, isopropyl, butyl, etc.
The substituent(s) of the substituted alkyl group include(s), for example, halogen atoms (which may be present on the same carbon atom in a number of 1 to 3 as the substituent(s)), hydroxyl group, alkoxy groups, amino group, alkylamino groups, dialkylamino groups, acylamino groups, phenyl group and substituted phenyl groups.
The lower alkyl group includes, for example, alkyl groups of 6 or less carbon atom(s), such as methyl, ethyl, propyl, isopropyl, butyl, isobutyl, sec-butyl, tert-butyl, pentyl, 1-methylbutyl, 2-methylbutyl, 3-methylbutyl, 1-ethylpropyl, hexyl, etc. Preferable examples of the lower alkyl group are methyl, ethyl, propyl and isopropyl.
The cycloalkyl group includes, for example, cycloalkyl groups of 3 to 8 carbon atoms, such as cyclopentyl, cyclohexyl, etc.
The substituent(s) of the substituted cycloalkyl group include(s), for example, hydroxyl group, alkoxy groups, amino group, alkylamino groups, dialkylamino groups and acylamino groups.
The alkoxy group includes, for example, alkoxy groups of 10 or less carbon atom(s), such as methoxy, ethoxy, propoxy, butoxy, isopropoxy, isobutoxy, sec-butoxy, tert-butoxy, pentoxy, hexoxy, heptoxy, octoxy, etc. Preferable examples of the alkoxy group are methoxy, ethoxy and isopropoxy.
The lower alkoxy group includes, for example, alkoxy groups of 6 or less carbon atom(s), such as methoxy, ethoxy, propoxy, butoxy, isopropoxy, isobutoxy, sec-butoxy, tert-butoxy, pentoxy, hexoxy, etc. Preferable examples of the lower alkoxy group are methoxy, ethoxy and isopropoxy.
The alkylthio group includes, for example, alkylthio groups of 10 or less carbon atom(s), such as methylthio, ethylthio, propylthio, butylthio, isopropylthio, isobutylthio, sec-butylthio, tert-butylthio, pentylthio, hexylthio, heptylthio, octylthio, etc.
The substituent(s) of each of the substituted alkoxy group and the substituted alkylthio group include(s), for example, halogen atoms (which may be present on the same carbon atom in a number of 1 to 3 as the substituent(s)), hydroxyl group, alkoxy groups, aryl groups and substituted aryl groups.
The acyl group includes, for example, alkanoyl groups, substituted alkanoyl groups, aroyl groups and substituted aroyl groups.
The alkanoyl groups include, for example, alkanoyl groups of 7 or less carbon atoms, such as acetyl, propionyl, etc.
The substituent(s) of the substituted alkanoyl group include(s), for example, halogen atoms (which may be present on the same carbon atom in a number of 1 to 3 as the substituent(s)), hydroxyl group, alkoxy groups, cycloalkyl groups, alkoxycarbonyl groups, alkylamino groups and dialkylamino groups.
The aroyl groups include, for examples, aroyl groups of 11 or less carbon atoms, such as benzoyl, toluoyl, naphthoyl, etc.
The substituent(s) of the substituted aroyl group include(s), for example, halogen atoms, hydroxyl group and lower alkoxy groups.
The alkanesulfonyl group includes, for example, alkanesulfonyl groups of 6 or less carbon atom(s), such as methanesulfonyl group, ethanesulfonyl group, etc.
The substituent(s) of the substituted alkanesulfonyl group include(s), for example, halogen atoms (which may be present on the same carbon atom in a number of 1 to 3 as the substituent(s)), hydroxyl group and alkoxy groups.
The alkylcarbamoyl group includes, for example, monoalkylcarbamoyl and dialkylcarbamoyl groups of 18 or

less carbon atoms, such as monoethylcarbamoyl group, dimethylcarbamoyl group, etc. The two alkyl groups of the dialkylcarbamoyl group may bind to each other to form a saturated 5- or 6-membered ring together with the nitrogen atom to which they are bonded, as in pyrrolidinecarbonyl group.

The substituent(s) of the substituted alkylcarbamoyl group include(s), for example, halogen atoms (which may be present on the same carbon atom in a number of 1 to 3 as the substituent(s)), hydroxyl group and alkoxy groups. The two alkyl groups of the substituted alkylcarbamoyl group may form a ring through a heteroatom as in morpholinecarbonyl group.

The alkylsulfamoyl group includes, for example, monoalkylsulfamoyl and dialkylsulfamoyl groups of 18 or less carbon atom(s), such as monomethylsulfamoyl group, dimethylsulfamoyl group, etc. The two alkyl groups of the dialkylsulfamoyl group may bind to each other to form a saturated 5- or 6-membered ring together with the nitrogen atom to which they are bonded, as in pyrrolidinesulfamoyl group.

The substituent(s) of the substituted alkylsulfamoyl group include(s), for example, halogen atoms (which may be present on the same carbon atom in a number of 1 to 3 as the substituent(s)), hydroxyl group, alkoxy groups and phenyl group. The two alkyl groups of the substituted alkylsulfamoyl group may form a ring through a heteroatom as in morpholinesulfamoyl group.

The alkoxycarbonyl group includes, for example, alkoxycarbonyl groups of 6 or less carbon atoms, such as methoxycarbonyl group, ethoxycarbonyl group, etc.

The substituent(s) of the substituted alkoxycarbonyl group include(s), for example, halogen atoms (which may be present on the same carbon atom in a number of 1 to 3 as the substituent(s)), hydroxyl group and alkoxy groups.

The substituent(s) of the substituted amidino group include(s), for example, lower alkyl groups, acyl groups and alkoxycarbonyl groups.

The cycloalkane ring for $Z^2$ includes, for example, cycloalkane rings having 3 to 8 carbon atoms, such as cyclopentane, cyclohexane, cycloheptane, etc.

The substituted amino group may have one substituent or two substituents which may be the same or different. Its substituent(s) include(s), for example, the above-exemplified alkyl groups, substituted alkyl groups, acyl groups, alkanesulfonyl groups, substituted alkanesulfonyl groups, carbamoyl groups, alkylcarbamoyl groups, substituted alkylcarbamoyl groups, alkylsulfamoyl groups, substituted alkylsulfamoyl groups, alkoxycarbonyl groups and substituted alkoxycarbonyl groups.

The alkylcarbamoyloxy group includes, for example, monoalkyl- and dialkylcarbamoyloxy groups of 12 or less carbon atoms, such as monoethylcarbamoyloxy group, dimethylcarbamoyloxy group, etc. The two alkyl groups of the dialkylcarbamoyloxy group may form a ring as in pyrrolidinecarbamoyloxy group.

The substituent(s) of the substituted alkylcarbamoyloxy group include(s), for example, halogen atoms (which may be present on the same carbon atom in a number of 1 to 3 as the substituent(s)), hydroxyl group and alkoxy groups. The two alkyl groups of the substituted alkylcarbamoyloxy group may form a ring through a heteroatom as in morpholinecarboxy group.

The aryl group includes, for example, aryl groups of 10 or less carbon atoms, such as phenyl, tolyl, naphthyl, etc.

The heteroaryl group includes, for example, 5- or 6-membered aromatic heterocyclic groups containing 1 to 3 heteroatoms selected from the group consisting of nitrogen atom, sulfur atom and oxygen atom. More specific examples thereof are pyridyl (whose nitrogen atom may be an oxidized one), thienyl, furanyl, pyrrolyl, pyrazolyl, imidazolyl, pyrazyl, pyrimidyl, pyridazyl, oxazolyl, thiazolyl, oxadiazolyl, triazolyl and tetrazolyl.

The substituent(s) of each of the substituted aryl group and the substituted heteroaryl group include(s), for example, halogen atoms, hydroxyl group and lower alkoxy groups.

Examples of the alkyl portion of each of the alkylamino group and the dialkylamino group are the same as those of the above-mentioned lower alkyl group.

Examples of the acyl portion of the acylamino group are the same as those of the above-mentioned acyl group.

The substituent(s) of the substituted phenyl group include(s), for example, halogen atoms, hydroxyl group and lower alkoxy groups.

The alkylene group includes, for example, linear or branched alkylene groups of 10 or less carbon atom(s).

The substituent(s) of the substituted alkylene group include(s), for example, lower alkyl groups, hydroxyl group, acyloxy groups, alkoxy groups and halogen atoms.

The substituted or unsubstituted alkylene group includes, for example, groups represented by the formula:

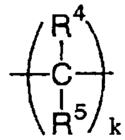

wherein k is an integer of 2, 3, 4, 5 or 6.

In the above formula, $R^4$s and $R^5$s are independently, for example, a hydrogen atom, a lower alkyl group, a hydroxyl group, an acyloxy group, an alkoxy group or a halogen atom. Alternatively, $R^4$ and $R^5$ may bind to each other to form a 3- to 8-membered cycloalkane ring together with the carbon atom to which they are bonded. The 3- to 8-membered cycloalkane ring includes, for example, cyclopentane ring and cyclohexane ting.

The acyloxy groups include, for example, alkanoyloxy groups, substituted alkanoyloxy groups and benzoyloxy group.

The alkanoyloxy groups include, for example, alkanoyloxy groups of 7 or less carbon atoms, such as acetyloxy, propionyloxy, etc.

The substituent(s) of the substituted alkanoyloxy group include(s), for example, halogen atoms (which may be present on the same carbon atom in a number of 1 to 3 as the substituent(s)), hydroxyl group and alkoxy groups.

The group represented by the above formula (A) or (B) includes, for example, groups represented by the following formulas:

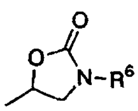
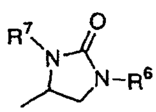
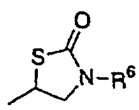
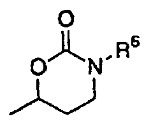
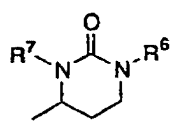

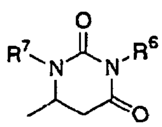
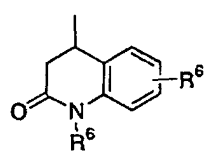
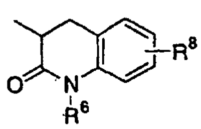
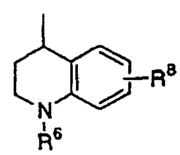

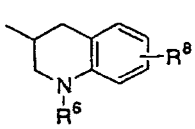
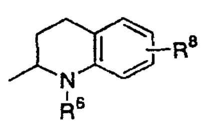
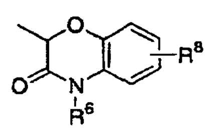
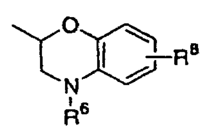

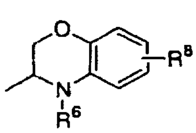
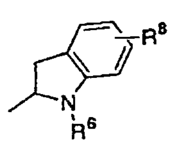
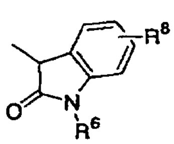
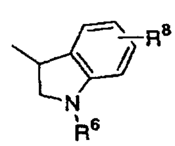

wherein $R^6$, $R^7$ and $R^8$ are as defined above.

The term "prodrug" used herein means a derivative that is decomposed in a living body by acid hydrolysis or enzymatically to give a compound of the above formula (1). For example, when the compound of the formula (1) has a hydroxyl group or an amino group, the prodrug may be produced by modifying such a group according to a conventional method.

The starting compounds (3) to (20) described hereinafter, some of which are novel, may be produced by the

processes described in the working examples described hereinafter, processes similar to these processes, or conventional processes.

A desired compound (1) or a salt thereof may be produced by any of the processes shown in the following schemes.

Production process 1 (alkylation of an amino group)

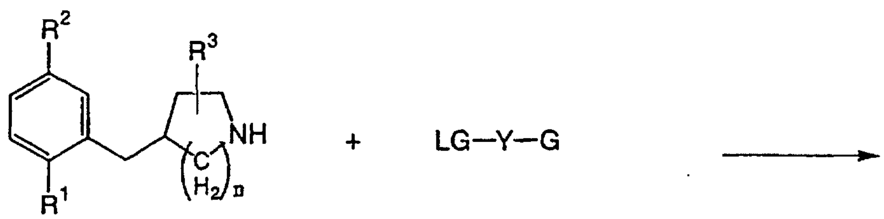

(3) (4)

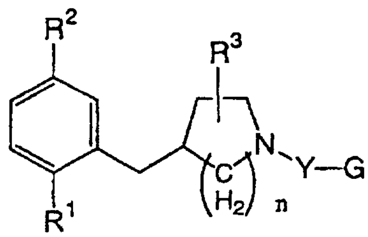

(1)

wherein $R^1$, $R^2$, $R^3$, Y, G and n are as defined above, and LG is a leaving group.

The leaving group LG includes, for example, halogen atoms such as chlorine atom, bromine atom, iodine atom, etc.; and acyloxy groups such as acetoxy, tosyloxy, mesyloxy, etc.

A desired compound (1) or a salt thereof may be obtained by reacting a compound (3) or a salt thereof with a compound (4) or a salt thereof. The reaction may be carried out for 10 minutes to 48 hours in a suitable inert solvent at a temperature in a range of about -20°C to the boiling point of the solvent used, optionally in the presence of a base and optionally in the presence of a phase transfer catalyst.

The base includes, for example, organic bases such as triethylamine, pyridine, etc.; inorganic bases such as potassium carbonate, sodium hydroxide, sodium hydride, etc.; and metal alkoxides such as sodium methoxide, potassium tert-butoxide, etc.

The phase transfer catalyst includes, for example, tetrabutylammonium hydrogensulfate.

The inert solvent includes, for example, acetonitrile; halogenated hydrocarbons such as chloroform, dichloromethane, etc.; aromatic hydrocarbons such as benzene, toluene, etc.; ether solvents such as diethyl ether, tetrahydrofuran (THF), 1,4-dioxane, etc.; lower alcohols such as methanol, ethanol, isopropanol, etc.; aprotic polar solvents such as dimethylformamide, N-methylpyrrolidone, dimethyl sulfoxide, etc.; and mixed solvents thereof.

<u>Production process 2 (reduction of an amide: when Y is a group represented by $-CH_2-Y^1$)</u>

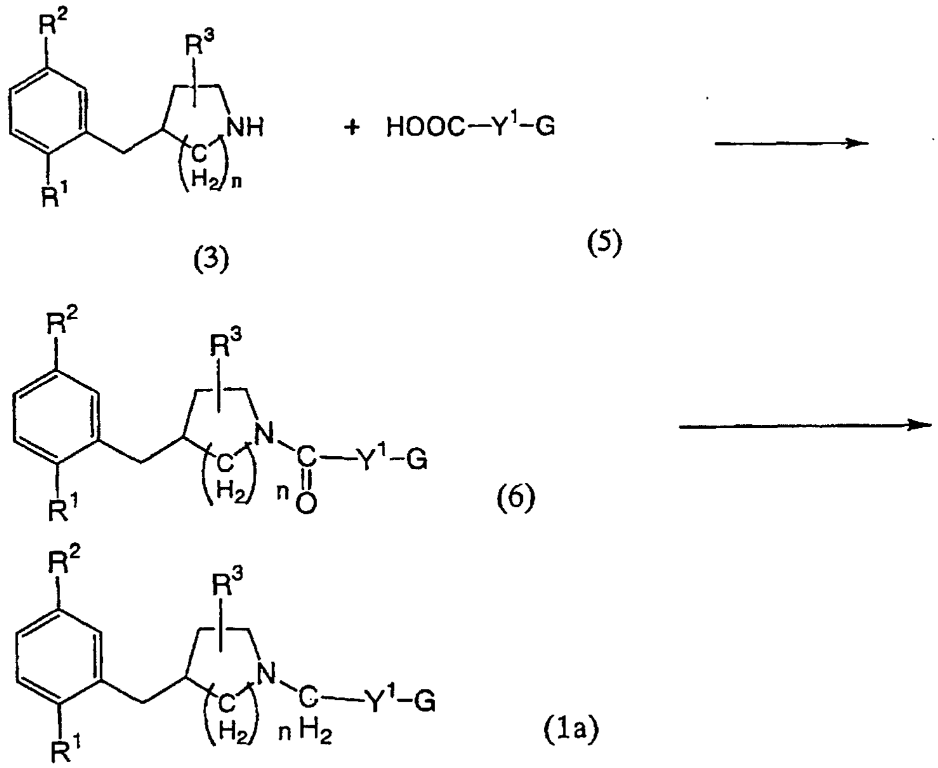

(3)

(5)

(6)

(1a)

wherein $R^1$, $R^2$, $R^3$, G and n are as defined above, $Y^1$ is an alkylene group or a substituted alkylene group, and $-CH_2-Y^1-$ corresponds to the above symbol Y.

An intermediate (6) may be produced by reacting a compound (3) or a salt thereof with a compound (5) or a salt thereof to form an amide linkage. This amide linkage formation reaction may be carried out by adopting a conventional method such as an acid chloride method using thionyl chloride, oxalyl chloride or the like; a mixed acid anhydride method using a chlorocarbonic acid ester or the like; or a method using a condensing agent such as dicyclohexylcarbodiimide, carbonyldiimidazole or the like.

A compound (1a) may be obtained by reacting the intermediate (6) for 10 minutes to 48 hours by the use of a suitable reducing agent (e.g. lithium aluminum hydride, sodium borohydride or diborane, etc.) in a suitable inert solvent (e.g. an ether solvent such as diethyl ether, tetrahydrofuran (THF) or 1,4-dioxane, etc.) at a temperature in a range of -20°C to the boiling point of the solvent used. More specifically, the compound (1a) may be obtained by carrying out the reduction for 20 minutes to 1 hour by the use of diborane in tetrahydrofuran (THF) under ice-cooling or at room temperature.

Production process 3 (reductive amination: when Y is a group represented by $-CH_2-Y^1-$)

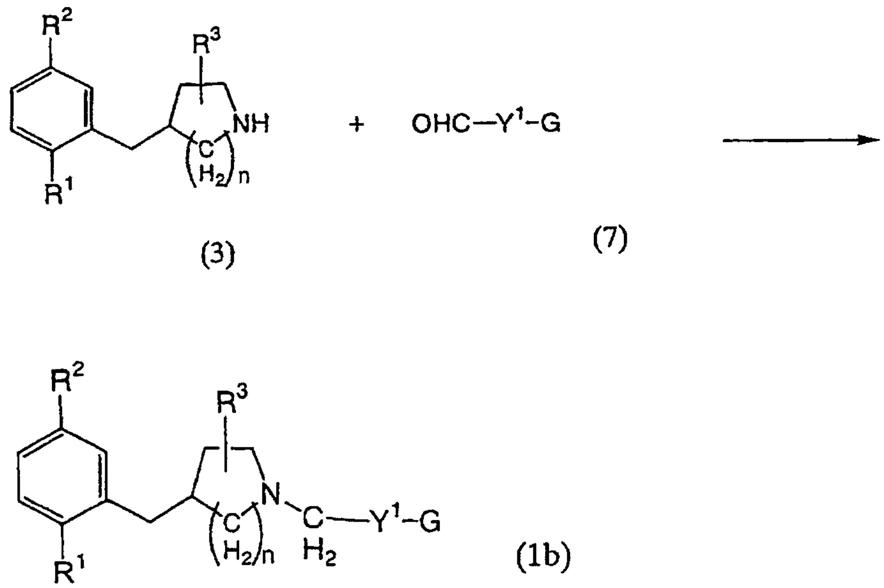

(3) (7)

(1b)

wherein $R^1$, $R^2$, $R^3$, G, n and $Y^1$ are as defined above.

A desired compound (1b) or a salt thereof may be obtained by reacting a compound (3) or a salt thereof with a compound (7) or a salt thereof under reductive amination conditions. As a reducing agent, sodium cyanoborohydride and sodium borohydride may be used. The compound (3) and the compound (7) may be mixed as they are or may be reacted after previous formation of an imine. The reaction may be carried out for 10 minutes to 48 hours in a suitable inert solvent (e.g. acetonitrile; a halogenated hydrocarbon such as chloroform, dichloromethane or the like; an aromatic hydrocarbon such as benzene, toluene or the like; an ether solvent such as diethyl ether, tetrahydrofuran, 1,4-dioxane or the like; a lower alcohol such as methanol, ethanol, isopropanol or the like; an aprotic polar solvent such as dimethylformamide, N-methylpyrrolidone, dimethyl sulfoxide or the like; or a mixed solvent thereof) at a temperature in a range of -20°C to the boiling point of the solvent used.

A certain desired compound (1) or a certain salt thereof may be produced by any of the processes shown in the following schemes.

Production process 4 (alkylation, acylation or the like of an amino group: when G is a group represented by the formula (2a))

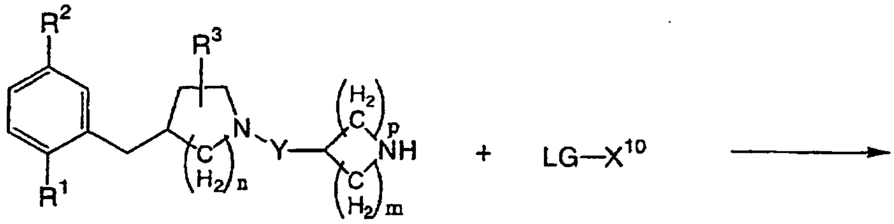

(8) (9)

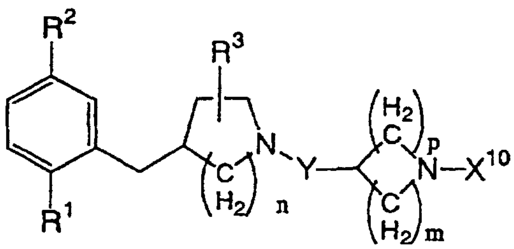

(1c)

wherein $R^1$, $R^2$, $R^3$, $X^{10}$, Y, p, m and n are as defined above, and LG is a leaving group.

The leaving group LG includes, for example, halogen atoms such as chlorine atom, bromine atom, iodine atom, etc.; and acyloxy groups such as acetoxy, tosyloxy, mesyloxy, etc.

A desired compound (1) or a salt thereof may be obtained by reacting a compound (8) or a salt thereof with a compound (9) or a salt thereof. The reaction may be carried out for 10 minutes to 48 hours in a suitable inert solvent at a temperature in a range of about -20°C to the boiling point of the solvent used, optionally in the presence of a base and optionally in the presence of a phase transfer catalyst.

The base includes, for example, organic bases such as triethylamine, pyridine, etc.; inorganic bases such as potassium carbonate, sodium hydroxide, sodium hydride, etc.; and metal alkoxides such as sodium methoxide, potassium tert-butoxide, etc.

The phase transfer catalyst includes, for example, tetrabutylammonium hydrogensulfate.

The inert solvent includes, for example, acetonitrile; halogenated hydrocarbons such as chloroform, dichloromethane, etc.; aromatic hydrocarbons such as benzene, toluene, etc.; ether solvents such as diethyl ether, tetrahydrofuran (THF), 1,4-dioxane, etc.; lower alcohols such as methanol, ethanol, isopropanol, etc.; aprotic polar solvents such as dimethylformamide, N-methylpyrrolidone, dimethyl sulfoxide, etc.; and mixed solvents thereof.

Production process 5 (reduction of an amide: when G is a group represented by the formula (2a) and $X^{10}$ is a group represented by $-CH_2-X^{11}$)

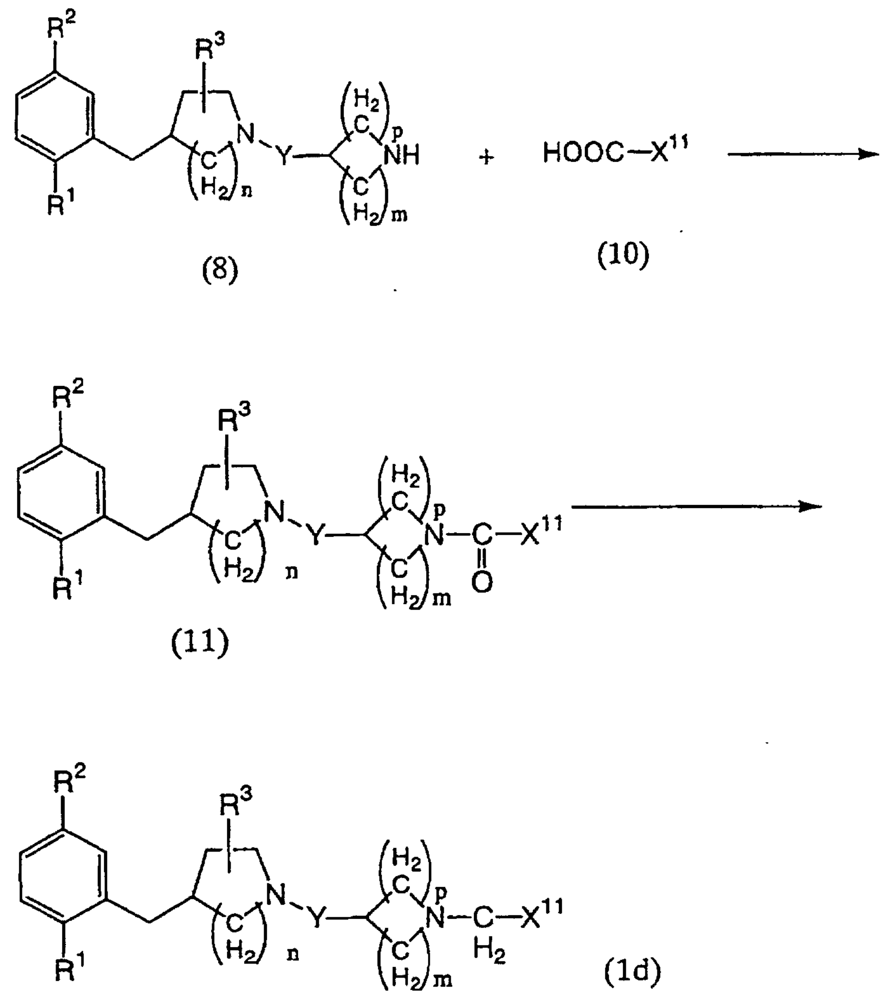

(8) (10)

(11)

(1d)

wherein $R^1$, $R^2$, $R^3$, Y, p, m and n are as defined above, $X^{11}$ is an alkyl group or a substituted alkyl group, and $-CH_2-X^{11}$ corresponds to the above symbol $X^{10}$.

An intermediate (11) may be produced by reacting a compound (8) or a salt thereof with a compound (10) or a salt thereof to form an amide linkage. This amide linkage formation reaction may be carried out by adopting a conventional method such as an acid chloride method using thionyl chloride, oxalyl chloride or the like; a mixed acid anhydride method using a chloroformate or the like; or a method using a condensing agent such as dicyclohexylcarbodiimide, carbonyldiimidazole or the like.

A compound (1d) may be obtained by reacting the intermediate (11) for 10 minutes to 48 hours by the use of a suitable reducing agent (e.g. lithium aluminum hydride, sodium borohydride or diborane, etc.) in a suitable inert solvent (e.g. an ether solvent such as diethyl ether, tetrahydrofuran (THF) or 1,4-dioxane, etc.) at a temperature in a range of -20°C to the boiling point of the solvent used. More specifically, the compound (1d) may be obtained by carrying out the reduction for 20 minutes to 1 hour by the use of diborane in tetrahydrofuran (THF) under ice-cooling or at room temperature.

Production process 6 (reductive amination: when G is a group represented by the formula (2a) and $X^{10}$ is a group represented by $-CH_2-X^{11}$)

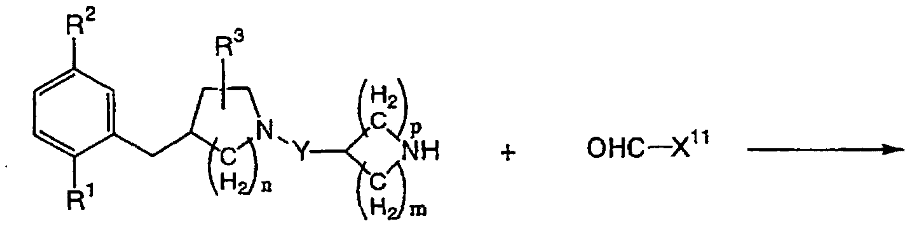

(8) (12)

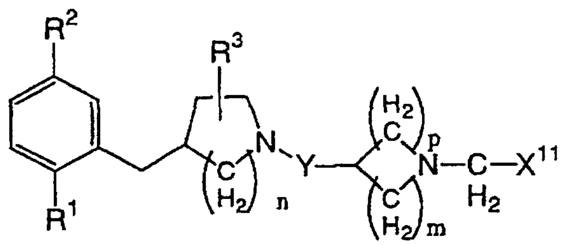

(1e)

wherein $R^1$, $R^2$, $R^3$, Y, p, m, n and $X^{11}$ are as defined above.

A desired compound (1e) or a salt thereof may be obtained by reacting a compound (8) or a salt thereof with a compound (12) or a salt thereof under reductive amination conditions. The reaction may be carried out by the same method as described in production process 3.

Production process of the intermediate (8)

The intermediate (8) can easily be synthesized from the intermediate (3) by a process based on production process 1, production process 2 or production process 3.

Production process 7 (alkylation, acylation or the like of an amino group: when G is a group represented by the formula (2b) and $X^{20}$ is a group represented by $N(R)X^{21}$)

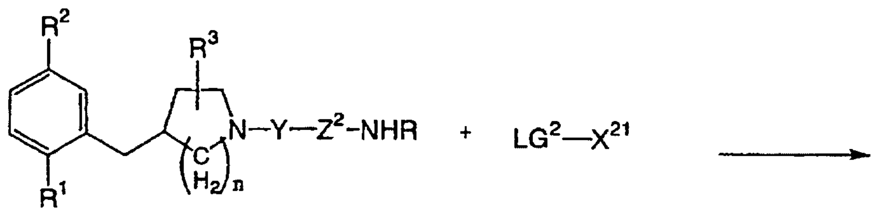

(1f) (13)

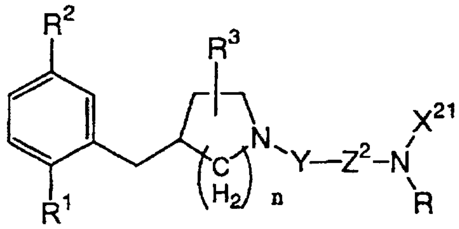

(1g)

wherein $R^1$, $R^2$, $R^3$, Y, $Z^2$ and n are as defined above; R is a hydrogen atom, an unsubstituted or substituted alkyl group, an unsubstituted or substituted acyl group, or the like; $X^{21}$ is an unsubstituted or substituted alkyl group, an unsubstituted or substituted acyl group, an unsubstituted or substituted alkanesulfonyl group, or the like; and $N(R)X^{21}$ corresponds to the above symbol $X^{20}$ when $X^{20}$ is a substituted amino group.

$LG^2$ has the same meaning as that of the above symbol LG when the above-mentioned reaction is alkylation. In addition, when the above-mentioned reaction is acylation, sulfonylation, sulfamoylation or the like, $LG^2$ is a halogen atom such as a chlorine atom, bromine atom or iodine atom, or $LG^2$-$X^{21}$ denotes an anhydride represented by $(X^{21})_2O$.

A desired compound (1g) or a salt thereof may be obtained also by reacting a compound (1f) or a salt thereof with a compound (13) or a salt thereof to convert the functional group of the compound (1f) or salt thereof. For example, when the reaction is alkylation, it may be carried out by the same method as described in production process 1. When the reaction is acylation, sulfonylation, sulfamoylation or the like, the reaction may be carried out by the use of a corresponding acid chloride, sulfonyl chloride, sulfamoyl chloride or the like, respectively. The reaction may be carried out for 10 minutes to 48 hours in a suitable inert solvent at a temperature in a range of about -20°C to the boiling point of the solvent used, optionally in the presence of a base and optionally in the presence of a phase transfer catalyst.

The base includes, for example, organic bases such as triethylamine, pyridine, etc.; inorganic bases such as potassium carbonate, sodium hydroxide, sodium hydride, etc.; and metal alkoxides such as sodium methoxide, potassium tert-butoxide, etc.

The phase transfer catalyst includes, for example, tetrabutylammonium hydrogensulfate.

The inert solvent includes, for example, acetonitrile; halogenated hydrocarbons such as chloroform, dichloromethane, etc.; aromatic hydrocarbons such as benzene, toluene, etc.; ether solvents such as diethyl ether, tetrahydrofuran (THF), 1,4-dioxane, etc.; lower alcohols such as methanol, ethanol, isopropanol, etc.; aprotic polar solvents such as dimethylformamide, N-methylpyrrolidone, dimethyl sulfoxide, etc.; and mixed solvents thereof.

Production process 8 (cyclization: when G is a group denoted by $Z^3$; $Z^3$ is represented by the formula (A); and $X^{31}$ is a bond or a methylene group)

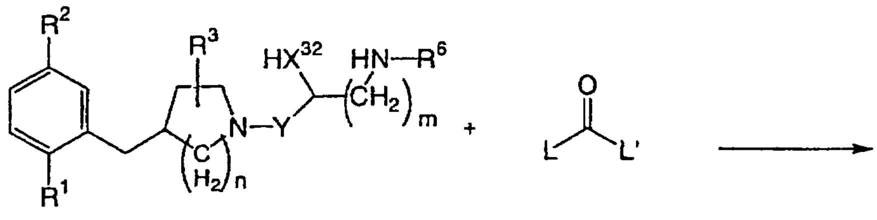

(14) (15)

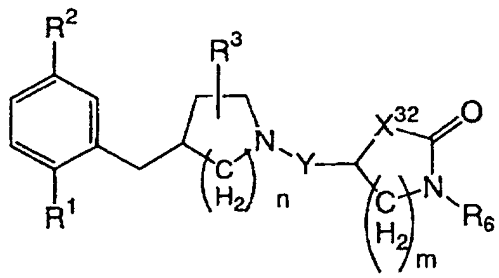

(1h)

wherein $R^1$, $R^2$, $R^3$, $R^6$, $X^{32}$, Y and n are as defined above; m is 1 or 2; and L and L' are independently Cl, Br, I, a free OH group or a reactive-functionally modified OH group.

A desired compound (1h) or a salt thereof may be obtained also by reacting a compound (14) or a salt thereof with a compound (15) or a salt thereof. A preferable compound as the compound (15) is a dialkyl carbonate such as dimethyl carbonate, ditrichloromethyl carbonate, diethyl carbonate or the like; a chloroformate such as methyl chloroformate, ethyl chloroformate or the like; N,N'-carbonyldiimidazole; or phosgene. The reaction may be carried out for 10 minutes to 48 hours in a suitable inert solvent (e.g. acetonitrile; a halogenated hydrocarbon such as chloroform, dichloromethane or the like; an aromatic hydrocarbon such as benzene, toluene or the like; an ether solvent such as diethyl ether, tetrahydrofuran, 1,4-dioxane or the like; an aprotic polar solvent such as dimethylformamide, N-methylpyrrolidone or the like; or a mixed solvent thereof) at a temperature in a range of 0°C to the boiling point of the solvent used.

The compound of the above formula (14) may be produced by the same process as described in the section of EXAMPLES.

A compound of the above formula (1) may be converted to another compound of the formula (1) by converting its functional group properly, without limiting its production to the production examples described above. The functional group may be converted by a conventional method [see, for example, R.C. Larock, Comprehensive Organic Transformations (1989)].

Production process of the compound (3)

The compound (3) or a salt thereof, which is useful as an intermediate, may be produced by the process shown in the following scheme:

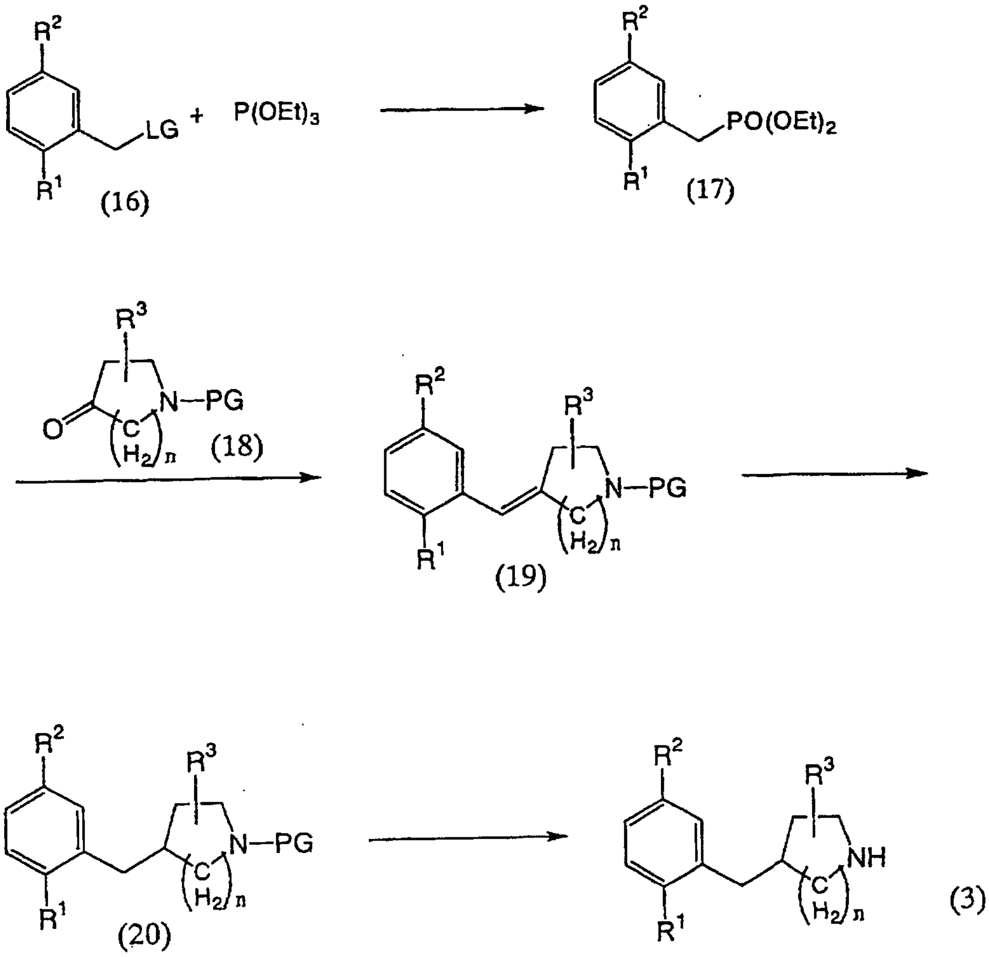

(16) (17) (18) (19) (20) (3)

wherein $R^1$, $R^2$, $R^3$, n and LG are as defined above, and PG is a protective group for the nitrogen atom.

The protective group for the nitrogen atom includes, for example, alkyloxycarbonyl groups such as t-butoxycarbonyl, 9-fluorenylmethyloxycarbonyl, etc. Examples of the leaving group LG are the same as those given above.

A compound (16) is converted to a Wittig-Horner reagent (17). This conversion may be carried out by reacting with triethyl phosphite for 1 hour to 3 days without a solvent or in an inert solvent at a temperature in a range of ice-cooling to the boiling point of the solvent used or triethyl phosphite. The Wittig-Horner reagent (17) may be converted to a compound (19) by reacting with a cyclic ketone (18) for 10 minutes to 48 hours in a suitable inert solvent in the presence of a base at a temperature of about -20°C to the boiling point of the solvent used.

The base includes, for example, organic bases such as triethylamine, pyridine, etc.; inorganic bases such as potassium carbonate, sodium hydroxide, sodium hydride, etc.; and metal alkoxides such as sodium methoxide, potassium tert-butoxide, etc.

The inert solvent includes, for example, acetonitrile; halogenated hydrocarbons such as chloroform, dichloromethane, etc.; aromatic hydrocarbons such as benzene, toluene, etc.; ether solvents such as diethyl ether, tetrahydrofuran, 1,4-dioxane, 1,2-dimethoxyethane, etc.; lower alcohols such as methanol, ethanol, isopropanol, etc.; aprotic polar solvents such as dimethylformamide, N-methylpyrrolidone, dimethyl sulfoxide, etc.; and mixed solvents thereof.

More specifically, the latter conversion may be carried out by conducting the reaction for 1 to 5 hours at 0°C to 50°C by using an inorganic base such as sodium hydride as the base and an ether solvent such as 1,2-dimethoxyethane as the inert solvent.

The compound (19) may be converted to a compound (20) by catalytic reduction. When $R^1$ or $R^2$ is a bromine atom, this conversion may be carried out by conducting the hydrogenation at 0°C to 50°C by the use of a rhodium catalyst (e.g. rhodium-carbon) as a catalyst for the hydrogenation. The desired compound (4) may be obtained by subjecting the compound (20) to deprotection by a conventional method.

Throughout the present description, protective groups, condensing agents and the like are indicated in some cases with abbreviations based on IUPAC-IUB (the committee on biochemical nomenclature) which are conventionally used in the art.

Suitable salts and pharmaceutically acceptable salts of the starting compounds and the desired compounds are conventional nontoxic salts. Those skilled in the art may properly select such salts from, for example, acid addition salts including organic acid salts (e.g. acetate, trifluoroacetate, maleate, fumarate, citrate, tartrate, methanesulfonate, benzenesulfonate, formate and toluenesulfonate, etc.) and inorganic acid salts (e.g. hydrochloride, hydrobromide, hydroiodide, sulfate, nitrate and phosphate, etc.); salts with amino acids (e.g. arginine, aspartic acid and glutamic acid, etc.); metal salts including alkali metal salts (e.g. sodium salt and potassium salt, etc.) and alkaline earth metal salts (e.g. calcium salt and magnesium salt, etc.); ammonium salts; and organic base salts (e.g. trimethylamine salt, triethylamine salt, pyridine salt, picoline salt, dicyclohexylamine salt and N,N'-dibenzylethylenediamine salt, etc.).

When a salt of a compound (1) is desired, it may be obtained as follow. When the compound (1) is obtained in the form of a salt, it is sufficient that the salt is purified as it is. When the compound (1) is obtained in a free form, it is sufficient that the compound (1) is dissolved or suspended in a suitable organic solvent and allowed to form a salt by a conventional method by the addition of an acid or a base. In addition, each of the compound (1) and a pharmacologically acceptable salt thereof exists in the form of an adduct with water or any of various solvents in some cases. The present invention also includes such an adduct.

In any of the production processes explained above, when any functional group in a site other than a reaction site changes under the reaction conditions explained above or is unsuitable for practicing the explained process, the desired compound may be obtained by protecting the functional group at the site other than the reaction site and carrying out the reaction, followed by deprotection. As a protective group, there may be used conventional protective groups such as those described, for example, in T. W. Green, Protective Groups in Organic Synthesis, John Wiley & Sons Inc. (1981). More specifically, a protective group for an amine includes ethoxycarbonyl, t-butoxycarbonyl, acetyl, benzyl, etc., and a protective group for a hydroxyl group includes trialkylsilyl, acetyl, benzyl, etc.

The introduction and removal of the protective group may be carried out by a method conventionally adopted in organic synthetic chemistry [see, for example, the above reference Protective Groups in Organic Synthesis] or a method based thereon.

A compound of the above formula (1) may be converted to another compound of the formula (1) by converting its functional group properly. The functional group may be converted by a conventional method [see, for example, R. C. Larock, Comprehensive Organic Transformations (1989)].

The intermediate(s) and desired compound in each of the production processes described above may be isolated and purified by purification methods conventionally adopted in organic synthetic chemistry, such as neutralization, filtration, extraction, washing, drying, concentration, recrystallization, various chromatographies, etc. The intermediate(s) may be subjected to the subsequent reaction without particular purification.

There are compounds (1) that can have tautomers. The present invention includes all possible isomers and mixtures thereof, inclusive of the tautomers.

A compound (1) and the other compounds can have one or more stereoisomers due to an asymmetric carbon atom(s). The present invention includes all of these isomers and mixtures thereof.

A desired compound (1) and a pharmaceutically acceptable salt thereof have pharmacological effects such as SRI (serotonin reuptake inhibition) effect. Therefore, they are useful for treating or preventing diseases in which the serotonin nervous system participates, for example, mood disorders including depression, seasonal affective disorder and dysthymia; anxiety disorders including obsessive-compulsive disorder and panic disorder; agoraphobia and avoidant personality disorder; social phobia; compulsive reaction; post-traumatic stress disorder; and psychosomatic disorder. Moreover, the desired compound (1) and pharmaceutically acceptable salt thereof of the present invention are useful also for treating or preventing memory impairments including dementia, forgetfulness and memory impairment associated with aging; eating disorders including anorexia nervosa and bulimia nervosa; obesity; somnipathy; schizophrenia; chemical dependency due to alcohol, tobacco, nicotine or the like; cluster headache; migraine; pains; Alzheimer's disease; chronic paroxysmal migraine; headache associated with vasculopathy; Parkinson's disease including dementia, depression and anxiety caused by Parkinson's disease, Parkinsonism induced by a neuroleptic agent, and tardive dyskinesia; and the like.

Furthermore, the desired compound (1) and pharmaceutically acceptable salt thereof of the present invention are useful for treating or preventing dysendocrinism such as hyperprolactinemia; vasospasm (in particular, cerebrovascular spasm); hypertension; kinetic gastrointestinal troubles and gastrointestinal troubles in which a secretion change participates; sexual dysfunction including premature ejaculation; drug dependence; and the like.

In addition, some of the desired compounds (1) and salts thereof of the present invention have lower affinity for histamine 1 receptors than their selective inhibitory effect on serotonin reuptake and affinity for serotonin 1A receptors. Therefore, they are less liable to induce sleep and hence are more useful.

For medical purposes, the compound (1) and pharmaceutically acceptable salt thereof of the present invention may be used in the form of a pharmaceutical composition as a mixture with a pharmaceutically acceptable carrier (e. g. a solid or liquid and organic or inorganic excipient) which is suitable for oral or parenteral administration or external use, which include local, enteral, intravenous, intramuscular, inhalational, nasal, intra-articular, intraspinal, transtracheal and transorbital administrations. The pharmaceutical composition includes solids, semisolids and liquids, such as capsules, tablets, pellets, sugar-coated tablets, powders, granules, suppositories, ointments, creams, lotions, inhalants, injections, poultices, gels, tapes, ophthalmic solutions, solutions, syrups, aerosols, suspensions, emulsions, etc. These pharmaceutical compositions may be prepared by conventional processes. If desired, conventional additives such as an assistant, stabilizer, wetting agent, emulsifier, buffer, etc. may be incorporated into the pharmaceutical compositions.

Although the dose of the compound (1) is varied depending on the age and condition of a patient, average doses of the compound (1) per administration of about 0.1 mg, 1 mg, 10 mg, 50 mg, 100 mg, 250 mg, 500 mg and 1000 mg are effective against diseases such as mood disorders including depression, seasonal affective disorder and dysthymia; and anxiety including generalized anxiety disorder and panic disorder. In general, when administered to human beings, the compound (1) may be administered in a dose of 0.1 mg/individual to about 1,000 mg/individual per day, preferably 1 mg/individual to about 100 mg/individual per day.

## EXAMPLES

The present invention is illustrated below in further detail with test examples, reference examples and working examples.

### Test Example 1 : Screening test using [$^3$H]paroxetine binding

#### 1-1. Preparation of a cerebral cortex membrane preparation

The membrane preparation was prepared according to the method[1a)] of D'amato et al. In detail, after a male rat was decapitated, the whole brain was immediately removed and the cerebral cortex was dissected therefrom under ice-cooling. A buffer solution (50 mM Tris-HCl (pH=7.4), 120 mM NaCl, 5 mM KCl) was added to the cerebral cortex in an amount of 25 times the wet weight of the cerebral cortex, followed by homogenization in an ice bath by the use of a Teflon-glass homogenizer. The homogenate was centrifuged at 4°C and 45,000 x g for 10 minutes, and the resulting precipitate was dispersed in an ice-cooled buffer solution (50 mM Tris-HCl (pH=7.4), 120 mM NaCl, 5 mM KCl) by the use of Hiscotron™, followed by centrifugation at 4°C and 45,000 x g for 10 minutes. The resulting precipitate was resuspended and the washing procedure was repeated. The membrane preparation thus obtained was suspended in the same buffer solution as described above, and then subjected to cryopreservation at -80°C.

#### 1-2. Receptor binding experiment

[$^3$H]paroxetine binding was measured according to the method[1b)] of Habert E et al. In detail, 200 μl of a dilution (final concentration: 0.15 nM) of [$^3$H]paroxetine with 50 mM Tris-HCl (pH=7.4) buffer containing 120 mM NaCl and 5 mM KCl, 790 μl of the cerebral cortex membrane preparation (150 μg/tube in terms of protein), 10 μl of a solution of a test drug in dimethyl sulfoxide and 1 ml of the above-mentioned buffer solution were mixed to prepare a liquid having a total volume of 2 ml. After this liquid was allowed to react at room temperature for 60 minutes, it was immediately filtered by suction at a low pressure through glass fiber filter. The glass fiber filter was washed 4 times with 4 ml of the same buffer solution as above and then transferred to a glass vial containing 4 ml of ACS-II (Amersham), and the radioactivity remaining on the filter was measured with a liquid scintillation counter.

Non-specific binding of [$^3$H]paroxetine was defined as the amount of [$^3$H]paroxetine bound in the presence of 100 μM 5-HT.

Rate of binding inhibition was calculated by the following equation:

Rate of binding inhibition (%) = 100 - 100 x

{[amount of [$^3$H]paroxetine bound in the presence of test substance] - [amount of [$^3$H]paroxetine bound in the presence of 100 μM 5-HT] / [amount of [$^3$H]paroxetine bound in the absence of test substance] - [amount of [$^3$H]paroxetine bound in the presence of 100 μM 5-HT]}

Cited reference:

1a) D'amato R.J. et al., J. Pharm. Exp. Ther., 242, 364-371 (1987),
1b) Habert E. et al., Eur. J. Pharmacol., 118, 107-114 (1985).

Test Example 2 : [$^3$H] 8-OH-DPAT binding test

2-1. Test Method

The test was carried out according to the method[2)] of Hall M.D. et al. After about one week of pre-breeding, a male rat was decapitated and the brain was immediately removed. The hippocampus was dissected from the brain on ice, and 50 mM Tris-HCl (pH=7.4) was added to the hippocampus in an amount of 40 times the wet weight of the hippocampus, followed by homogenization (3 min, 1 stroke/min) in an ice bath by the use of a Teflon-glass homogenizer. Then, the homogenate was centrifuged (4°C) at 40,000 x g for 10 minutes. The resulting precipitate was dispersed in an ice-cooled buffer solution (50 mM Tris-HCl (pH=7.4)) by the use of Hiscotron™, followed by centrifugation (4°C) at 40,000 x g for 10 minutes. Moreover, the resulting precipitate was re-suspended and the washing procedure was repeated once. The precipitate thus obtained was dispersed in an ice-cooled buffer solution (50 mM Tris-HCl (pH=7.4)) by the use of Hiscotron™, and the resulting dispersion was incubated at 37°C for 1 hour and then centrifuged (4°C) at 40,000 x g for 10 minutes. Furthermore, the resulting precipitate was re-suspended and the washing procedure was repeated once. The membrane preparation thus obtained was suspended in the same buffer solution as described above, and then subjected to cryopreservation at -80°C.

Measurement was carried out by using a liquid for reaction (total volume: 200 µl) obtained by adding 50 µl of [$^3$H]8-OH-DPAT (final concentration: 2 nM), 4 µl of a solution of a test drug and 146 µl of the hippocampus membrane preparation (80 µg/tube in terms of protein) to a buffer solution containing 50 mM Tris-HCl (pH=7.4) and 4 mM $CaCl_2$. After the liquid for reaction was allowed to react at room temperature for 30 minutes, it was immediately filtered by suction at a low pressure through glass fiber filter. The glass fiber filter was washed twice with 250 µl of a buffer solution and then added to a glass vial containing 4 ml of ACS-II (Amersham), and the radioactivity bound to receptors and remaining on the filter was measured with a liquid scintillation counter. Non-specific binding of [$^3$H]8-OH-DPAT was defined as the amount of [$^3$H]8-OH-DPAT bound in the presence of 1 µM 8-OH-DPAT.

Rate of binding inhibition was calculated by the following equation:

Rate of binding inhibition (%) = 100 - 100 x
{[amount of [$^3$H] 8-OH-DPAT bound in the presence of test substance] - [amount of [$^3$H]8-OH-DPAT bound in the presence of 1 µM 8-OH-DPAT] / [amount of [$^3$H]8-OH-DPAT bound in the absence of test substance] - [amount of [$^3$H]8-OH-DPAT bound in the presence of 1 µM 8-OH-DPAT]}

Cited reference:

2) Hall M.D. et al., J. Neurochem., 44, 1685-1696 (1985).

Test Example 3 : 5-$HT_{1A}$ receptor stimulation test

3-1. Cells used and preparation of a membrane preparation

CHO cells expressing human 5-$HT_{1A}$ receptor (human 5-$HT_{1A}$/CHO) were used in the experiment. The cells were cultured in F12 containing 10% FCS, 500 µg/ml Geneticin and 100 U/ml penicillin-100 µg/ml streptomycin (all available from GIBCO) in a 5% $CO_2$ incubator, and a membrane preparation was prepared according to the method[3)] of A. Newman et al. In detail, cells scraped and collected by the use of buffer solution A (20 mM HEPES, 5 mM $MgSO_4$)

were homogenized in a Teflon™ homogenizer, followed by centrifugation (50,000 x g, 30 min, 4°C). The precipitate was re-suspended in a suitable volume of buffer solution A and stored at -80°C until use. The amount of protein in the membrane preparation was determined with Dye Reagent Concentrate (BIO-RAD) by using Albumin Bovine (SIGMA) as a standard substance.

3-2. Experimental method

Binding of [$^{35}$S]GTPγS to human 5-$HT_{1A}$ receptors was measured by the use of the above-mentioned membrane preparation according to the method[3] of A. Newman et al. In detail, 0.05 nM of [$^{35}$S]GTPγS (E.I.du Pont de Nemours & Co. NEN) and a definite amount (about 50 μg/tube) of the membrane preparation were added to buffer solution B (20 mM HEPES, 3 mM $MgSO_4$, 3 μM GDP, 1mM DTT) containing $10^{-5}$ M of each test substance, and the resulting liquid for reaction having a total volume of 1 ml was incubated at 22°C for 20 minutes. After completion of the reaction, the reaction mixture was diluted with 5 ml of ice-cooled buffer solution B, and the dilution was immediately filtered by suction through glass fiber filter (Whatman, GF/B) to terminate the reaction. The glass fiber filter was washed twice with the same buffer solution as above and placed in a vial, and 4 ml of ACS-II was added thereto. The radioactivity of [$^{35}$S]GTPγS on the filter was measured with a liquid scintillation counter. The amount of [$^{35}$S]GTPγS specifically bound was calculated from the amount of [$^{35}$S]GTPγS non-specifically bound in the presence of 10 μM of GTPγS (Sigma). The 5-$HT_{1A}$ receptor stimulating activity of each test substance was expressed in terms of the rate of increase in [$^{35}$S]GTPγS binding by taking an increase in [$^{35}$S]GTPγS binding caused by 10 μM of 5-HT, as 100%.

Cited reference:

3) Adrian Newman-Tancredi et al., Eur. J. Pharmacol., 307, 107-111 (1996).

For the compounds obtained in the working examples, the results of the above Test Example 1, Test Example 2 and Test Example 3 were as shown in Table 1.

Table 1: Test results

Table 1

| Compound (No. of Example) | Rate of inhibition of [$^3$H] paroxetine binding (%) | Rate of inhibition of [$^3$H] 8-OH-DPAT binding (%) | 5-$HT_{1A}$ receptor stimulating activity (%) |
|---|---|---|---|
| 1 | 81 | 47 | |
| 4 | 90 | 85 | 31 |
| 6 | 87 | 71 | 53 |
| 8 | 93 | 96 | 43 |
| 9 | 79 | 76 | |
| 18 | 80 | 95 | 40 |
| 20 | 83 | 79 | 53 |
| 22 | 82 | 74 | 55 |
| 25 | 94 | 97 | 24 |
| 29 | 95 | 81 | 46 |
| 32 | 97 | 73 | |
| 38 | 100 | 96 | 48 |
| 41 | 78 | 86 | |
| 44 | 99 | 92 | 51 |
| 46 | 100 | 65 | 62 |
| 47 | 100 | 95 | 37 |
| 48 | 99 | 90 | 33 |
| 52 | 90 | 60 | |
| 54 | 80 | 99 | 16 |
| 58 | 100 | 92 | 30 |
| 60 | 91 | 98 | |

Table 1 (continued)

| Compound (No. of Example) | Rate of inhibition of [$^3$H] paroxetine binding (%) | Rate of inhibition of [$^3$H] 8-OH-DPAT binding (%) | 5-$HT_{1A}$ receptor stimulating activity (%) |
|---|---|---|---|
| 64 | 96 | 96 | 19 |
| 65 | 97 | 60 | 64 |
| 67 | 81 | 73 | 74 |
| 69 | 95 | 96 | 63 |
| 70 | 84 | 86 | 7 |
| 72 | 99 | 97 | |

Reference Example 1

4-(2-Bromo-5-methoxybenzyl)piperidine hydrochloride

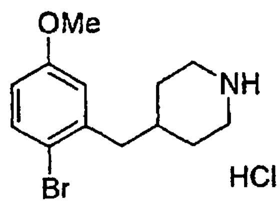

1-1) N-tert-butoxycarbonyl-4-piperidone

A solution of di-tert-butyl dicarbonate (41.3 g, 189 mmol) in 1,4-dioxane (120 mL) and a 1N aqueous sodium hydroxide solution (200 mL) were added dropwise at the same time to an ice-cooled mixture of 4-piperidone hydrochloride monohydrate (29 g, 189 mmol) and 1,4-dioxane (120 mL), and stirred for 30 minutes. After the 1,4-dioxane was distilled off under reduced pressure, the residue was extracted twice with ethyl acetate. The combined organic layer was washed successively with a 5% aqueous potassium hydrogensulfate solution, distilled water and a saturated aqueous sodium chloride solution, and then dried over anhydrous magnesium sulfate. The solvent was distilled off under reduced pressure to obtain N-tert-butoxycarbonyl-4-piperidone (34.8 g, 92.5%) as white crystals.

$^1$H-NMRδ ($CDCl_3$, ppm): 3. 72 (t, J = 6. 3 Hz, 4H), 2.44 (t, J = 6.3Hz, 4H), 1.50 (s, 9H).

1-2) 2-Bromo-5-methoxybenzyl bromide

Azobisisobutyronitrile (0.60 g) was added to a mixture of 2-bromo-5-methoxytoluene (77.38 g, 384 mmol), 5,5-dimethy-1,3-dibromohydantoin (57.5 g, 200 mmol) and chlorobenzene (1500 mL), followed by stirring at 70°C for 2 hours. Azobisisobutyronitrile (0.60 g) was added thereto again and stirred for another 1 hour. A 10% aqueous sodium thiosulfate solution (500 mL) was added to the reaction mixture, and the organic layer was washed with a saturated aqueous sodium chloride solution. The organic layer washed was dried over anhydrous magnesium sulfate and then concentrated to dryness to obtain a crude product of 2-bromo-5-methoxybenzyl bromide. Thereto was added 100 mL of ice-cooled hexane to obtain a homogeneous slurry, which was filtered. The residue was washed with 100 mL of ice-cooled hexane and then dried under reduced pressure to obtain 65.75 g (235 mmol, 61.1%) of 2-bromo-5-methoxybenzyl bromide.

NMR ($CDCl_3$) δ 3.79 (s, 3H), 4.55 (s, 2H), 6.73 (dd, 1H, J = 3.0, 8.9 Hz), 6.99 (d, 1H, J = 3.0 Hz), 7.40 (d, 1H, J = 8.9 Hz).

1-3) 4-(2-Bromo-5-methoxybenzyl)piperidine hydrochloride

A mixture of 2-bromo-5-methoxybenzyl bromide (33.2 g) and triethyl phosphite (19.2 g, 116 mmol) was stirred at 90°C for 12 hours. The bromoethane produced was distilled off to obtain 42.4 g of a crude product of diethyl 2-bromo-5-methoxybenzylphosphonate.

NMR ($CDCl_3$) δ 1.27 (t, 6H, J = 6.9 Hz), 3.37 (d, 2H, J = 22.1 Hz), 3.79 (s, 3H), 3.98-4.16 (m, 4H), 6.63-6.72 (m, 1H), 7.02 (t, 1H, J = 2.8 Hz), 7.43 (d, 1H, J = 8.6 Hz).

Sodium hydride (60% in paraffin, 14.4 g, 0.360 mol) was added in small portions to a mixture of the crude product of diethyl 2-bromo-5-methoxybenzylphosphonate (147 g), N-tert-butoxycarbonyl-4-piperidone (65.1 g, 0.327 mol) and 1,2-dimethoxyethane (200 mL) at room temperature, and the resulting mixture was stirred as it was for 1.5 hours. Water (1200 mL) was added to the reaction mixture, followed by extraction with ether (500 mL x 3). The combined organic layer was washed with water (200 mL) and a saturated aqueous sodium chloride solution (200 mL). The combined organic layer washed was dried over anhydrous magnesium sulfate and then concentrated to dryness, and the residue was washed with hexane to obtain 98.2 g (0.257 mol, 79%) of N-tert-butoxycarbonyl-4-(2-bromo-5-methoxybenzal)piperidine.

NMR ($CDCl_3$) δ 1.48 (s, 9H), 2.36-2.51 (m, 4H), 3.41 (t, 2H, J = 5.8 Hz), 3.53 (t, 2H, J = 5.9 Hz), 3.78 (s, 3H), 6.27 (s, 1H), 6.66 (dd, 1H, J = 3.0, 8.9 Hz), 6.71 (d, 1H, J = 3.0 Hz), 7.45 (d, 2H, J = 8.9 Hz).

A mixture of N-tert-butoxycarbonyl-4-(2-bromo-5-methoxybenzal)piperidine (87.7 g, 0.229 mol), 5% rhodium-carbon (25.6 g), ethyl acetate (150 mL) and ethyl alcohol (150 mL) was stirred at 40°C for 16 hours under a hydrogen atmosphere. The precipitate was filtered and then washed with ethyl acetate, and the filtrate was concentrated to dryness. The residue was diluted with ethyl acetate (800 mL) and washed with a saturated aqueous sodium chloride solution (100 mL). The dilution washed was dried over anhydrous magnesium sulfate and then concentrated to dryness to obtain 78.7 g (0.205 mol, 89%) of N-tert-butoxycarbonyl-4-(2-bromo-5-methoxybenzyl)piperidine.

NMR ($CDCl_3$) δ 1.08-1.36 (m, 2H), 1.46 (s, 9H), 1.52-1.70 (m, 2H), 1.70-1.88 (m, 1H), 2.55-2.75 (m, 4H), 3.78 (s, 3H), 3.98-4.18 (m, 2H), 6.64 (dd, 1H, J = 3.1, 8.9 Hz), 6.70 (d, 1H, J = 3.3 Hz), 7.42 (d, 2H, J = 8.6 Hz).

At room temperature, a 4N-hydrogen chloride/1,4-dioxane solution (35 mL) was added dropwise to a solution of the crude product of N-tert-butoxycarbonyl-4-(2-bromo-5-methoxybenzyl)piperidine (18.3 g) in acetic acid (50 mL), and the resulting mixture was stirred at 60°C for 1 hour. The solvent was distilled off and ethyl acetate was added to the residue to effect crystallization. The crystals were collected by filtration, washed with ethyl acetate and then dried to obtain 12.9 g (40.2 mmol) of 4-(2-bromo-5-methoxybenzyl)-piperidine hydrochloride.

Retention time: 3.89 minutes.

The following compounds of Reference Examples 2 to 8 were produced in the same manner as in Reference Example 1.

Reference Examples 2 to 8

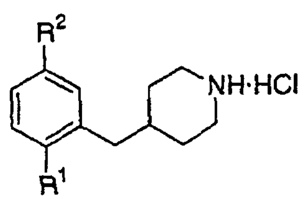

Table 2

| No. of Reference Example | $R^1$ | $R^2$ | Retention time (min.) |
|---|---|---|---|
| 2 | Br | H | 3.73 |
| 3 | Cl | OMe | 3.47 |
| 4 | Br | F | 3.16 |
| 5 | Br | Cl | 4.06 |
| 6 | Br | OEt | 5.01 |
| 7 | Br | OiPr | 6.02 |
| 8 | Br | $OCHF_2$ | 2.39 |
| (In the table, iPr denotes an isopropyl group (hereinafter the same applied.)) | | | |

Reference Example 9

4-(2-Bromo-5-hydroxybenzyl)piperidine

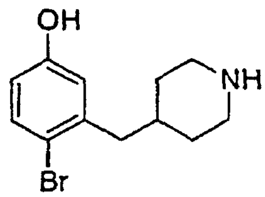

After 4-(2-bromo-5-methoxybenzyl)piperidine hydrochloride (16.00 g, 50.22 mmol) was suspended in a 1N aqueous sodium hydroxide solution (100 ml), the organic substance liberated was extracted with diethyl ether, and the organic layer was dried over anhydrous potassium carbonate and distilled under reduced pressure to remove the solvent. While cooling a solution of the thus obtained free amine in dichloromethane (100 ml) at 0°C, a 1.0M solution of boron tribromide in dichloromethane (100 ml) was added dropwise thereto over a period of 5 hours and the resulting mixture was stirred at room temperature for 3.5 hours. The reaction was quenched by adding methanol (500 ml), and the reaction mixture was stirred for 10 minutes and then distilled under reduced pressure to remove the solvent. The residue was dissolved in a 4N aqueous sodium hydroxide solution (200 ml) and washed with diethyl ether, and then acetic acid was added dropwise thereto until the aqueous layer became neutral. The gummous substance produced was extracted with ethyl acetate and the organic layer was dried over anhydrous sodium sulfate and distilled under reduced pressure to remove the solvent, whereby 4-(2-bromo-5-hydroxybenzyl)piperidine (9.93 g, 37.03 mmol) was obtained in a yield of 74%.
Retention time: 2.10 minutes.

Reference Example 10

trans-3-(4-((tert-Butoxycarbonyl)amino)cyclohexyl)propionic acid

10-1) trans-3-(4-Aminocyclohexyl)propionic acid

A mixture of trans-3-(4-(acetylamino)cyclohexyl)propionic acid (3.0 g, 14.1 mmol) and 47% hydrobromic acid (15 mL) was stirred with heating at 120°C for 100 hours under a nitrogen atmosphere. The reaction mixture was concentrated under reduced pressure to obtain 3.39 g of the title compound as a pale ocherous solid.

10-2) trans-3-(4-((tert-Butoxycarbonyl)amino)cyclohexyl)propionic acid

A solution of di-tert-butyl dicarbonate (3.23 g) in 1,4-dioxane (27 ml) was added dropwise to a mixture of trans-3-(4-aminocyclohexyl)propionic acid (3.39 g) and a 1N-aqueous sodium hydroxide solution (27 ml) over a period of 10 minutes, and the resulting mixture was stirred at room temperature for 5 hours. The reaction mixture was distilled under reduced pressure to remove the volatile component, and then the residue was washed with hexane. The aqueous layer was acidified with a 5% aqueous potassium hydrogensulfate solution and extracted twice with ethyl acetate. The extract solution was washed with a saturated aqueous sodium chloride solution, dried over magnesium sulfate and then concentrated to obtain 3.06 g of the title compound as a white solid.

Reference Example 11

1-Amino-3-(4-(2-bromo-5-methoxybenzyl)piperidin-1-yl)propan-2-ol

11-1) 4-(2-Bromo-5-methoxybenzyl)-1-(oxiran-2-ylmethyl)piperidine

Chloroform and a 1N-aqueous sodium hydroxide solution were added to the compound of Reference Example 1 (6.41 g, 20.0 mmol), then separated, and the chloroform layer was dried over potassium carbonate, filtered and then concentrated to make said compound into a free compound. Potassium carbonate (6.91 g, 50.0 mmol) was added to

a solution of the free compound and epichlorohydrin (1.72 mL, 22.0 mmol) in N,N-dimethylformamide (40 mL), and the resulting mixture was stirred at 50°C for 3 hours. Then, epichlorohydrin (0.86 mL, 11.0 mmol) was further added, followed by stirring at 50°C for 3 hours. After the reaction mixture was cooled, water was added thereto, followed by extraction with ethyl acetate/toluene (1 : 1, twice). The organic layer was washed with water and then a saturated aqueous sodium chloride solution, dried over anhydrous magnesium sulfate and then concentrated under reduced pressure. The residue was purified by a silica gel column chromatography (silica gel 350 mL, chloroform/methanol = 1 : 0 to 100 : 1) to obtain 5.6 g (82%) of the title compound as a yellow oil.

$^1$H-NMR (400 MHz, $CDCl_3$)

δ 1.06 (9H, s), 1.20-1.30 (2H, m), 1.50-1.60 (6H, m), 1.66 (2H, t, J = 5.4 Hz), 3.43 (2H, s), 3.52 (1H, br), 3.71 (2H, t, J = 5.4 Hz), 7.35-7.45 (6H, m), 7.65-7.70 (4H, m).

11-2) 1-Azido-3-(4-(2-bromo-5-methoxybenzyl)piperidin-1-yl)propan-2-ol

A solution of sodium azide (1.61 g, 24.8 mmol) in water (4.1 mL) and 18-crown-6 (264 mg, 1.00 mmol) were added to a solution of 4-(2-bromo-5-methoxybenzyl)-1-(oxiran-2-ylmethyl)piperidine (5.6 g, 16.5 mmol) in 1,4-dioxane (30 mL) at 80°C, and the resulting mixture was warmed to 110°C and stirred for 14 hours. Sodium azide (1.07 g, 16.5 mmol) and 18-crown-6 (264 mg, 1.00 mmol) were added to the reaction mixture, followed by stirring at 110°C for another 10 hours. After the reaction mixture was cooled, a 10% aqueous potassium carbonate solution was added thereto, followed by extraction (twice) with diethyl ether. The ether layer was dried over anhydrous magnesium sulfate and concentrated under reduced pressure. The concentration residue was purified by a silica gel column chromatography (silica gel 400 mL, chloroform/methanol = 100 : 1 to 50 : 1) to obtain 4.57 g (72%) of the title compound as a colorless oil.

1R (neat. NaCl)

ν 3500, 2933, 2099 $cm^{-1}$

11-3) 1-Amino-3-(4-(2-bromo-5-methoxybenzyl)piperidin-1-yl)propan-2-ol

A solution of triphenylphosphine (3.44 g, 13.1 mmol) in tetrahydrofuran (27 mL) was added to a solution of 1-azido-3-(4-(2-bromo-5-methoxybenzyl)piperidin-1-yl)propan-2-ol (4.57 g, 11.9 mmol) in tetrahydrofuran-water (5 : 1, 33 mL) at room temperature, and the resulting mixture was heated under reflux for 6 hours. After the reaction mixture was concentrated under reduced pressure, toluene was added thereto and a 4N hydrogen chloride-dioxane solution (10 mL) was added thereto with stirring. The supernatant was removed by decantation and the residue was dissolved in a small volume of chloroform. Toluene was added to the resulting solution to effect re-precipitation and the supernatant was removed by decantation. The residue was made into a methanolic solution, which was concentrated to dryness to obtain 4.39 g (86%) of the title compound as a yellow glassy solid.

LC-MS (ES+)

m/z 357 (M+1), 340 ($M-NH_2$).

Reference Example 12

5-Hydroxymethyl-3-(4-methoxyphenyl)oxazolidin-2-one

12-1) 1-(4-Methoxyphenyl)aminopropane-2,3-diol

To a solution of glycidol (7.41 g, 100 mmol) in ethanol (100 mL) was added p-anisidine (12.32 g, 100 mmol), and the resulting mixture was heated under reflux for 30 hours. The reaction mixture was concentrated under reduced pressure and the concentration residue was purified by a silica gel column chromatography (silica gel 300 mL, hexane/ethyl acetate = 1 : 1 to 0 : 1) to obtain 10.24 g (52%) of the title compound as a pale brown solid.

$^1$H-NMR (400 MHz, $CDCl_3$)

δ 3.16 (1H, dd, J = 12.8, 7.5 Hz), 3.26 (1H, dd, J = 12.8, 4.1 Hz), 3.65 (1H, dd, J = 11.3, 5.8 Hz), 3.75 (3H, s), 3.79 (1H, dd, J = 11.3, 3.6 Hz), 3.90-4.00 (1H, m), 6.65 (2H, d, J = 8.9 Hz), 6.79 (2H, d, J = 8.9 Hz).

12-2) 5-Hydroxymethyl-3-(4-methoxyphenyl)oxazolidin-2-one

Sodium methoxide (a 28% methanolic solution, 3.16 mL, 15.6 mmol) was added to a solution of 1-(4-methoxyphenyl)aminopropane-2,3-diol (10.24 g, 51.9 mmol) and diethyl carbonate (18.9 mL, 156 mmol) in toluene (52 mL) at 105°C, and stirred for 8 hours. After the reaction mixture was concentrated under reduced pressure, ethyl acetate (400 mL) was added to the resulting residue and the resulting mixture was heated until its boiling. The insoluble material

was removed by filtration with heating and the filtrate was concentrated under reduced pressure. The concentration residue was recrystallized from ethyl acetate to obtain 7.33 g (61%) of the title compound as white powdery crystals.

$^1$H-NMR (400 MHz, DMSO-$d_6$)

δ 3.55 (1H, ddd, J = 12.3, 5.8, 4.3 Hz), 3.66 (1H, ddd, J = 12.3, 5.6, 3.5 Hz), 3.74 (3H, s), 3.79 (1H, dd, J = 8.7, 6.4 Hz), 4.04 (1H, t, J = 8.9 Hz), 4.60-4.70 (1H, m), 5.20 (1H, t, J = 5.7 Hz), 6.95 (2H, d, J = 9.1 Hz), 7.47 (2H, d, J = 9.1 Hz).

Reference Example 13

3-(2-Bromo-5-methoxyethoxybenzyl)pyrrolidine hydrochloride

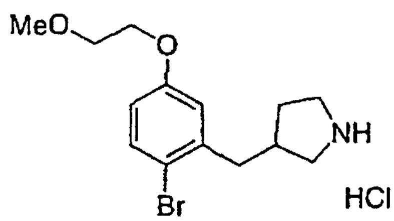

13-1) N-benzyl-3-(2-bromo-5-methoxyethoxybenzylidene)pyrrolidine

Under nitrogen, 3-methoxyethoxybenzyltriphenylphosphonium bromide (3.18 g, 6.28 mmol) was suspended in tetrahydrofuran (30 ml), followed by adding thereto potassium tert-butoxide (0.768 g, 6.85 mmol), and the resulting mixture was stirred at room temperature for 30 minutes.

A solution of 1-benzyl-3-pyrrolidinone (1.0 g, 5.71 mmol) in tetrahydrofuran (5 ml) was added dropwise thereto over a period of 5 minutes, and the resulting mixture was stirred for about 4 days while being maintained at 60°C.

The reaction mixture was cooled to room temperature and water (about 40 ml) was added thereto, followed by extraction (twice) with ethyl acetate. The combined extract layer was washed with water and then a saturated aqueous sodium chloride solution, dried by the addition of anhydrous magnesium sulfate, filtered, and then the solvent was removed under reduced pressure to afford the residue (5.50 g), which was purified by a column chromatography by the use of 200 ml of silica gel and n-hexane/ethyl acetate = 3/1 ~ 2/1 ~ 1/1 to obtain N-benzyl-3-(3-methoxyethoxybenzylidene)pyrrolidine (1.05 g, yield 56.9%).

$^1$H-NMR (300 MHz, $CDCl_3$) σ ppm: 7.38-7.17 (m, 6H), 6.87 (d, 1H, J = 8.6 Hz), 6.75 (m, 2H), 6.26 (s, 1H), 4.10 (m, 2H), 4.73 (m, 4H), 3.49 (m, 1H), 3.44 (s, 3H), 3.32 (m, 1H), 2.76 (s, 2H), 2.67 (s, 2H).

LC-MS: 324 ($M^+$+1)

13-2) 3-(3-Methoxyethoxybenzyl)pyrrolidine

Under nitrogen, N-benzyl-3-(3-methoxyethoxybenzylidene)pyrrolidine (1.0 g, 3.09 mmol) was dissolved in acetic acid (16 ml), followed by adding thereto 10%-Pd/C (0.10 g), and hydrogen was added thereto at room temperature and stirred for 3.5 hours.

After replacement with nitrogen, the Pd/C was filtered off and the solvent was distilled off under reduced pressure. The concentration residue (5.50 g) thus obtained was dissolved in methanol (20 ml), and formic acid (1 ml) and 10%-Pd/C (0.10 g) were added thereto, followed by stirring at room temperature for 12 hours.

The Pd/C was filtered off and the solvent was distilled off under reduced pressure, followed by two runs of azeotropy with toluene, whereby 3-(3-methoxyethoxybenzyl)pyrrolidine (0.957 g) was obtained.

$^1$H-NMR (300 MHz, $CDCl_3$) σ ppm: 8.49 (br.s, 1H), 7.19 (t, 1H, J = 7.9 Hz), 6.76 (m, 3H), 4.10 (m, 2H), 3.75 (m, 2H), 3.45 (s, 3H), 3.35-3.13 (m, 3H), 2.86 (m, 1H), 2.70 (m, 2H), 2.58 (m, 1H), 2.02 (m, 1H), 1.68 (m, 1H).

LC-MS: 236 ($M^+$+1)

13-3) N-tert-butoxycarbonyl-3-(3-methoxyethoxybenzyl)pyrrolidine

Under nitrogen, 3-(3-methoxyethoxybenzyl)pyrrolidine (624 mg, 2.65 mmol) was dissolved in tetrahydrofuran

(10 ml), followed by adding thereto di-tert-butyl carbonate (868 mg, 3.98 mmol), and the resulting mixture was stirred at room temperature for 2 hours.

The solvent was distilled off under reduced pressure and the resulting residue was purified by a column chromatography by the use of 80 ml of silica gel and n-hexane/ethyl acetate = 5/1 ~ 4/1 ~ 3/1 to obtain N-tert-butoxycarbonyl-3-(3-methoxyethoxybenzyl)pyrrolidine (432 mg, yield 48.6%).

$^1$H-NMR (300 MHz, $CDCl_3$) σ ppm: 7.19 (m, 1H), 6.75 (m, 3H), 4.10 (m, 2H), 3.75 (dd, 2H, J = 4.9, 4.4 Hz), 3.49 (m, 2H), 3.41 (s, 3H), 3.24 (m, 1H), 2.97 (m, 1H), 2.63 (m, 2H), 2.41 (m, 1H), 1.90 (m, 1H), 1.55 (m, 1H), 1.45 (s, 9H).

13-4) N-tert-butoxycarbonyl-3-(2-bromo-5-methoxyethoxybenzyl)pyrrolidine

Under nitrogen, N-tert-butoxycarbonyl-3-(3-methoxyethoxybenzyl)pyrrolidine (407 mg, 1.21 mmol) was dissolved in N,N-dimethylformamide (4 ml), and N-bromosuccinimide (238 mg, 1.33 mmol) was added thereto with stirring under ice-cooling. The resulting mixture was stirred under ice-cooling for 1 hour and then at room temperature for 1.5 hours.

A saturated aqueous sodium hydrogencarbonate solution and water were added to the mixture and stirred, followed by extraction with toluene/ethyl acetate = 1/1. The extract layer was washed twice with water and then with a saturated aqueous sodium chloride solution, dried by the addition of anhydrous magnesium sulfate, filtered, and then the solvent was removed under reduced pressure to afford the residue (524 mg), which was purified by a column chromatography by the use of 70 ml of silica gel and n-hexane/ethyl acetate = 4/1 to obtain N-tert-butoxycarbonyl-3-(2-bromo-5-methoxyethoxybenzyl)pyrrolidine (470 mg, yield 93.5%).

$^1$H-NMR (300 MHz, $CDCl_3$) σ ppm: 7.41 (dd, 1H, J = 8.6, 5.3 Hz), 6.78 (s, 1H), 6.67 (m, 1H), 4.08 (m, 2H), 3.73 (dd, 2H, J = 4.9, 4.2 Hz), 3.49 (m, 2H), 3.43 (s, 3H), 3.26 (m, 1H), 3.01 (m, 1H), 2.75 (m, 2H), 2.52 (m, 1H), 1.91 (m, 1H), 1.62 (m, 1H), 1.45 (s, 9H).

LC-MS: 414 ($M^+$)

13-5) 3-(2-Bromo-5-methoxyethoxybenzyl)pyrrolidine hydrochloride

Under nitrogen, N-tert-butoxycarbonyl-3-(2-bromo-5-methoxyethoxybenzyl)pyrrolidine (447 mg, 1.08 mmol) was dissolved in acetic acid (6 ml), followed by adding thereto a 4N-hydrochloric acid/1,4-dioxane solution (3 ml), and the resulting mixture was stirred at room temperature for 2 hours.

The mixture was filtered and the solvent was distilled off under reduced pressure, followed by two runs of azeotropy with toluene, whereby 3-(2-bromo-5-methoxyethoxybenzyl)pyrrolidine hydrochloride (351 mg, yield 92.8%) was obtained.

$^1$H-NMR (300 MHz, $CDCl_3$) σ ppm: 9.72 (br. m, 2H), 7.41 (d, 1H, J = 8.8 Hz), 6.86 (d, 1H, J = 2.9 Hz), 6.69 (dd, 1H, J = 8.8, 2.9 Hz), 4.09 (m, 2H), 3.73 (dd, 2H, J = 4.8, 4.4 Hz), 3.46 (s, 3H), 3.39 (m, 3H), 3.02 (m, 1H), 2.84 (m, 2H), 2.75 (m, 1H), 2.10 (m, 1H), 1.79 (m, 1H).

LC-MS: 314 ($M^+$)

Conditions for carrying out the liquid-chromatographic analysis in which the retention times described in the above reference examples were as follows:

Table 3

| LC measurement conditions: | |
|---|---|
| Column | Puresil™ 5 μC18 120A 150 × 4.6 mm |
| Flow rate | 1.0 ml/min |
| Measuring wavelength | 220, 280 nm |
| Mobile phase | liquid A : liquid B = 65 : 35 |
| Liquid A | methanol (for electronics industry) 5 mM sodium |
| Liquid B | heptanesulfonate-phosphoric acid (pH = 3) |

Example 1

4-(2-Bromo-5-methoxybenzyl)-1-(2-(4-piperidinyl)ethyl)piperidine dihydrochloride

1-1) tert-Butyl 4-(2-(4-(2-bromo-5-methoxybenzyl)piperidin-1-yl)2-oxoethyl)piperidine-1-carboxylate

Under a nitrogen atmosphere, triethylamine (5.74 g, 56.7 mmol) was added to a solution of 4-(2-bromo-5-methoxybenzyl)piperidine hydrochloride (15.15 g, 47.25 mmol), N-tert-butoxycarbonyl-4-piperidylacetic acid (12.07 g, 49.61 mmol), 1-hydroxybenzotriazole monohydrate (14.47 g, 94.5 mmol) and 1-ethyl-3-(3-dimethylaminopropyl)-carbodiimide hydrochloride (192 mg, 1.0 mmol) in dimethylformamide (150 ml) at room temperature, and the resulting mixture was stirred at room temperature for 2 hours and allowed to stand overnight at room temperature. The reaction mixture was poured into a mixture of water (1 L) and a 4N aqueous sodium hydroxide solution (10 ml) and extracted with ethyl acetate. The extract solution was washed successively with water, an aqueous ammonium chloride solution and a saturated aqueous sodium chloride solution, dried over magnesium sulfate, concentrated, and dried under reduced pressure to obtain 33.28 g of the title compound as a yellow viscous oil.

1-2) 4-(2-Bromo-5-methoxybenzyl)-1-(4-piperidinylacetyl)piperidine

Trifluoroacetic acid (50 ml) was added dropwise to a solution of tert-butyl 4-(2-(4-(2-bromo-5-methoxybenzyl)-1-piperidinyl)2-oxoethyl)piperidine-1-carboxylate (33.28 g) in dichloromethane (80 ml) over a period of 10 minutes, and the resulting mixture was stirred at room temperature for 2.5 hours. The reaction mixture was concentrated and a 0.36N aqueous sodium hydroxide solution was added thereto, followed by extraction with ethyl acetate. The extract was washed with a saturated aqueous sodium chloride solution, dried over magnesium sulfate and then concentrated to obtain 19.05 g of the title compound as a yellow viscous oil.

1-3) 4-(2-Bromo-5-methoxybenzyl)-1-(2-(4-piperidinyl)ethyl)piperidine dihydrochloride

Under a nitrogen atmosphere, tert-butyl 4-(2-(4-(2-bromo-5-methoxybenzyl)-1-piperidinyl)2-oxoethyl)piperidine-1-carboxylate (1.80 g) was dissolved in tetrahydrofuran (10 ml), and a solution of a borane•tetrahydrofuran complex in tetrahydrofuran (1M, 12 ml, 12 mmol) was added thereto at room temperature. After the resulting mixture was stirred at room temperature for 3 hours, methanol (5 ml) was carefully added thereto with stirring and the resulting mixture was heated under reflux for 1 hour. This mixture was concentrated under reduced pressure and the residue was separated with a 1N aqueous hydrochloric acid solution and a mixture of ethyl acetate and toluene (1 : 1). The aqueous layer was adjusted to pH 9 with an aqueous sodium hydroxide solution and then extracted with chloroform. The extract was dried over magnesium sulfate and concentrated under reduced pressure, and the residue was purified by a flash silica gel column chromatography (chloroform/methanol/triethylamine = 95/5/0 to 80/20/2) to obtain 4-(2-bromo-5-methoxybenzyl)-1-(2-(4-piperidinyl)ethyl)piperidine (959 mg) as an oil.

This compound was dissolved in dioxane, followed by adding thereto a 4N hydrogen chloride/1,4-dioxane solution, and the resulting mixture was concentrated. The residue was suspended in ethyl acetate and collected by filtration to obtain the title compound as white powder. Melting point: 278°C.

The following compounds of Examples 2 to 5 were produced in the same manner as in Example 1.

Examples 2 to 5

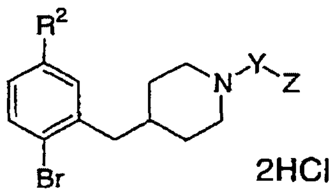

Table 4

| No. of Example | $R^2$ | Y | Z | Melting point (°C) |
|---|---|---|---|---|
| 2 | $OCH_3$ | $CH_2CH_2CH_2$ | 4-piperidyl | 209 |
| 3 | OiPr | $CH_2CH_2$ | 4-piperidyl | 288 |
| 4 | $OCH_3$ | $CH_2CH_2CH_2$ | 3-piperidyl | 274 |

Table 4 (continued)

| No. of Example | $R^2$ | Y | Z | Melting point (°C) |
|---|---|---|---|---|
| 5 | $OCH_3$ | $CH_2CH_2$ | 3-piperidyl | 254 |

Example 6

2-(4-(2-(4-(2-Bromo-5-methoxybenzyl)-1-piperidinyl)ethyl)-1-piperidinyl)ethanol dihydrochloride

The compound of Example 1 (150 mg, 0.32 mmol) was dissolved in N,N-dimethylformamide (5 ml), followed by adding thereto triethylamine (0.5 ml), potassium carbonate (powder, 200 mg) and 2-bromoethanol (200 mg) in that order, and the resulting mixture was stirred for 2 hours at a water bath temperature of 50°C. The reaction mixture was poured into water and extracted with ethyl acetate. The extract solution was washed with an aqueous sodium thiosulfate solution and then a saturated aqueous sodium chloride solution successively, dried over magnesium sulfate and then concentrated. The residue was dissolved in a mixture of methanol (5 ml) and toluene (10 ml), and a 4N-hydrogen chloride/1,4-dioxane solution (2 ml) was added thereto. The resulting mixture was concentrated and the resulting residue was dried under reduced pressure. Ethyl acetate was added thereto to obtain a suspension, and the suspension was treated with ultrasonic waves and then filtered. The precipitate was washed with ethyl acetate and dried under reduced pressure to obtain 93 mg of the title compound as white powdery crystals. Yield: 57%. Melting point: 271-273°C.
The following compounds of Examples 7 to 16 were produced in the same manner as in Example 6.

Examples 7 to 16

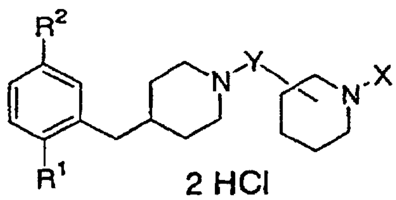

2 HCl

Table 5

| No. of Example | $R^1$ | $R^2$ | Y | X | Melting point (°C) |
|---|---|---|---|---|---|
| 7 | Br | $OCH_3$ | $CH_2CH_2CH_2$ (3-piperidyl) | ethyl | 249 |
| 8 | Br | OiPr | $CH_2CH_2$ (4-piperidyl) | 3-diethylaminopropyl | 280* |
| 9 | Br | $OCH_3$ | $CH_2CH_2$ (4-piperidyl) | isopropyl | 291 |
| 10 | Br | $OCH_3$ | $CH_2CH_2$ (4-piperidyl) | 4-hydroxybutyl | 268 |
| 11 | Cl | $OCH_3$ | $CH_2CH_2$ (4-piperidyl) | 2-methoxyethyl | 287 |
| 12 | Br | $OCH_3$ | $CH_2CH_2$ (4-piperidyl) | 3-methoxypropyl | >300 |
| 13 | Cl | $OCH_3$ | $CH_2CH_2$ (4-piperidyl) | 3-methoxypropyl | 293 |

* trihydrochloride

Table 5 (continued)

| No. of Example | $R^1$ | $R^2$ | Y | X | Melting point (°C) |
|---|---|---|---|---|---|
| 14 | Br | OEt | $CH_2CH_2$ (4-piperidyl) | 2-hydroxyethyl | 287 |
| 15 | Br | OEt | $CH_2CH_2$ (4-piperidyl) | 3-hydroxypropyl | 288 |
| 16 | Br | OiPr | $CH_2CH_2$ (4-piperidyl) | 2-hydroxyethyl | 295 |

Example 17

1-Propionyl-(3-(3-(4-(2-bromo-5-methoxybenzyl)-1-piperidinyl)propyl)piperidine hydrochloride

Under a nitrogen atmosphere, propionyl chloride (52 µl, 0.6 mmol) was added to a mixture of the compound of Example 4 (205 mg, 0.5 mmol), triethylamine (0.14 mL) and tetrahydrofuran (3 mL) under ice-cooling, and an ice bath was removed, followed by stirring for 2 hours. The reaction mixture was diluted with chloroform, washed with a saturated aqueous sodium hydrogencarbonate solution and then a saturated aqueous sodium chloride solution, and dried over anhydrous sodium sulfate. The solvent was distilled off under reduced pressure and the residue was purified by a flash silica gel column chromatography (chloroform/methanol 0 to 5 %) to obtain 1-propionyl-(3-(3-(4-(2-bromo-5-methoxybenzyl)piperidin-1-yl)propyl)piperidine (192 mg) as an oil. This compound was dissolved in tert-butyl methyl ether (4 ml), followed by adding thereto a 4N-hydrogen chloride/1,4-dioxane solution (0.38 ml), and the crystals precipitated were collected by filtration. The crystals were washed with tert-butyl methyl ether and dried under reduced pressure to obtain 155 mg of the title compound as white, powdery crystals. Melting point: 116°C.

The following compounds of Examples 18 to 30 were produced in the same manner as in Example 17.

Examples 18 to 30

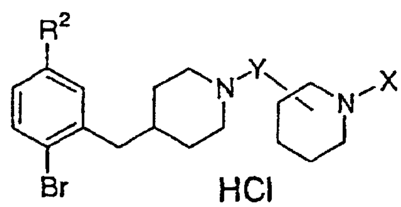

Table 6

| No. of Example | $R^2$ | Y | X | Melting point (°C) |
|---|---|---|---|---|
| 18 | OiPr | $CH_2CH_2CH_2$ (4-piperidyl) | acetyl | 137 |
| 19 | $OCH_3$ | $CH_2CH_2$ (4-piperidyl) | trifluoroacetyl | 187 |
| 20 | $OCH_3$ | $CH_2CH_2$ (4-piperidyl) | hydroxyacetyl | 202 |
| 21 | $OCH_3$ | $CH_2CH_2$ (4-piperidyl) | methoxycarbonyl | 249 |
| 22 | OiPr | $CH_2CH_2$ (4-piperidyl) | methoxycarbonyl | 204 |

Table 6 (continued)

| No. of Example | $R^2$ | Y | X | Melting point (°C) |
|---|---|---|---|---|
| 23 | $OCH_3$ | $CH_2CH_2$ (4-piperidyl) | dimethylcarbamoyl | 158 |
| 24 | OiPr | $CH_2CH_2$ (4-piperidyl) | dimethylcarbamoyl | 153 |
| 25 | $OCH_3$ | $CH_2CH_2CH_2$ (3-piperidyl) | methanesulfonyl | 158-166 |
| 26 | $OCH_3$ | $CH_2CH_2$ (4-piperidyl) | methanesulfonyl | 300 |
| 27 | $OCH_3$ | $CH_2CH_2$ (4-piperidyl) | ethanesulfonyl | 182 |
| 28 | OiPr | $CH_2CH_2$ (4-piperidyl) | ethanesulfonyl | 218 |
| 29 | $OCH_3$ | $CH_2CH_2$ (4-piperidyl) | dimethylsulfamoyl | 201 |
| 30 | OiPr | $CH_2CH_2$ (4-piperidyl) | dimethylsulfamoyl | 212 |

Example 31

4-(2-(4-(2-Bromo-5-isopropoxybenzyl)-1-piperidinyl)ethyl)N-ethyl-1-piperidinecarboxamide hydrochloride

Ethyl isocyanate (119 µl, about 1.5 mmol) was added to a mixture of the free form of the compound of Example 3 (150 mg, 0.3 mmol) and a solution of triethylamine (1 ml) in dichloromethane (5 ml) at room temperature. The resulting mixture was stirred at room temperature for 3 hours and then allowed to stand overnight. The reaction mixture was diluted with ethyl acetate, washed with water and then a saturated aqueous sodium chloride solution, dried over magnesium sulfate, and then concentrated under reduced pressure to obtain a brown oil. The brown oil was dissolved in methanol (10 ml), followed by adding thereto a 4N-hydrogen chloride/1,4-dioxane solution (1 ml), and the resulting mixture was concentrated under reduced pressure. The solid thus obtained was suspended in ethyl acetate and treated with ultrasonic waves, followed by filtration, washed with ethyl acetate and dried under reduced pressure. As a white powdery solid, 139 mg (87%) of the title compound was obtained. Melting point: 261°C.

The following compound of Example 32 was produced in the same manner as in Example 31.

Example 32

4-(2-(4-(2-Bromo-5-methoxybenzyl)piperidine-1-ylethyl)N-ethyl-1-piperidinecarboxamide hydrochloride

Melting point: 230°C.

Example 33

4-(2-Bromo-5-methoxybenzyl)-1-(2-(1-(2,2,2-trifluoroethyl)-4-piperidinyl)ethyl)piperidine dihydrochloride

The free form of the compound of Example 19 (200 mg) was dissolved in tetrahydrofuran (4 ml), followed by adding dropwise thereto a boranetetrahydrofuran complex (0.93 M, 5 ml, 4,7 mmol) at room temperature, and the resulting mixture was stirred at room temperature for 18 hours. Methanol (5 ml) was added thereto, and after foaming ceased, the solvent was distilled off. The residue was dissolved in methanol (5 ml) and the resulting solution was heated under reflux for 4 hours and then allowed to cool. The solvent was distilled off and the residue was purified by a silica gel column chromatography (chloroform/methanol = 10/1) to obtain 4-(2-bromo-5-methoxybenzyl)-1-(2-(1-(2,2,2-trifluoroethyl)pieridin-4-yl)ethyl)piperidine (152 mg) as a white solid. This compound was dissolved in ethyl acetate-isopropanol (3.5 ml/0.1 mL), followed by adding thereto a 4N-hydrogen chloride/1,4-dioxane solution (0.09 ml), and the crystals precipitated were collected by filtration, washed with ethyl acetate and then dried under reduced pressure. As a white powdery solid, 138 mg of the title compound was obtained. Melting point: 259°C.

Example 34

trans-4-(3-(4-(2-Bromo-5-methoxybenzyl)piperidin-1-yl)propyl)cyclohexanamine dihydrochloride

34-1) trans-tert-Butyl 4-(3-(4-(2-bromo-5-methoxybenzyl)piperidin-1-yl)3-oxopropyl)cyclohexylcarbamate

Under a nitrogen atmosphere, triethylamine (1.3 ml, 9.21 mmol) was added to a solution of 4-(2-bromo-5-methoxybenzyl)piperidine hydrochloride (1.18 g, 3.69 mmol), trans-3-(4-((tert-butoxycarbonyl)amino)cyclohexyl)propionic acid (1.0 g, 3.69 mmol), 1-hydroxybenzotriazole (0.50 g, 3.69 mmol) and 1-ethyl-3-(3-dimethylaminopropyl)-carbodiimide hydrochloride (0.85 mg, 4.42 mmol) in N,N-dimethylformamide (10 ml) at room temperature, and the resulting mixture was stirred at room temperature for 2 hours and allowed to stand overnight at room temperature. The reaction mixture was poured into water and extracted with toluene : ethyl acetate (1 : 1). The extract solution was washed successively with water, a 5% aqueous potassium hydrogensulfate solution, a saturated aqueous sodium hydrogencarbonate solution and a saturated aqueous sodium chloride solution, dried over magnesium sulfate and then concentrated. The concentrate was dried under reduced pressure to obtain 1.92 g of the title compound as a yellow viscous oil.

34-2) trans-4-(3-(4-(2-Bromo-5-methoxybenzyl)piperidin-1-yl)3-oxopropyl)cyclohexanamine

A 4N hydrogen chloride/1,4-dioxane solution (3.6 ml) was added to a solution of trans-tert-butyl 4-(3-(4-(2-bromo-5-methoxybenzyl)piperidin-1-yl)3-oxopropyl)cyclohexylcarbamate (1.92 g) in acetic acid (20 ml), and the resulting mixture was stirred at 50°C for 4 hours. A procedure of concentrating the reaction mixture, adding heptane thereto and concentrating the resulting mixture under reduced pressure was repeated three times to obtain 2.0 g of the title compound as a yellow viscous oil.

34-3) trans-4-(3-(4-(2-Bromo-5-methoxybenzyl)piperidin-1-yl)propyl)cyclohexanamine dihydrochloride

Under a nitrogen atmosphere, trans-4-(3-(4-(2-bromo-5-methoxybenzyl)piperidin-1-yl)3-oxopropyl)cyclohexanamine (2.00 g) was dissolved in tetrahydrofuran (21 ml), and a solution of a borane•tetrahydrofuran complex in tetrahydrofuran (1M, 21.4 ml, 21.4 mmol) was added thereto at room temperature. After the resulting mixture was stirred at room temperature for 3 hours, methanol (8 ml) was carefully added thereto with stirring and the resulting mixture was heated under reflux for 1 hour. This mixture was concentrated under reduced pressure and 6N hydrochloric acid (21 mL) and methanol (21 mL) were added to the residue, and the resulting mixture was stirred with heating at a bath temperature of 80°C for 4 hours. This mixture was distilled under reduced pressure to remove the volatile component, and the residue was adjusted to pH 11 with an aqueous sodium hydroxide solution and then extracted twice with ethyl acetate. The combined extract solution was washed with a saturated aqueous sodium chloride solution, dried over magnesium sulfate and then concentrated under reduced pressure to obtain trans-4-(3-(4-(2-bromo-5-methoxybenzyl)piperidin-1-yl)propyl)cyclohexanamine (1.07 g) as a white solid.

This compound was dissolved in dioxane, followed by adding thereto a 4N hydrogen chloride/1,4-dioxane solution, and the resulting mixture was concentrated. The residue was suspended in ethyl acetate and collected by filtration to obtain the title compound as white powder. Melting point: 214°C.

The following compounds of Examples 35 to 37 were produced in the same manner as in Example 34.

Example 35

cis-4-(3-(4-(2-Bromo-5-methoxybenzyl)piperidin-1-yl)propyl)cyclohexanamine dihydrochloride

Melting point: 238°C.

Example 36

trans-4-(2-(4-(2-Bromo-5-methoxybenzyl)piperidin-1-yl)ethyl)cyclohexanamine dihydrochloride

Example 37

cis-4-(2-(4-(2-Bromo-5-methoxybenzyl)piperidin-1-yl)ethyl)cyclohexanamine dihydrochloride

Example 38

trans-N-(4-(2-(4-(2-bromo-5-methoxybenzyl)piperidin-1-yl)ethyl)cyclohexyl)-N-ethylamine dihydrochloride

38-1) trans-N-(4-(2-(4-(2-bromo-5-methoxybenzyl)piperidin-1-yl)-2-oxoethyl)cyclohexyl)acetamide

Under a nitrogen atmosphere, triethylamine (1.2 ml, 8.75 mmol) was added to a solution of 4-(2-bromo-5-methoxybenzyl)piperidine hydrochloride (1.12 g, 3.5 mmol), trans-2-(4-(acetylamino)cyclohexyl)acetic acid (0.7 g, 3.5 mmol), 1-hydroxybenzotriazole (0.47 g, 3.5 mmol) and 1-ethyl-3-(3-dimethylaminopropyl)carbodiimide hydrochloride (0.81 mg, 4.2 mmol) in N,N-dimethylformamide (10 ml) at room temperature, and the resulting mixture was stirred at room temperature for 2 hours and allowed to stand overnight at room temperature. The reaction mixture was poured into water and extracted with toluene : ethyl acetate (1 : 1). The extract solution was washed successively with water, a 5% aqueous potassium hydrogensulfate solution, a saturated aqueous sodium hydrogencarbonate solution and a saturated aqueous sodium chloride solution, dried over magnesium sulfate and then concentrated. The concentrate was dried under reduced pressure to obtain 1.59 g of the title compound as a white solid.

38-2) trans-N-(4-(2-(4-(2-bromo-5-methoxybenzyl)piperidin-1-yl)ethyl)cyclohexyl)-N-ethylamine dihydrochloride

Under a nitrogen atmosphere, trans-N-(4-(2-(4-(2-bromo-5-methoxybenzyl)piperidin-1-yl)-2-oxoethyl)cyclohexyl)acetamide (1.57 g) was dissolved in tetrahydrofuran (17 ml), and a solution of a borane•tetrahydrofuran complex in tetrahydrofuran (1M, 17 ml, 17 mmol) was added thereto at room temperature. After the resulting mixture was stirred at room temperature for 3 hours, methanol (8 ml) was carefully added thereto with stirring and the resulting mixture was heated under reflux for 1 hour. This mixture was concentrated under reduced pressure and 6N hydrochloric acid (17 mL) and methanol (17 mL) were added to the residue, and the resulting mixture was stirred with heating at a bath temperature of 80°C for 4 hours. This mixture was distilled under reduced pressure to remove the volatile component, and the residue was adjusted to pH 11 with an aqueous sodium hydroxide solution and then extracted twice with ethyl acetate. The combined extract solution was washed with a saturated aqueous sodium chloride solution, dried over magnesium sulfate and then concentrated under reduced pressure to obtain trans-N-(4-(2-(4-(2-bromo-5-methoxybenzyl)piperidin-1-yl)ethyl)cyclohexyl)-N-ethylamine (1.44 g) as a white solid.

200 mg of this compound was dissolved in ethyl acetate, followed by adding thereto a 4N hydrogen chloride/1,4-dioxane solution (0.14 ml), and the solid precipitated was collected by filtration to obtain the title compound as white powder. Melting point: 285°C.

The following compounds of Examples 39 to 45 were produced in the same manner as in Example 38.

Example 39

cis-N-(4-(2-(4-(2-bromo-5-methoxybenzyl)piperidin-1-yl)ethyl)cyclohexyl)-N-ethylamine dihydrochloride

Example 40

trans-N-(4-(2-(4-(2-bromo-5-isopropoxybenzyl)piperidin-1-yl)ethyl)cyclohexyl)-N-ethylamine dihydrochloride

Melting point: 290°C.

Example 41

cis-N-(4-(2-(4-(2-bromo-5-isopropoxybenzyl)piperidin-1-yl)ethyl)cyclohexyl)-N-ethylamine dihydrochloride

Melting point: 247°C.

Example 42

cis-N-(4-(3-(4-(2-bromo-5-methoxybenzyl)piperidin-1-yl)propyl)cyclohexyl)-N-ethylamine dihydrochloride

Melting point: 234°C.

Example 43

trans-N-(4-(3-(4-(2-bromo-5-isopropoxybenzyl)piperidin-1-yl)propyl)cyclohexyl)-N-ethylamine dihydrochloride

Example 44

trans-4-(2-Bromo-5-isopropoxybenzyl)-1-(2-(4-methoxycyclohexyl)ethyl)piperidine hydrochloride

Melting point: 196°C.

Example 45

cis-4-(2-Bromo-5-isopropoxybenzyl)-1-(3-(4-methoxycyclohexyl)propyl)piperidine hydrochloride

Melting point: 134°C.

Example 46

trans-N-(4-(3-(4-(2-bromo-5-isopropoxybenzyl)piperidin-1-yl)propyl)cyclohexyl)-N-ethylmethanesulfonamide hydrochloride

Under a nitrogen atmosphere, methanesulfonyl chloride (19 μl, 0.25 mmol) was added to a mixture of the compound of Example 10 (99 mg, 0.2 mmol), triethylamine (43 μL) and N,N-dimethylformamide (1 mL), and the resulting mixture was stirred at 50°C for 5 hours. Triethylamine (43 μL) and methanesulfonyl chloride (19 μl, 0.25 mmol) were further added thereto, and the resulting mixture was stirred at 50°C for 5 hours. A 1N aqueous sodium hydroxide solution was added to the reaction mixture, followed by extraction (twice) with toluene-ethyl acetate (1 : 1). The combined organic layer was washed with a saturated aqueous sodium chloride solution and dried over anhydrous sodium sulfate. The solvent was distilled off under reduced pressure and the residue was purified by a flash silica gel column chromatography (chloroform/methanol 0 to 10%) to obtain trans-N-(4-(3-(4-(2-bromo-5-isopropoxybenzyl)piperidin-1-yl)propyl)cyclohexyl)-N-ethylmethanesulfonamide (87 mg) as an oil. This compound was dissolved in tert-butyl methyl ether (2 ml), followed by adding thereto a 4N-hydrogen chloride/1,4-dioxane solution (44 μl), and the crystals precipitated were filtered. The crystals were washed with tert-butyl methyl ether and dried under reduced pressure to obtain 75 mg of the title compound as white, powdery crystals. Melting point: 165°C.

The following compounds of Examples 47 to 53 were produced in the same manner as in Example 46.

Example 47

trans-N'-(4-(2-(4-(2-bromo-5-methoxybenzyl)piperidin-1-yl)ethyl)cyclohexyl)-N,N-dimethylsulfamide hydrochloride

Melting point: 214°C.

Example 48

trans-N-(4-(2-(4-(2-bromo-5-methoxybenzyl)piperidin-1-yl)ethyl)cyclohexyl)-2,2,2-trifluoro-acetamide hydrochloride

Melting point: 230°C.

Example 49

trans-N-(4-(2-(4-(2-bromo-5-methoxybenzyl)piperidin-1-yl)ethyl)cyclohexyl)-2-methoxyacetamide hydrochloride

Melting point: 132°C.

Example 50

cis-N-(4-(3-(4-(2-bromo-5-methoxybenzyl)piperidin-1-yl)propyl)cyclohexyl)-N-ethylacetamide hydrochloride

Example 51

trans-N-(4-(2-(4-(2-bromo-5-methoxybenzyl)piperidin-1-yl)ethyl)cyclohexyl)-N-ethylacetamide hydrochloride

Example 52

trans-N-(4-(2-(4-(2-bromo-5-isopropoxybenzyl)piperidin-1-yl)ethyl)cyclohexyl)-N-ethylacetamide hydrochloride

Example 53

trans-N-(4-(2-(4-(2-bromo-5-methoxybenzyl)piperidin-1-yl)ethyl)cyclohexyl)-2-hydroxyacetamide hydrochloride

Example 54

cis-N-(4-(3-(4-(2-bromo-5-methoxybenzyl)piperidin-1-yl)propyl)cyclohexyl)-N,N-diethylamine dihydrochloride

The compound of Example 50 (200 mg, 0.38 mmol) was dissolved in tetrahydrofuran (1.9 ml), and a solution of a borane•tetrahydrofuran complex in tetrahydrofuran (1M, 1.9 ml, 1.9 mmol) was added thereto at room temperature. After the resulting mixture was stirred at room temperature for 3 hours, methanol (8 ml) was carefully added thereto with stirring and the resulting mixture was heated under reflux for 1 hour. This mixture was concentrated under reduced pressure and a 6N aqueous hydrochloric acid solution (17 mL) and methanol (17 mL) were added to the residue, and the resulting mixture was stirred with heating at a bath temperature of 80°C for 4 hours. This mixture was distilled under reduced pressure to remove the volatile component, and the residue was adjusted to pH 11 with an aqueous sodium hydroxide solution and then extracted twice with ethyl acetate. The combined extract solution was washed with a saturated aqueous sodium chloride solution, dried over magnesium sulfate and then concentrated under reduced pressure, and the concentration residue was purified by a flash silica gel column chromatography (chloroform/methanol 0 to 20%) to obtain cis-N-(4-(3-(4-(2-bromo-5-methoxybenzyl)piperidin-1-yl)propyl)cyclohexyl)-N,N-diethylamine (142 mg) as an oil.

In ethyl acetate was dissolved 138 mg of the obtained compound, followed by adding thereto a 4N hydrogen chloride/1,4-dioxane solution (86 μl), and the solid precipitated was collected by filtration to obtain 82 mg of the title compound as white powder. Melting point: 219°C.

The following compounds of Examples 55 to 59 were produced in the same manner as in Example 54.

Example 55

trans-N-(4-(2-(4-(2-bromo-5-methoxybenzyl)piperidin-1-yl)ethyl)cyclohexyl)-N,N-diethylamine dihydrochloride

Melting point: 242°C.

Example 56

trans-N-(4-(2-(4-(2-bromo-5-isopropoxybenzyl)piperidin-1-yl)ethyl)cyclohexyl)-N,N-diethylamine dihydrochloride

Melting point: 253°C.

Example 57

trans-N-(4-(2-(4-(2-bromo-5-methoxybenzyl)piperidin-1-yl)ethyl)cyclohexyl)-N-ethyl-N-(2,2,2-trifluoroethyl)amine dihydrochloride

Melting point: 209°C.

Example 58

trans-N-(4-(2-(4-(2-bromo-5-methoxybenzyl)piperidin-1-yl)ethyl)cyclohexyl)-N-ethyl-N-(2-methoxyethyl)amine dihydrochloride

Melting point: 78°C.

Example 59

trans-N-(4-(2-(4-(2-bromo-5-methoxybenzyl)piperidin-1-yl)ethyl)cyclohexyl)-N-ethyl-N-(2-hydroxyethyl)amine dihydrochloride

Melting point: 259°C.

Example 60

2-(1-(2-(4-(2-Bromo-5-methoxybenzyl)piperidin-1-yl)ethyl)cyclohexyl)ethanol hydrochloride

60-1) (1-(2-(4-(2-Bromo-5-methoxybenzyl)piperidin-1-yl)-2-oxoethyl)cyclohexyl)acetic acid

Under a nitrogen atmosphere, triethylamine (0.52 ml, 3.75 mmol) was added to a solution of 4-(2-bromo-5-methoxybenzyl)piperidine hydrochloride (0.48 g, 1.5 mmol), 1,1-cyclohexanediacetic acid (0.6 g, 3.0 mmol), 1-hydroxybenzotriazole (0.20 g, 1.5 mmol) and 1-ethyl-3-(3-dimethylaminopropyl)-carbodiimide hydrochloride (0.35 mg, 1.8 mmol) in N,N-dimethyl-formamide (10 ml) at room temperature, and the resulting mixture was stirred at room temperature for 2 hours and allowed to stand overnight at room temperature. The reaction mixture was poured into water and extracted with toluene : ethyl acetate (1 : 1). The combined extract solution was washed with a saturated aqueous sodium chloride solution, dried over magnesium sulfate and then concentrated, and the residue was purified by a silica gel column chromatography (hexane/ethyl acetate = 3 : 1 to 1 : 1) to obtain (1-(2-(4-(2-bromo-5-methoxybenzyl)piperidin-1-yl)-2-oxoethyl)cyclohexyl)acetic acid (503 mg) as a white amorphous.

60-2) 2-(1-(2-(4-(2-Bromo-5-methoxybenzyl)piperidin-1-yl)ethyl)cyclohexyl)ethanol hydrochloride

Under a nitrogen atmosphere, (1-(2-(4-(2-bromo-5-methoxybenzyl)piperidin-1-yl)-2-oxoethyl)cyclohexyl)acetic acid (450 mg) was dissolved in tetrahydrofuran (4 ml), and a solution of a borane•tetrahydrofuran complex in tetrahydrofuran (1M, 3.86 ml, 3.86 mmol) was added thereto at room temperature. After the resulting mixture was stirred at room temperature for 3 hours, methanol (1.5 ml) was carefully added thereto with stirring and the resulting mixture was heated under reflux for 1 hour. This mixture was concentrated under reduced pressure and a 6N aqueous hydrochloric acid solution (4.5 mL) and methanol (4.5 mL) were added to the residue, and the resulting mixture was stirred with heating at a bath temperature of 80°C for 4 hours. This mixture was distilled under reduced pressure to remove the volatile component, and the residue was adjusted to pH 11 with an aqueous sodium hydroxide solution and then extracted twice with ethyl acetate. The combined extract solution was washed with a saturated aqueous sodium chloride solution, dried over magnesium sulfate and then concentrated under reduced pressure, and the residue was purified by a silica gel column chromatography (chloroform/methanol 0 to 10%) to obtain 2-(1-(2-(4-(2-bromo-5-methoxybenzyl)piperidin-1-yl)ethyl)cyclohexyl)ethanol (365 mg) as a white oil.

This compound was dissolved in ethyl acetate (8 ml), followed by adding thereto a 4N hydrogen chloride/1,4-dioxane solution (0.24 ml), and the solid precipitated was collected by filtration to obtain the title compound as white powder. Melting point: 187°C.

The following compounds of Examples 61 to 64 were produced in the same manner as in Example 60.

Example 61

2-(1-(2-(4-(2-Bromo-5-methoxybenzyl)piperidin-1-yl)ethyl)cyclopentyl)ethanol hydrochloride

Melting point: 184°C.

Example 62

cis-(4-(2-(4-(2-Bromo-5-methoxybenzyl)piperidin-1-yl)ethyl)cyclohexyl)methanol hydrochloride

Melting point: 209°C.

Example 63

cis-2-(4-(2-(4-(2-Bromo-5-methoxybenzyl)piperidin-1-yl)ethyl)cyclohexyl)ethanol hydrochloride

Melting point: 172°C.

Example 64

cis-2-(4-(2-(4-(2-Bromo-5-methoxybenzyl)piperidin-1-yl)ethyl)cyclohexyl)ethanol hydrochloride

Melting point: 273°C.

Example 65

2-((4-(2-Bromo-5-isopropoxybenzyl)piperidin-1-yl)methyl)-1,2,3,4-tetrahydroquinoline dihydrochloride

65-1) 2-((4-(2-Bromo-5-isopropoxybenzyl)piperidin-1-yl)carbonyl)-1,2,3,4-tetrahydroquinoline

Under a nitrogen atmosphere, triethylamine (0.42 ml, 3.0 mmol) was added to a solution of 4-(2-bromo-5-isopropoxybenzyl)piperidine hydrochloride (418 mg, 1.2 mmol), 1,2,3,4-tetrahydroquinoline-2-carboxylic acid (177 mg, 1.0 mmol), 1-hydroxybenzotriazole (135 mg, 1.0 mmol) and 1-ethyl-3-(3-dimethylaminopropyl)carbodiimide hydrochloride (249 mg, 1.2 mmol) in N,N-dimethylformamide (3 ml) at room temperature, and the resulting mixture was stirred at room temperature for 2 hours and allowed to stand overnight at room temperature. The reaction mixture was poured into water and extracted with toluene : ethyl acetate (1 : 1). The extract solution was washed successively with water, a 5% aqueous potassium hydrogensulfate solution, a saturated aqueous sodium hydrogencarbonate solution and a saturated aqueous sodium chloride solution, dried over magnesium sulfate and then concentrated. The concentrate was dried under reduced pressure to obtain 280 mg of the title compound as a yellow viscous oil.

65-2) 2-((4-(2-Bromo-5-isopropoxybenzyl)piperidin-1-yl)methyl)-1,2,3,4-tetrahydroquinoline dihydrochloride

Under a nitrogen atmosphere, 2-((4-(2-bromo-5-isopropoxybenzyl)piperidin-1-yl)carbonyl)-1,2,3,4-tetrahydroquinoline (280 mg) was dissolved in tetrahydrofuran (2 ml), and a solution of a borane•tetrahydrofuran complex in tetrahydrofuran (1M, 2.6 ml, 2.6 mmol) was added thereto at room temperature. After the resulting mixture was stirred at room temperature for 3 hours, methanol (0.6 ml) was carefully added thereto with stirring and the resulting mixture was heated under reflux for 1 hour. This mixture was concentrated under reduced pressure and a 6N hydrochloric acid (2.5 mL) and methanol (2.5 mL) were added to the residue, and the resulting mixture was stirred with heating at a bath temperature of 80°C for 4 hours. This mixture was distilled under reduced pressure to remove the volatile component, and the residue was adjusted to pH 11 with an aqueous sodium hydroxide solution and then extracted twice with ethyl acetate. The combined extract solution was washed with a saturated aqueous sodium chloride solution, dried over magnesium sulfate and then concentrated under reduced pressure, and the residue was purified by a flash silica gel column chromatography (hexane/ethyl acetate = 4/1 to 1/1) to obtain 2-((4-(2-bromo-5-isopropoxybenzyl)piperidin-1-yl)methyl)-1,2,3,4-tetrahydroquinoline (194 mg) as a white solid. This compound was dissolved in ethyl acetate, followed by adding thereto a 4N hydrogen chloride/1,4-dioxane solution, and the crystals precipitated were collected by filtration to obtain the title compound as white powder. Melting point: 224-226°C.

The following compounds of Examples 66 to 69 were produced in the same manner as in Example 65.

Example 66

2-((4-(2-Bromo-5-ethoxybenzyl)piperidin-1-yl)methyl)-1,2,3,4-tetrahydroquinoline dihydrochloride

Melting point: 228°C.

Example 67

2-((4-(2-Bromo-5-chlorobenzyl)piperidin-1-yl)methyl)-1,2,3,4-tetrahydroquinoline dihydrochloride

Melting point: 227°C.

Example 68

2-((4-(2-Bromo-5-fluorobenzyl)piperidin-1-yl)methyl)-1,2,3,4-tetrahydroquinoline dihydrochloride

Melting point: 245°C.

Example 69

2-((4-(2-Bromo-5-methoxybenzyl)piperidin-1-yl)methyl)-1,2,3,4-tetrahydroquinoline dihydrochloride

Example 70

3-((4-(2-Bromo-5-methoxybenzyl)piperidin-1-yl)methyl)-1,2,3,4-tetrahydroquinoline dihydrochloride 70-1)

tert-Butyl 3-((4-(2-bromo-5-methoxybenzyl)piperidin-1-yl)methyl)-1,2,3,4-tetrahydroquinoline-1-carboxylate

Under a nitrogen atmosphere, a solution of 4-(2-bromo-5-methoxybenzyl)piperidine hydrochloride (326 mg, 1.0 mmol), tert-butyl 3-bromomethyl-1,2,3,4-tetrahydroquinoline-1-carboxylate (385 mg, 1.2 mmol), potassium carbonate (552 mg, 4.0 mmol) and tetrabutylammonium hydrogensulfate (17 mg, 0.05 mmol) in acetonitrile (3 ml) was stirred at 60°C for 4 hours. After the reaction mixture was allowed to cool to room temperature, the inorganic material was removed by filtration and the filtrate was concentrated under reduced pressure. The residue was purified by a flash silica gel column chromatography (hexane/ethyl acetate = 5/1 to 1/1) to obtain the title compound (114 mg) as a white solid.

70-2) 3-((4-(2-Bromo-5-methoxybenzyl)piperidin-1-yl)methyl)-1,2,3,4-tetrahydroquinoline dihydrochloride

Under a nitrogen atmosphere, tert-butyl 3-((4-(2-bromo-5-methoxybenzyl)piperidin-1-yl)methyl)-1,2,3,4-tetrahydroquinoline-1-carboxylate (112 mg) was dissolved in acetic acid (0.8 ml), and a 4N hydrogen chloride/1,4-dioxane solution (0.2 ml) was added thereto at room temperature. The resulting mixture was stirred at 50°C for 1 hour and then concentrated under reduced pressure, followed by two runs of azeotropy with heptane. To the residue was added tert-butyl methyl ether and the resulting mixture was stirred with heating at a bath temperature of 60°C for 1 hour and then cooled to room temperature. The crystals precipitated were collected by filtration to obtain 86 mg of the title compound as white powder. Melting point: 235-237°C.

The following compound of Example 71 was produced in the same manner as in Example 70.

Example 71

3-((4-(2-Bromo-5-isopropoxybenzyl)piperidin-1-yl)methyl)-1,2,3,4-tetrahydroquinoline dihydrochloride

Example 72

5-(4-(2-Bromo-5-methoxybenzyl)piperidin-1-yl)methyl-3-(4-methoxyphenyl)oxazolidin-2-one hydrochloride

Under a nitrogen atmosphere, methanesulfonyl chloride (85 µl, 1.1 mmol) was added to a mixture of the compound of Example 12 (223 mg, 1.0 mmol), triethylamine (167 µl, 1.2 mmol) and dichloromethane (7 ml) at 0°C and stirred for 30 minutes. Water was added to the reaction mixture, followed by extraction with ethyl acetate. The extract was washed successively with a 5% aqueous potassium hydrogensulfate solution, a saturated aqueous sodium hydrogencarbonate solution and a saturated aqueous sodium chloride solution, dried over anhydrous magnesium sulfate and then concentrated to obtain a corresponding sulfonic ester.

The compound of Reference Example 1 (320 mg, 1.0 mmol) was added to a 2N aqueous sodium hydroxide solution, followed by extraction with diethyl ether, and the extract solution was dried over anhydrous potassium carbonate and concentrated to obtain a free compound corresponding to the compound of Reference Example 1. Potassium carbonate (346 mg, 2.5 mmol) was added to a solution of the free compound and the sulfonic ester obtained above in acetonitrile (5 m), and the resulting mixture was stirred at 50°C for 6 hours under a nitrogen atmosphere. The inorganic material was removed by filtration and the filtrate was concentrated under reduced pressure. The residue was purified by a flash silica gel column chromatography (chloroform/ethyl acetate = 3/1 to 1/1) to obtain 5-(4-(2-bromo-5-methoxybenzyl)piperidin-1-yl)methyl-3-(4-methoxyphenyl)oxazolidin-2-one (76 mg) as a white solid. This compound was dissolved in ethyl acetate, followed by adding thereto a 4N hydrogen chloride/1,4-dioxane solution, and the crystals precipitated were collected by filtration to obtain the title compound as white powder.

Melting point: 171-175°C.

Example 73

5-(4-(2-Bromo-5-methoxybenzyl)piperidin-1-yl)methyl-3-cyclohexyloxazolidin-2-one

73-1) 3-(4-(2-Bromo-5-methoxybenzyl)piperidin-1-yl)-1-cyclohexylaminopropan-2-ol

Sodium cyanoborohydride (314 mg, 5.00 mmol) was added to a solution of the compound of Reference Example 10 (430 mg, 1.00 mmol) and cyclohexanone (0.104 mL, 1.00 mmol) in dichloromethane (5 mL) at room temperature and stirred overnight. A one-normal aqueous sodium hydroxide solution was added to the reaction mixture, followed by extraction with ethyl acetate. The organic layer was washed with a saturated aqueous sodium chloride solution, dried over anhydrous magnesium sulfate and then concentrated under reduced pressure. Six-normal hydrochloric acid was added to the residue and stirred for 10 minutes, and the resulting mixture was made basic with a two-normal aqueous sodium hydroxide solution and then extracted with ethyl acetate. The organic layer was washed with a saturated aqueous sodium chloride solution, dried over anhydrous magnesium sulfate and then concentrated under reduced pressure. The residue was purified by a silica gel column chromatography (chloroform/methanol/aqueous ammonia = 1 : 0 : 0 ~ 10 : 1 : 0 ~ 10 : 1 : 0.1) to obtain 200 mg (46%) of the title compound as a colorless oil.

73-2) 5-(4-(2-Bromo-5-methoxybenzyl)piperidin-1-yl)methyl-3-cyclohexyloxazolidin-2-one

Triphosgene (55 mg, 0.19 mmol) was added to a solution of 3-(4-(2-bromo-5-methoxybenzyl)piperidin-1-yl)-1-cyclohexylaminopropan-2-ol (200 mg, 0.461 mmol) and triethylamine (0.083 mL, 0.60 mmol) in tetrahydrofuran (5 mL) at room temperature and stirred for 6 hours. A one-normal aqueous sodium hydroxide solution was added to the reaction mixture, followed by extraction with ethyl acetate. The organic layer was dried over anhydrous magnesium sulfate and concentrated under reduced pressure, and the residue was purified by a silica gel column chromatography (chloroform/methanol = 0 to 10%) to obtain 214 mg (100%) of the desired compound as a colorless oil.

$^1$H-NMR (300 MHz, $CDCl_3$)

δ 1.00-1.40 (7H, m), 1.55-1.75 (4H, m), 1.75-1.90 (4H, m), 2.03 (1H, td, J = 11.4, 2.1 Hz), 2.12 (1H, td, J = 11.5, 2.0 Hz), 2.53 (1H, dd, J = 13.4, 5.7 Hz), 2.61 (2H, d, J = 6.6 Hz), 2.62 (1H, dd, J = 13.3, 5.9 Hz), 2.83 (1H, d, J = 11.2 Hz), 2.93 (1H, d, J = 11.3 Hz), 3.24 (1H, dd, J = 8.5, 7.1 Hz), 3.54 (1H, t, J = 8.5 Hz), 3.60-3.70 (1H, m), 3.78 (3H, s), 4.55-4.65 (1H, m), 6.63 (1H, dd, J = 8.7, 3.0 Hz), 6.70 (1H, d, J = 2.9 Hz), 7.41 (1H, d, J = 8.6 Hz).

The following compounds may be produced in the same manner as in Example 6 or 17:

- 1-acetyl-4-(2-(4-(2-bromo-5-methoxybenzyl)-1-piperidinyl)ethyl)piperidine,
- 1-acetyl-4-(2-(4-(2-bromo-5-isopropoxybenzyl)-1-piperidinyl)ethyl)piperidine,
- 1-acetyl-4-(2-(4-(2-bromo-5-chlorobenzyl)-1-piperidinyl)ethyl)piperidine,
- 1-acetyl-4-(2-(4-(2-bromo-5-ethoxybenzyl)-1-piperidinyl)ethyl)piperidine,
- 1-acetyl-4-(2-(3-(2-bromo-5-methoxybenzyl)-1-pyrrolidinyl)ethyl)piperidine,
- 1-acetyl-4-(3-(4-(2-bromo-5-methoxybenzyl)-1-piperidinyl)propyl)piperidine,
- 1-acetyl-4-(3-(4-(2-bromo-5-isopropoxybenzyl)-1-piperidinyl)propyl)piperidine,
- 1-acetyl-4-(3-(4-(2-bromo-5-ethoxybenzyl)-1-piperidinyl)propyl)piperidine,
- 1-acetyl-4-(3-(4-(2-bromo-5-chlorobenzyl)-1-piperidinyl)propyl)piperidine,
- 1-acetyl-4-(3-(3-(2-bromo-5-methoxybenzyl)-1-pyrrolidinyl)propyl)piperidine,
- 1-acetyl-3-(2-(4-(2-bromo-5-methoxybenzyl)-1-piperidinyl)ethyl)piperidine,
- 1-acetyl-3-(2-(4-(2-bromo-5-isopropoxybenzyl)-1-piperidinyl)ethyl)piperidine,
- 1-acetyl-3-(2-(4-(2-bromo-5-ethoxybenzyl)-1-piperidinyl)ethyl)piperidine,
- 1-acetyl-3-(2-(4-(2-bromo-5-chlorobenzyl)-1-piperidinyl)ethyl)piperidine,
- 1-acetyl-3-(2-(3-(2-bromo-5-methoxybenzyl)-1-pyrrolidinyl)ethyl)piperidine,
- 1-acetyl-3-(3-(4-(2-bromo-5-methoxybenzyl)-1-piperidinyl)propyl)piperidine,
- 1-acetyl-3-(3-(4-(2-bromo-5-isopropoxybenzyl)-1-piperidinyl)propyl)piperidine,

- 1-acetyl-3-(3-(4-(2-bromo-5-chlorobenzyl)-1-piperidinyl)propyl)piperidine,
- 1-acetyl-3-(3-(4-(2-bromo-5-ethoxybenzyl)-1-piperidinyl)propyl)piperidine,
- 1-acetyl-3-(3-(3-(2-bromo-5-methoxybenzyl)-1-pyrrolidinyl)propyl)piperidine,
- 4-(2-bromo-5-methoxybenzyl)-1-(3-(1-ethyl-4-piperidinyl)propyl)piperidine,
- 4-(2-bromo-5-methoxybenzyl)-1-(2-(1-ethyl-3-piperidinyl)ethyl)piperidine,
- 4-(2-bromo-5-methoxybenzyl)-1-(2-(1-methyl-4-piperidinyl)ethyl)piperidine,
- 4-(2-bromo-5-methoxybenzyl)-1-(3-(1-methyl-4-piperidinyl)propyl)piperidine,
- 4-(2-bromo-5-methoxybenzyl)-1-(2-(1-(2-methoxyethyl)-4-piperidinyl)ethyl)piperidine,
- 4-(2-bromo-5-methoxybenzyl)-1-(3-(1-(2-methoxyethyl)-4-piperidinyl)propyl)piperidine,
- 4-(2-bromo-5-methoxybenzyl)-1-(2-(1-(2-methoxyethyl)-3-piperidinyl)ethyl)piperidine,
- 4-(2-bromo-5-methoxybenzyl)-1-(3-(1-(2-methoxyethyl)-3-piperidinyl)propyl)piperidine,
- 4-(2-bromo-5-methoxybenzyl)-1-(3-(1-(2,2,2-trifluoroethyl)-4-piperidinyl)propyl)piperidine,
- 4-(2-bromo-5-methoxybenzyl)-1-(2-(1-(2,2,2-trifluoroethyl)-3-piperidinyl)ethyl)piperidine,
- 4-(2-bromo-5-methoxybenzyl)-1-(3-(1-(2,2,2-trifluoroethyl)-3-piperidinyl)propyl)piperidine,
- 1-propionyl-4-(2-(4-(2-bromo-5-methoxybenzyl)-1-piperidinyl)ethyl)piperidine,
- 1-propionyl-4-(3-(4-(2-bromo-5-methoxybenzyl)-1-piperidinyl) propyl) piperidine,
- 1-propionyl-3-(2-(4-(2-bromo-5-methoxybenzyl)-1-piperidinyl)ethyl)piperidine,
- 1-propionyl-3-(3-(4-(2-bromo-5-methoxybenzyl)-1-piperidinyl)propyl)piperidine,
- 1-hydroxyacetyl-4-(3-(4-(2-bromo-5-methoxybenzyl)-1-piperidinyl)propyl)piperidine,
- 1-hydroxyacetyl-3-(2-(4-(2-bromo-5-methoxybenzyl)-1-piperidinyl)ethyl)piperidine,
- 1-hydroxyacetyl-3-(3-(4-(2-bromo-5-methoxybenzyl)-1-piperidinyl)propyl)piperidine,
- 1-methoxyacetyl-4-(2-(4-(2-bromo-5-methoxybenzyl)-1-piperidinyl)ethyl)piperidine,
- 1-methoxyacetyl-4-(3-(4-(2-bromo-5-methoxybenzyl)-1-piperidinyl)propyl)piperidine,
- 1-methoxyacetyl-3-(2-(4-(2-bromo-5-methoxybenzyl)-1-piperidinyl)ethyl)piperidine,
- 1-methoxyacetyl-3-(3-(4-(2-bromo-5-methoxybenzyl)-1-piperidinyl)propyl)piperidine,
- 1-(2,2,2-trifluoroacetyl)-4-(2-(4-(2-bromo-5-methoxybenzyl)-1-piperidinyl)ethyl)piperidine,
- 1-(2,2,2-trifluoroacetyl)-4-(3-(4-(2-bromo-5-methoxybenzyl)-1-piperidinyl)propyl)piperidine,
- 1-(2,2,2-trifluoroacetyl)-3-(2-(4-(2-bromo-5-methoxybenzyl)-1-piperidinyl)ethyl)piperidine,
- 1-(2,2,2-trifluoroacetyl)-3-(3-(4-(2-bromo-5-methoxybenzyl)-1-piperidinyl)propyl)piperidine,
- 4-(2-bromo-5-methoxybenzyl)-1-(3-(1-methylsulfonyl-4-piperidinyl)propyl)piperidine,
- 4-(2-bromo-5-methoxybenzyl)-1-(2-(1-methylsulfonyl-3-piperidinyl)ethyl)piperidine,
- 4-(3-(4-(2-bromo-5-methoxybenzyl)-1-piperidinyl)propyl)-N-ethyl-1-piperidinecarboxamide,
- 3-(2-(4-(2-bromo-5-methoxybenzyl)-1-piperidinyl)ethyl)-N-ethyl-1-piperidinecarboxamide,
- 3-(3-(4-(2-bromo-5-methoxybenzyl)-1-piperidinyl)propyl)-N-ethyl-1-piperidinecarboxamide,
- 4-(2-(4-(2-bromo-5-methoxybenzyl)-1-piperidinyl)ethyl)-1-piperidinecarboxamide,
- 4-(3-(4-(2-bromo-5-methoxybenzyl)-1-piperidinyl)propyl)-1-piperidinecarboxamide,
- 3-(2-(4-(2-bromo-5-methoxybenzyl)-1-piperidinyl)ethyl)-1-piperidinecarboxamide,
- 3-(3-(4-(2-bromo-5-methoxybenzyl)-1-piperidinyl)propyl)-1-piperidinecarboxamide,
- 4-(3-(4-(2-bromo-5-methoxybenzyl)-1-piperidinyl)propyl)-N,N-dimethyl-1-piperidinecarboxamide,
- 3-(2-(4-(2-bromo-5-methoxybenzyl)-1-piperidinyl)ethyl)-N,N-dimethyl-1-piperidinecarboxamide,
- 3-(3-(4-(2-bromo-5-methoxybenzyl)-1-piperidinyl)propyl)-N,N-dimethyl-1-piperidinecarboxamide,
- methyl 4-(3-(4-(2-bromo-5-methoxybenzyl)-1-piperidinyl)propyl)-1-piperidinecarboxylate,
- methyl 3-(2-(4-(2-bromo-5-methoxybenzyl)-1-piperidinyl)ethyl)-1-piperidinecarboxylate,
- methyl 3-(3-(4-(2-bromo-5-methoxybenzyl)-1-piperidinyl)propyl)-1-piperidinecarboxylate,
- 4-(3-(4-(2-bromo-5-methoxybenzyl)-1-piperidinyl)propyl)-N,N-dimethyl-1-piperidinesulfonamide,
- 3-(2-(4-(2-bromo-5-methoxybenzyl)-1-piperidinyl)ethyl)-N,N-dimethyl-1-piperidinesulfonamide,
- 3-(3-(4-(2-bromo-5-methoxybenzyl)-1-piperidinyl)propyl)-N,N-dimethyl-1-piperidinesulfonamide,
- 4-(2-(4-(2-bromo-5-methoxybenzyl)-1-piperidinyl)ethyl)-1-piperidinecarboximidamide,
- 4-(3-(4-(2-bromo-5-methoxybenzyl)-1-piperidinyl)propyl)-1-piperidinecarboximidamide,
- 3-(2-(4-(2-bromo-5-methoxybenzyl)-1-piperidinyl)ethyl)-1-piperidinecarboximidamide,
- 3-(3-(4-(2-bromo-5-methoxybenzyl)-1-piperidinyl)propyl)-1-piperidinecarboximidamide,
- 3-(4-(2-(4-(2-bromo-5-methoxybenzyl)-1-piperidinyl)ethyl)-1-piperidinyl)-N,N-diethyl-3-oxo-1-propanamine,
- 3-(4-(3-(4-(2-bromo-5-methoxybenzyl)-1-piperidinyl)propyl)-1-piperidinyl)-N,N-diethyl-3-oxo-1-propanamine,
- 3-(3-(2-(4-(2-bromo-5-methoxybenzyl)-1-piperidinyl)ethyl)-1-piperidinyl)-N,N-diethyl-3-oxo-1-propanamine,
- 3-(3-(3-(4-(2-bromo-5-methoxybenzyl)-1-piperidinyl)propyl)-1-piperidinyl)-N,N-diethyl-3-oxo-1-propanamine,
- 3-(4-(2-(4-(2-bromo-5-methoxybenzyl)-1-piperidinyl)ethyl)-1-piperidinyl)-N,N-diethyl-1-propanamine,
- 3-(4-(3-(4-(2-bromo-5-methoxybenzyl)-1-piperidinyl)propyl)-1-piperidinyl)-N,N-diethyl-1-propanamine,
- 3-(3-(2-(4-(2-bromo-5-methoxybenzyl)-1-piperidinyl)ethyl)-1-piperidinyl)-N,N-diethyl-1-propanamine,

- 3-(3-(3-(4-(2-bromo-5-methoxybenzyl)-1-piperidinyl)propyl)-1-piperidinyl)-N,N-diethyl-1-propanamine,
- 1-propionyl-4-(2-(4-(2-bromo-5-isopropoxybenzyl)-1-piperidinyl)ethyl)piperidine,
- 1-propionyl-4-(3-(4-(2-bromo-5-isopropoxybenzyl)-1-piperidinyl)propyl)piperidine,
- 1-propionyl-3-(2-(4-(2-bromo-5-isopropoxybenzyl)-1-piperidinyl)ethyl)piperidine,
- 1-propionyl-3-(3-(4-(2-bromo-5-isopropoxybenzyl)-1-piperidinyl)propyl)piperidine,
- 1-hydroxyacetyl-4-(3-(4-(2-bromo-5-isopropoxybenzyl)-1-piperidinyl)propyl)piperidine,
- 1-hydroxyacetyl-3-(2-(4-(2-bromo-5-isopropoxybenzyl)-1-piperidinyl)ethyl)piperidine,
- 1-hydroxyacetyl-3-(3-(4-(2-bromo-5-isopropoxybenzyl)-1-piperidinyl)propyl)piperidine,
- 1-methoxyacetyl-4-(2-(4-(2-bromo-5-isopropoxybenzyl)-1-piperidinyl)ethyl)piperidine,
- 1-methoxyacetyl-4-(3-(4-(2-bromo-5-isopropoxybenzyl)-1-piperidinyl)propyl)piperidine,
- 1-methoxyacetyl-3-(2-(4-(2-bromo-5-isopropoxybenzyl)-1-piperidinyl)ethyl)piperidine,
- 1-methoxyacetyl-3-(3-(4-(2-bromo-5-isopropoxybenzyl)-1-piperidinyl)propyl)piperidine,
- 1-(2,2,2-trifluoroacetyl)-4-(2-(4-(2-bromo-5-isopropoxybenzyl)-1-piperidinyl)ethyl)piperidine,
- 1-(2,2,2-trifluoroacetyl)-4-(3-(4-(2-bromo-5-isopropoxybenzyl)-1-piperidinyl)propyl)piperidine,
- 1-(2,2,2-trifluoroacetyl)-3-(2-(4-(2-bromo-5-isopropoxybenzyl)-1-piperidinyl)ethyl)piperidine,
- 1-(2,2,2-trifluoroacetyl)-3-(3-(4-(2-bromo-5-isopropoxybenzyl)-1-piperidinyl)propyl)piperidine,
- 4-(2-bromo-5-isopropoxybenzyl)-1-(3-(1-methylsulfonyl-4-piperidinyl)propyl)piperidine,
- 4-(2-bromo-5-isopropoxybenzyl)-1-(2-(1-methylsulfonyl-3-piperidinyl)ethyl)piperidine,
- 4-(3-(4-(2-bromo-5-isopropoxybenzyl)-1-piperidinyl)propyl)-N-ethyl-1-piperidinecarboxamide,
- 3-(2-(4-(2-bromo-5-isopropoxybenzyl)-1-piperidinyl)ethyl)-N-ethyl-1-piperidinecarboxamide,
- 3-(3-(4-(2-bromo-5-isopropoxybenzyl)-1-piperidinyl)propyl)-N-ethyl-1-piperidinecarboxamide,
- 4-(2-(4-(2-bromo-5-isopropoxybenzyl)-1-piperidinyl)ethyl)-1-piperidinecarboxamide,
- 4-(3-(4-(2-bromo-5-isopropoxybenzyl)-1-piperidinyl)propyl)-1-piperidinecarboxamide,
- 3-(2-(4-(2-bromo-5-isopropoxybenzyl)-1-piperidinyl)ethyl)-1-piperidinecarboxamide,
- 3-(3-(4-(2-bromo-5-isopropoxybenzyl)-1-piperidinyl)propyl)-1-piperidinecarboxamide,
- 4-(3-(4-(2-bromo-5-isopropoxybenzyl)-1-piperidinyl)propyl)-N,N-dimethyl-1-piperidinecarboxamide,
- 3-(2-(4-(2-bromo-5-isopropoxybenzyl)-1-piperidinyl)ethyl)-N,N-dimethyl-1-piperidinecarboxamide,
- 3-(3-(4-(2-bromo-5-isopropoxybenzyl)-1-piperidinyl)propyl)-N,N-dimethyl-1-piperidinecarboxamide,
- methyl 4-(3-(4-(2-bromo-5-isopropoxybenzyl)-1-piperidinyl)propyl)-1-piperidinecarboxylate,
- methyl 3-(2-(4-(2-bromo-5-isopropoxybenzyl)-1-piperidinyl)ethyl)-1-piperidinecarboxylate,
- methyl 3-(3-(4-(2-bromo-5-isopropoxybenzyl)-1-piperidinyl)propyl)-1-piperidinecarboxylate,
- 4-(3-(4-(2-bromo-5-isopropoxybenzyl)-1-piperidinyl)propyl)-N,N-dimethyl-1-piperidinesulfonamide,
- 3-(2-(4-(2-bromo-5-isopropoxybenzyl)-1-piperidinyl)ethyl)-N,N-dimethyl-1-piperidinesulfonamide,
- 3-(3-(4-(2-bromo-5-isopropoxybenzyl)-1-piperidinyl)propyl)-N,N-dimethyl-1-piperidinesulfonamide,
- 4-(2-(4-(2-bromo-5-isopropoxybenzyl)-1-piperidinyl)ethyl)-1-piperidinecarboximidamide,
- 4-(3-(4-(2-bromo-5-isopropoxybenzyl)-1-piperidinyl)propyl)-1-piperidinecarboximidamide,
- 3-(2-(4-(2-bromo-5-isopropoxybenzyl)-1-piperidinyl)ethyl)-1-piperidinecarboximidamide,
- 3-(3-(4-(2-bromo-5-isopropoxybenzyl)-1-piperidinyl)propyl)-1-piperidinecarboximidamide,
- 1-propionyl-4-(2-(4-(2-bromo-5-chlorobenzyl)-1-piperidinyl)ethyl)piperidine,
- 1-propionyl-4-(3-(4-(2-bromo-5-chlorobenzyl)-1-piperidinyl)propyl)piperidine,
- 1-propionyl-3-(2-(4-(2-bromo-5-chlorobenzyl)-1-piperidinyl)ethyl)piperidine,
- 1-propionyl-3-(3-(4-(2-bromo-5-chlorobenzyl)-1-piperidinyl)propyl)piperidine,
- 1-hydroxyacetyl-4-(3-(4-(2-bromo-5-chlorobenzyl)-1-piperidinyl)propyl)piperidine,
- 1-hydroxyacetyl-3-(2-(4-(2-bromo-5-chlorobenzyl)-1-piperidinyl)ethyl)piperidine,
- 1-hydroxyacetyl-3-(3-(4-(2-bromo-5-chlorobenzyl)-1-piperidinyl)propyl)piperidine,
- 1-methoxyacetyl-4-(2-(4-(2-bromo-5-chlorobenzyl)-1-piperidinyl)ethyl)piperidine,
- 1-methoxyacetyl-4-(3-(4-(2-bromo-5-chlorobenzyl)-1-piperidinyl)propyl)piperidine,
- 1-methoxyacetyl-3-(2-(4-(2-bromo-5-chlorobenzyl)-1-piperidinyl)ethyl)piperidine,
- 1-methoxyacetyl-3-(3-(4-(2-bromo-5-chlorobenzyl)-1-piperidinyl)propyl)piperidine,
- 1-(2,2,2-trifluoroacetyl)-4-(2-(4-(2-bromo-5-chlorobenzyl)-1-piperidinyl)ethyl)piperidine,
- 1-(2,2,2-trifluoroacetyl)-4-(3-(4-(2-bromo-5-chlorobenzyl)-1-piperidinyl)propyl)piperidine,
- 1-(2,2,2-trifluoroacetyl)-3-(2-(4-(2-bromo-5-chlorobenzyl)-1-piperidinyl)ethyl)piperidine
- 1-(2,2,2-trifluoroacetyl)-3-(3-(4-(2-bromo-5-chlorobenzyl)-1-piperidinyl)propyl)piperidine
- 4-(2-bromo-5-chlorobenzyl)-1-(3-(1-methylsulfonyl-4-piperidinyl)propyl)piperidine,
- 4-(2-bromo-5-chlorobenzyl)-1-(2-(1-methylsulfonyl-3-piperidinyl)ethyl)piperidine,
- 4-(3-(4-(2-bromo-5-chlorobenzyl)-1-piperidinyl)propyl)-N-ethyl-1-piperidinecarboxamide,
- 3-(2-(4-(2-bromo-5-chlorobenzyl)-1-piperidinyl)ethyl)-N-ethyl-1-piperidinecarboxamide,
- 3-(3-(4-(2-bromo-5-chlorobenzyl)-1-piperidinyl)propyl)-N-ethyl-1-piperidinecarboxamide,

- 4-(2-(4-(2-bromo-5-chlorobenzyl)-1-piperidinyl)ethyl)-1-piperidinecarboxamide,
- 4-(3-(4-(2-bromo-5-chlorobenzyl)-1-piperidinyl)propyl)-1-piperidinecarboxamide,
- 3-(2-(4-(2-bromo-5-chlorobenzyl)-1-piperidinyl)ethyl)-1-piperidinecarboxamide,
- 3-(3-(4-(2-bromo-5-chlorobenzyl)-1-piperidinyl)propyl)-1-piperidinecarboxamide,
- 4-(3-(4-(2-bromo-5-chlorobenzyl)-1-piperidinyl)propyl)-N,N-dimethyl-1-piperidinecarboxamide,
- 3-(2-(4-(2-bromo-5-chlorobenzyl)-1-piperidinyl)ethyl)-N,N-dimethyl-1-piperidinecarboxamide,
- 3-(3-(4-(2-bromo-5-chlorobenzyl)-1-piperidinyl)propyl)-N,N-dimethyl-1-piperidinecarboxamide,
- methyl 4-(3-(4-(2-bromo-5-chlorobenzyl)-1-piperidinyl)propyl)-1-piperidinecarboxylate,
- methyl 3-(2-(4-(2-bromo-5-chlorobenzyl)-1-piperidinyl)ethyl)-1-piperidinecarboxylate,
- methyl 3-(3-(4-(2-bromo-5-chlorobenzyl)-1-piperidinyl)propyl)-1-piperidinecarboxylate,
- 4-(3-(4-(2-bromo-5-chlorobenzyl)-1-piperidinyl)propyl)-N,N-dimethyl-1-piperidinesulfonamide,
- 3-(2-(4-(2-bromo-5-chlorobenzyl)-1-piperidinyl)ethyl)-N,N-dimethyl-1-piperidinesulfonamide,
- 3-(3-(4-(2-bromo-5-chlorobenzyl)-1-piperidinyl)propyl)-N,N-dimethyl-1-piperidinesulfonamide,
- 4-(2-(4-(2-bromo-5-chlorobenzyl)-1-piperidinyl)ethyl)-1-piperidinecarboximidamide,
- 4-(3-(4-(2-bromo-5-chlorobenzyl)-1-piperidinyl)propyl)-1-piperidinecarboximidamide,
- 3-(2-(4-(2-bromo-5-chlorobenzyl)-1-piperidinyl)ethyl)-1-piperidinecarboximidamide,
- 3-(3-(4-(2-bromo-5-chlorobenzyl)-1-piperidinyl)propyl)-1-piperidinecarboximidamide,
- 1-propionyl-4-(2-(3-(2-bromo-5-methoxybenzyl)-1-pyrrolidinyl)ethyl)piperidine,
- 1-propionyl-4-(3-(3-(2-bromo-5-methoxybenzyl)-1-pyrrolidinyl)propyl)piperidine,
- 1-propionyl-3-(2-(3-(2-bromo-5-methoxybenzyl)-1-pyrrolidinyl)ethyl)piperidine,
- 1-propionyl-3-(3-(3-(2-bromo-5-methoxybenzyl)-1-pyrrolidinyl)propyl)piperidine,
- 1-hydroxyacetyl-4-(3-(3-(2-bromo-5-methoxybenzyl)-1-pyrrolidinyl)propyl)piperidine,
- 1-hydroxyacetyl-3-(2-(3-(2-bromo-5-methoxybenzyl)-1-pyrrolidinyl)ethyl)piperidine,
- 1-hydroxyacetyl-3-(3-(3-(2-bromo-5-methoxybenzyl)-1-pyrrolidinyl)propyl)piperidine,
- 1-methoxyacetyl-4-(2-(3-(2-bromo-5-methoxybenzyl)-1-pyrrolidinyl)ethyl)piperidine,
- 1-methoxyacetyl-4-(3-(3-(2-bromo-5-methoxybenzyl)-1-pyrrolidinyl)propyl)piperidine,
- 1-methoxyacetyl-3-(2-(3-(2-bromo-5-methoxybenzyl)-1-pyrrolidinyl)ethyl)piperidine,
- 1-methoxyacetyl-3-(3-(3-(2-bromo-5-methoxybenzyl)-1-pyrrolidinyl)propyl)piperidine,
- 1-(2,2,2-trifluoroacetyl)-4-(2-(3-(2-bromo-5-methoxybenzyl)-1-pyrrolidinyl)ethyl)piperidine,
- 1-(2,2,2-trifluoroacetyl)-4-(3-(3-(2-bromo-5-methoxybenzyl)-1-pyrrolidinyl)propyl)piperidine
- 1-(2,2,2-trifluoroacetyl)-3-(2-(3-(2-bromo-5-methoxybenzyl)-1-pyrrolidinyl)ethyl)piperidine
- 1-(2,2,2-trifluoroacetyl)-3-(3-(3-(2-bromo-5-methoxybenzyl)-1-pyrrolidinyl)propyl)piperidine
- 4-(2-bromo-5-chlorobenzyl)-1-(3-(3-(2-bromo-5-methoxybenzyl)-1-pyrrolidinyl)propyl)piperidine,
- 3-(2-bromo-5-methoxybenzyl)-1-(2-(1-methylsulfonyl-3-piperidinyl)ethyl)pyrrolidine,
- 4-(3-(3-(2-bromo-5-methoxybenzyl)-1-pyrrolidinyl)propyl)-N-ethyl-1-piperidinecarboxamide,
- 3-(2-(3-(2-bromo-5-methoxybenzyl)-1-pyrrolidinyl)ethyl)-N-ethyl-1-piperidinecarboxamide,
- 3-(3-(3-(2-bromo-5-methoxybenzyl)-1-pyrrolidinyl)propyl)-N-ethyl-1-piperidinecarboxamide,
- 4-(2-(3-(2-bromo-5-methoxybenzyl)-1-pyrrolidinyl)ethyl)-1-piperidinecarboxamide,
- 4-(3-(3-(2-bromo-5-methoxybenzyl)-1-pyrrolidinyl)propyl)-1-piperidinecarboxamide,
- 3-(2-(3-(2-bromo-5-methoxybenzyl)-1-pyrrolidinyl)ethyl)-1-piperidinecarboxamide,
- 3-(3-(3-(2-bromo-5-methoxybenzyl)-1-pyrrolidinyl)propyl)-1-piperidinecarboxamide,
- 4-(3-(3-(2-bromo-5-methoxybenzyl)-1-pyrrolidinyl)propyl)-N,N-dimethyl-1-piperidinecarboxamide,
- 3-(2-(3-(2-bromo-5-methoxybenzyl)-1-pyrrolidinyl)ethyl)-N,N-dimethyl-1-piperidinecarboxamide,
- 3-(3-(3-(2-bromo-5-methoxybenzyl)-1-pyrrolidinyl)propyl)-N,N-dimethyl-1-piperidinecarboxamide,
- methyl 4-(3-(3-(2-bromo-5-methoxybenzyl)-1-pyrrolidinyl)propyl)-1-piperidinecarboxylate,
- methyl 3-(2-(3-(2-bromo-5-methoxybenzyl)-1-pyrrolidinyl)ethyl)-1-piperidinecarboxylate,
- methyl 3-(3-(3-(2-bromo-5-methoxybenzyl)-1-pyrrolidinyl)propyl)-1-piperidinecarboxylate,
- 4-(3-(4-(3-(2-bromo-5-methoxybenzyl)-1-pyrrolidinyl)propyl)-N,N-dimethyl-1-piperidinesulfonamide,
- 3-(2-(3-(2-bromo-5-methoxybenzyl)-1-pyrrolidinyl)ethyl)-N,N-dimethyl-1-piperidinesulfonamide,
- 3-(3-(3-(2-bromo-5-methoxybenzyl)-1-pyrrolidinyl)propyl)-N,N-dimethyl-1-piperidinesulfonamide,
- 4-(2-(3-(2-bromo-5-methoxybenzyl)-1-pyrrolidinyl)ethyl)-1-piperidinecarboximidamide,
- 4-(3-(3-(2-bromo-5-methoxybenzyl)-1-pyrrolidinyl)propyl)-1-piperidinecarboximidamide,
- 3-(2-(3-(2-bromo-5-methoxybenzyl)-1-pyrrolidinyl)ethyl)-1-piperidinecarboximidamide,
- 3-(3-(3-(2-bromo-5-methoxybenzyl)-1-pyrrolidinyl)propyl)-1-piperidinecarboximidamide,
- 1-propionyl-4-(2-(3-(2-bromo-5-methoxyethoxybenzyl)1-pyrrolidinyl)ethyl)piperidine,
- 1-hydroxyacetyl-4-(3-(3-(2-bromo-5-methoxyethoxybenzyl)-1-pyrrolidinyl)propyl)piperidine,
- 1-methoxyacetyl-4-(2-(3-(2-bromo-5-methoxyethoxybenzyl)-1-pyrrolidinyl)ethyl)piperidine
- 1-(2,2,2-trifluoroacetyl)-4-(2-(3-(2-bromo-5-methoxyethoxybenzyl)-1-pyrrolidinyl)ethyl)piperidine,

- 3-(2-bromo-5-methoxyethoxybenzyl)-1-(2-(1-methylsulfonyl-3-piperidinyl)ethyl)pyrrolidine,
- 4-(3-(3-(2-bromo-5-methoxyethoxybenzyl)-1-pyrrolidinyl)propyl)-N-ethyl-1-piperidinecarboxamide,
- 4-(2-(3-(2-bromo-5-methoxyethoxybenzyl)-1-pyrrolidinyl)ethyl)-1-piperidinecarboxamide,
- 4-(3-(3-(2-bromo-5-methoxyethoxybenzyl)-1-pyrrolidinyl)propyl)-N,N-dimethyl-1-piperidinecarboxamide,
- methyl 4-(3-(3-(2-bromo-5-methoxyethoxybenzyl)-1-pyrrolidinyl)propyl)-1-piperidinecarboxylate,
- 4-(3-(4-(3-(2-bromo-5-methoxyethoxybenzyl)-1-pyrrolidinyl)propyl)-N,N-dimethyl-1-piperidinesulfonamide, and
- 4-(2-(3-(2-bromo-5-methoxyethoxybenzyl)-1-pyrrolidinyl)ethyl)-1-piperidinecarboximidamide.

The following compounds may be produced in the same manner as in Example 34, 38, 46 or 54.

- trans-4-(3-(4-(2-bromo-5-isopropoxybenzyl)piperidin-1-yl)propyl)cyclohexanamine,
- cis-4-(3-(4-(2-bromo-5-isopropoxybenzyl)piperidin-1-yl)propyl)cyclohexanamine,
- trans-4-(3-(4-(2-bromo-5-chlorobenzyl)piperidin-1-yl)propyl)cyclohexanamine,
- cis-4-(3-(4-(2-bromo-5-chlorobenzyl)piperidin-1-yl)propyl)cyclohexanamine,
- trans-4-(3-(4-(2-bromo-5-fluorobenzyl)piperidin-1-yl)propyl)cyclohexanamine,
- cis-4-(3-(4-(2-bromo-5-fluorobenzyl)piperidin-1-yl)propyl)cyclohexanamine,
- trans-4-(3-(3-(2-bromo-5-methoxybenzyl)pyrrolidin-1-yl)propyl)cyclohexanamine,
- cis-4-(3-(3-(2-bromo-5-methoxybenzyl)pyrrolidin-1-yl)propyl)cyclohexanamine,
- trans-4-(2-(4-(2-bromo-5-isopropoxybenzyl)piperidin-1-yl)ethyl)cyclohexanamine,
- cis-4-(2-(4-(2-bromo-5-isopropoxybenzyl)piperidin-1-yl)ethyl)cyclohexanamine,
- trans-4-(2-(4-(2-bromo-5-chlorobenzyl)piperidin-1-yl)ethyl)cyclohexanamine,
- cis-4-(2-(4-(2-bromo-5-chlorobenzyl)piperidin-1-yl)ethyl)cyclohexanamine,
- trans-4-(2-(4-(2-bromo-5-fluorobenzyl)piperidin-1-yl)ethyl)cyclohexanamine,
- cis-4-(2-(4-(2-bromo-5-fluorobenzyl)piperidin-1-yl)ethyl)cyclohexanamine,
- trans-4-(2-(3-(2-bromo-5-methoxybenzyl)pyrrolidin-1-yl)ethyl)cyclohexanamine,
- cis-4-(2-(3-(2-bromo-5-methoxybenzyl)pyrrolidin-1-yl)ethyl)cyclohexanamine,
- trans-N-(4-(3-(4-(2-bromo-5-methoxybenzyl)piperidin-1-yl)propyl)cyclohexyl)-N-ethylamine,
- cis-N-(4-(3-(4-(2-bromo-5-isopropoxybenzyl)piperidin-1-yl)propyl)cyclohexyl)-N-ethylamine,
- trans-N-(4-(3-(4-(2-bromo-5-chlorobenzyl)piperidin-1-yl)propyl)cyclohexyl)-N-ethylamine,
- cis-N-(4-(3-(4-(2-bromo-5-chlorobenzyl)piperidin-1-yl)propyl)cyclohexyl)-N-ethylamine,
- trans-N-(4-(3-(4-(2-bromo-5-fluorobenzyl)piperidin-1-yl)propyl)cyclohexyl)-N-ethylamine,
- cis-N-(4-(3-(4-(2-bromo-5-fluorobenzyl)piperidin-1-yl)propyl)cyclohexyl)-N-ethylamine,
- trans-N-(4-(2-(4-(2-bromo-5-chlorobenzyl)piperidin-1-yl)ethyl)cyclohexyl)-N-ethylamine,
- cis-N-(4-(2-(4-(2-bromo-5-chlorobenzyl)piperidin-1-yl)ethyl)cyclohexyl)-N-ethylamine,
- trans-N-(4-(2-(4-(2-bromo-5-fluorobenzyl)piperidin-1-yl)ethyl)cyclohexyl)-N-ethylamine,
- cis-N-(4-(2-(4-(2-bromo-5-fluorobenzyl)piperidin-1-yl)ethyl)cyclohexyl)-N-ethylamine,
- trans-N-(4-(3-(3-(2-bromo-5-methoxybenzyl)pyrrolidin-1-yl)propyl)cyclohexyl)-N-ethylamine,
- cis-N-(4-(3-(3-(2-bromo-5-methoxybenzyl)pyrrolidin-1-yl)propyl)cyclohexyl)-N-ethylamine,
- trans-N-(4-(2-(4-(2-bromo-5-methoxybenzyl)piperidin-1-yl)ethyl)cyclohexyl)acetamide,
- trans-N-(4-(2-(4-(2-bromo-5-isopropoxybenzyl)piperidin-1-yl)ethyl)cyclohexyl)acetamide,
- trans-N-(4-(2-(4-(2-bromo-5-chlorobenzyl)piperidin-1-yl)ethyl)cyclohexyl)acetamide,
- trans-N-(4-(2-(4-(2-bromo-5-fluorobenzyl)piperidin-1-yl)ethyl)cyclohexyl)acetamide,
- trans-N-(4-(3-(4-(2-bromo-5-methoxybenzyl)piperidin-1-yl)propyl)cyclohexyl)acetamide,
- trans-N-(4-(3-(4-(2-bromo-5-isopropoxybenzyl)piperidin-1-yl)propyl)cyclohexyl)acetamide,
- trans-N-(4-(3-(4-(2-bromo-5-chlorobenzyl)piperidin-1-yl)propyl)cyclohexyl)acetamide,
- trans-N-(4-(3-(4-(2-bromo-5-fluorobenzyl)piperidin-1-yl)propyl)cyclohexyl)acetamide,
- trans-N-(4-(2-(3-(2-bromo-5-methoxybenzyl)pyrrolidin-1-yl)ethyl)cyclohexyl)acetamide,
- trans-N-(4-(2-(4-(2-bromo-5-methoxybenzyl)piperidin-1-yl)ethyl)cyclohexyl)propionamide,
- trans-N-(4-(2-(4-(2-bromo-5-isopropoxybenzyl)piperidin-1-yl)ethyl)cyclohexyl)propionamide,
- trans-N-(4-(2-(4-(2-bromo-5-chlorobenzyl)piperidin-1-yl)ethyl)cyclohexyl)propionamide,
- trans-N-(4-(2-(4-(2-bromo-5-fluorobenzyl)piperidin-1-yl)ethyl)cyclohexyl)propionamide,
- trans-N-(4-(3-(4-(2-bromo-5-methoxybenzyl)piperidin-1-yl)propyl)cyclohexyl)propionamide,
- trans-N-(4-(3-(4-(2-bromo-5-isopropoxybenzyl)piperidin-1-yl)propyl)cyclohexyl)propionamide,
- trans-N-(4-(3-(4-(2-bromo-5-chlorobenzyl)piperidin-1-yl)propyl)cyclohexyl)propionamide,
- trans-N-(4-(3-(4-(2-bromo-5-fluorobenzyl)piperidin-1-yl)propyl)cyclohexyl)propionamide, trans-N-(4-(2-(3-(2-bromo-5-methoxybenzyl)pyrrolidin-1-yl)ethyl)cyclohexyl)propionamide,
- trans-N-(4-(2-(4-(2-bromo-5-isopropoxybenzyl)piperidin-1-yl)ethyl)cyclsohexyl)-2-hydroxyacetamide,
- trans-N-(4-(2-(4-(2-bromo-5-chlorobenzyl)piperidin-1-yl)ethyl)cyclohexyl)-2-hydroxyacetamide,

- trans-N-(4-(2-(4-(2-bromo-5-fluorobenzyl)piperidin-1-yl)ethyl)cyclohexyl)-2-hydroxyacetamide,
- trans-N-(4-(3-(4-(2-bromo-5-methoxybenzyl)piperidin-1-yl)propyl)cyclohexyl)-2-hydroxyacetamide,
- trans-N-(4-(3-(4-(2-bromo-5-isopropoxybenzyl)piperidin-1-yl)propyl)cyclohexyl)-2-hydroxyacetamide,
- trans-N-(4-(3-(4-(2-bromo-5-chlorobenzyl)piperidin-1-yl)propyl)cyclohexyl)-2-hydroxyacetamide,
- trans-N-(4-(3-(4-(2-bromo-5-fluorobenzyl)piperidin-1-yl)propyl)cyclohexyl)-2-hydroxyacetamide,
- trans-N-(4-(2-(3-(2-bromo-5-methoxybenzyl)pyrrolidin-1-yl)ethyl)cyclohexyl)-2-hydroxyacetamide,
- trans-N-(4-(2-(4-(2-bromo-5-isopropoxybenzyl)piperidin-1-yl)ethyl)cyclohexyl)-2-methoxyacetamide,
- trans-N-(4-(2-(4-(2-bromo-5-chlorobenzyl)piperidin-1-yl)ethyl)cyclohexyl)-2-methoxyacetamide,
- trans-N-(4-(2-(4-(2-bromo-5-fluorobenzyl)piperidin-1-yl)ethyl)cyclohexyl)-2-methoxyacetamide,
- trans-N-(4-(3-(4-(2-bromo-5-methoxybenzyl)piperidin-1-yl)propyl)cyclohexyl)-2-methoxyacetamide,
- trans-N-(4-(3-(4-(2-bromo-5-isopropoxybenzyl)piperidin-1-yl)propyl)cyclohexyl)-2-methoxyacetamide,
- trans-N-(4-(3-(4-(2-bromo-5-chlorobenzyl)piperidin-1-yl)propyl)cyclohexyl)-2-methoxyacetamide,
- trans-N-(4-(3-(4-(2-bromo-5-fluorobenzyl)piperidin-1-yl)propyl)cyclohexyl)-2-methoxyacetamide,
- trans-N-(4-(3-(3-(2-bromo-5-methoxybenzyl)pyrrolidin-1-yl)propyl)cyclohexyl)-2-methoxyacetamide,
- trans-N-(4-(2-(4-(2-bromo-5-isopropoxybenzyl)piperidin-1-yl)ethyl)cyclohexyl)-2,2,2-trifluoroacetamide,
- trans-N-(4-(2-(4-(2-bromo-5-chlorobenzyl)piperidin-1-yl)ethyl)cyclohexyl)-2,2,2-trifluoroacetamide,
- trans-N-(4-(2-(4-(2-bromo-5-fluorobenzyl)piperidin-1-yl)ethyl)cyclohexyl)-2,2,2-trifluoroacetamide,
- trans-N-(4-(3-(4-(2-bromo-5-methoxybenzyl)piperidin-1-yl)propyl)cyclohexyl)-2,2,2-trifluoroacetamide,
- trans-N-(4-(3-(4-(2-bromo-5-isopropoxybenzyl)piperidin-1-yl)propyl)cyclohexyl)-2,2,2-trifluoroacetamide,
- trans-N-(4-(3-(4-(2-bromo-5-chlorobenzyl)piperidin-1-yl)propyl)cyclohexyl)-2,2,2-trifluoroacetamide,
- trans-N-(4-(3-(4-(2-bromo-5-fluorobenzyl)piperidin-1-yl)propyl)cyclohexyl)-2,2,2-trifluoroacetamide,
- trans-N-(4-(2-(3-(2-bromo-5-methoxybenzyl)pyrrolidin-1-yl)ethyl)cyclohexyl)-2,2,2-trifluoroacetamide,
- trans-N-(4-(2-(4-(2-bromo-5-methoxybenzyl)piperidin-1-yl)ethyl)cyclohexyl)-2,2,2-trifluoroacetamide,
- trans-N-(4-(2-(4-(2-bromo-5-isopropoxybenzyl)piperidin-1-yl)ethyl)cyclohexyl)2,2,2-trifluoroacetamide,
- trans-N-(4-(2-(4-(2-bromo-5-chlorobenzyl)piperidin-1-yl)ethyl)cyclohexyl)-2,2,2-trifluoroacetamide,
- trans-N-(4-(2-(4-(2-bromo-5-fluorobenzyl)piperidin-1-yl)ethyl)cyclohexyl)-2,2,2-trifluoroacetamide,
- trans-N-(4-(3-(4-(2-bromo-5-methoxybenzyl)piperidin-1-yl)propyl)cyclohexyl)-2,2,2-trifluoroacetamide,
- trans-N-(4-(3-(4-(2-bromo-5-isopropoxybenzyl)piperidin-1-yl)propyl)cyclohexyl)-2,2,2-trifluoroacetamide,
- trans-N-(4-(3-(4-(2-bromo-5-chlorobenzyl)piperidin-1-yl)propyl)cyclohexyl)-2,2,2-trifluoroacetamide,
- trans-N-(4-(3-(4-(2-bromo-5-fluorobenzyl)piperidin-1-yl)propyl)cyclohexyl)-2,2,2-trifluoroacetamide,
- trans-N-(4-(2-(3-(2-bromo-5-methoxybenzyl)pyrrolidin-1-yl)ethyl)cyclohexyl)-2,2,2-trifluoroacetamide,
- trans-N-(4-(2-(4-(2-bromo-5-methoxybenzyl)piperidin-1-yl)ethyl)cyclohexyl)methanesulfonamide,
- trans-N-(4-(2-(4-(2-bromo-5-isopropoxybenzyl)piperidin-1-yl)ethyl)cyclohexyl)methanesulfonamide,
- trans-N-(4-(2-(4-(2-bromo-5-chlorobenzyl)piperidin-1-yl)ethyl)cyclohexyl)methanesulfonamide,
- trans-N-(4-(2-(4-(2-bromo-5-fluorobenzyl)piperidin-1-yl)ethyl)cyclohexyl)methanesulfonamide,
- trans-N-(4-(3-(4-(2-bromo-5-isopropoxybenzyl)piperidin-1-yl)propyl)cyclohexyl)methanesulfonamide,
- trans-N-(4-(3-(4-(2-bromo-5-chlorobenzyl)piperidin-1-yl)propyl)cyclohexyl)methanesulfonamide,
- trans-N-(4-(3-(4-(2-bromo-5-fluorobenzyl)piperidin-1-yl)propyl)cyclohexyl)methanesulfonamide,
- trans-N-(4-(2-(3-(2-bromo-5-methoxybenzyl)pyrrolidin-1-yl)ethyl)cyclohexyl)methanesulfonamide,
- trans-N-(4-(2-(4-(2-bromo-5-chlorobenzyl)piperidin-1-yl)ethyl)cyclohexyl)-N-ethylacetamide,
- trans-N-(4-(2-(4-(2-bromo-5-fluorobenzyl)piperidin-1-yl)ethyl)cyclohexyl)-N-ethylacetamide,
- trans-N-(4-(3-(4-(2-bromo-5-methoxybenzyl)piperidin-1-yl)propyl)cyclohexyl)-N-ethylacetamide,
- trans-N-(4-(3-(4-(2-bromo-5-isopropoxybenzyl)piperidin-1-yl)propyl)cyclohexyl)-N-ethylacetamide,
- trans-N-(4-(3-(4-(2-bromo-5-chlorobenzyl)piperidin-1-yl)propyl)cyclohexyl)-N-ethylacetamide,
- trans-N-(4-(3-(4-(2-bromo-5-fluorobenzyl)piperidin-1-yl)propyl)cyclohexyl)-N-ethylacetamide,
- trans-N-(4-(2-(3-(2-bromo-5-chlorobenzyl)pyrrolidin-1-yl)ethyl)cyclohexyl)-N-ethylacetamide,
- trans-N-(4-(2-(4-(2-bromo-5-chlorobenzyl)piperidin-1-yl)ethyl)cyclohexyl)-N,N-diethylamine,
- trans-N-(4-(2-(4-(2-bromo-5-fluorobenzyl)piperidin-1-yl)ethyl)cyclohexyl)-N,N-diethylamine,
- trans-N-(4-(3-(4-(2-bromo-5-methoxybenzyl)piperidin-1-yl)propyl)cyclohexyl)-N,N-diethylamine,
- trans-N-(4-(3-(4-(2-bromo-5-isopropoxybenzyl)piperidin-1-yl)propyl)cyclohexyl)-N,N-diethylamine,
- trans-N-(4-(3-(4-(2-bromo-5-chlorobenzyl)piperidin-1-yl)propyl)cyclohexyl)-N,N-diethylamine,
- trans-N-(4-(3-(4-(2-bromo-5-fluorobenzyl)piperidin-1-yl)propyl)cyclohexyl)-N,N-diethylamine,
- trans-N-(4-(2-(3-(2-bromo-5-methoxybenzyl)pyrrolidin-1-yl)ethyl)cyclohexyl)-N,N-diethylamine,
- trans-N-(4-(2-(4-(2-bromo-5-isopropoxybenzyl)piperidin-1-yl)ethyl)cyclohexyl)-N-ethyl-N-(2-hydroxyethyl)amine,
- trans-N-(4-(2-(4-(2-bromo-5-chlorobenzyl)piperidin-1-yl)ethyl)cyclohexyl)-N-ethyl-N-(2-hydroxyethyl)amine,
- trans-N-(4-(2-(4-(2-bromo-5-fluorobenzyl)piperidin-1-yl)ethyl)cyclohexyl)-N-ethyl-N-(2-hydroxyethyl)amine,
- trans-N-(4-(3-(4-(2-bromo-5-methoxybenzyl)piperidin-1-yl)propyl)cyclohexyl)-N-ethyl-N-(2-hydroxyethyl)amine,
- trans-N-(4-(3-(4-(2-bromo-5-isopropoxybenzyl)piperidin-1-yl)propyl)cyclohexyl)-N-ethyl-N-(2-hydroxyethyl)amine,

- trans-N-(4-(3-(4-(2-bromo-5-chlorobenzyl)piperidin-1-yl)propyl)cyclohexyl)-N-ethyl-N-(2-hydroxyethyl)amine,
- trans-N-(4-(3-(4-(2-bromo-5-fluorobenzyl)piperidin-1-yl)propyl)cyclohexyl)-N-ethyl-N-(2-hydroxyethyl)amine,
- trans-N-(4-(2-(3-(2-bromo-5-methoxybenzyl)pyrrolidin-1-yl)ethyl)cyclohexyl)-N-ethyl-N-(2-hydroxyethyl)amine,
- trans-N-(4-(2-(4-(2-bromo-5-isopropoxybenzyl)piperidin-1-yl)ethyl)cyclohexyl)-N-ethyl-N-(2-methoxyethyl)amine,
- trans-N-(4-(2-(4-(2-bromo-5-chlorobenzyl)piperidin-1-yl)ethyl)cyclohexyl)-N-ethyl-N-(2-methoxyethyl)amine,
- trans-N-(4-(2-(4-(2-bromo-5-fluorobenzyl)piperidin-1-yl)ethyl)cyclohexyl)-N-ethyl-N-(2-methoxyethyl)amine,
- trans-N-(4-(3-(4-(2-bromo-5-methoxybenzyl)piperidin-1-yl)propyl)cyclohexyl)-N-ethyl-N-(2-methoxyethyl)amine,
- trans-N-(4-(3-(4-(2-bromo-5-isopropoxybenzyl)piperidin-1-yl)propyl)cyclohexyl)-N-ethyl-N-(2-methoxyethyl) amine,
- trans-N-(4-(3-(4-(2-bromo-5-chlorobenzyl)piperidin-1-yl)propyl)cyclohexyl)-N-ethyl-N-(2-methoxyethyl)amine,
- trans-N-(4-(3-(4-(2-bromo-5-fluorobenzyl)piperidin-1-yl)propyl)cyclohexyl)-N-ethyl-N-(2-methoxyethyl)amine,
- trans-N-(4-(2-(3-(2-bromo-5-methoxybenzyl)pyrrolidin-1-yl)ethyl)cyclohexyl)-N-ethyl-N-(2-methoxyethyl)amine,
- trans-N-(4-(2-(4-(2-bromo-5-isopropoxybenzyl)piperidin-1-yl)ethyl)cyclohexyl)-N-ethyl-N-(2,2,2-trifluoroethyl) amine,
- trans-N-(4-(2-(4-(2-bromo-5-chlorobenzyl)piperidin-1-yl)ethyl)cyclohexyl)-N-ethyl-N-(2,2,2-trifluoroethyl)amine,
- trans-N-(4-(2-(4-(2-bromo-5-fluorobenzyl)piperidin-1-yl)ethyl)cyclohexyl)-N-ethyl-N-(2,2,2-trifluoroethyl)amine,
- trans-N-(4-(3-(4-(2-bromo-5-methoxybenzyl)piperidin-1-yl)propyl)cyclohexyl)-N-ethyl-N-(2,2,2-trifluoroethyl) amine,
- trans-N-(4-(3-(4-(2-bromo-5-isopropoxybenzyl)piperidin-1-yl)propyl)cyclohexyl)-N-ethyl-N-(2,2,2-trifluoroethyl) amine,
- trans-N-(4-(3-(4-(2-bromo-5-chlorobenzyl)piperidin-1-yl)propyl)cyclohexyl)-N-ethyl-N-(2,2,2-trifluoroethyl)amine,
- trans-N-(4-(3-(4-(2-bromo-5-fluorobenzyl)piperidin-1-yl)propyl)cyclohexyl)-N-ethyl-N-(2,2,2-trifluoroethyl)amine,
- trans-N-(4-(2-(3-(2-bromo-5-methoxybenzyl)pyrrolidin-1-yl)ethyl)cyclohexyl)-N-ethyl-N-(2,2,2-trifluoroethyl) amine,
- trans-N'-(4-(2-(4-(2-bromo-5-isopropoxybenzyl)piperidin-1-yl)ethyl)cyclohexyl)-N,N-dimethylsulfamide,
- trans-N'-(4-(2-(4-(2-bromo-5-chlorobenzyl)piperidin-1-yl)ethyl)cyclohexyl)-N,N-dimethylsulfamide,
- trans-N'-(4-(2-(4-(2-bromo-5-fluorobenzyl)piperidin-1-yl)ethyl)cyclohexyl)-N,N-dimethylsulfamide,
- trans-N'-(4-(3-(4-(2-bromo-5-methoxybenzyl)piperidin-1-yl)propyl)cyclohexyl)-N,N-dimethylsulfamide,
- trans-N'-(4-(3-(4-(2-bromo-5-isopropoxybenzyl)piperidin-1-yl)propyl)cyclohexyl)-N,N-dimethylsulfamide,
- trans-N'-(4-(3-(4-(2-bromo-5-chlorobenzyl)piperidin-1-yl)propyl)cyclohexyl)-N,N-dimethylsulfamide,
- trans-N'-(4-(3-(4-(2-bromo-5-fluorobenzyl)piperidin-1-yl)propyl)cyclohexyl)-N,N-dimethylsulfamide,
- trans-N'-(4-(2-(3-(2-bromo-5-methoxybenzyl)pyrrolidin-1-yl)ethyl)cyclohexyl)-N,N-dimethylsulfamide,
- cis-N'-(4-(2-(4-(2-bromo-5-methoxybenzyl)piperidin-1-yl)ethyl)cyclohexyl)-N,N-dimethylsulfamide,
- cis-N'-(4-(2-(4-(2-bromo-5-isopropoxybenzyl)piperidin-1-yl)ethyl)cyclohexyl)-N,N-dimethylsulfamide,
- cis-N'-(4-(2-(4-(2-bromo-5-chlorobenzyl)piperidin-1-yl)ethyl)cyclohexyl)-N,N-dimethylsulfamide,
- cis-N'-(4-(2-(4-(2-bromo-5-fluorobenzyl)piperidin-1-yl)ethyl)cyclohexyl)-N,N-dimethylsulfamide,
- cis-N'-(4-(3-(4-(2-bromo-5-methoxybenzyl)piperidin-1-yl)propyl)cyclohexyl)-N,N-dimethylsulfamide,
- cis-N'-(4-(3-(4-(2-bromo-5-isopropoxybenzyl)piperidin-1-yl)propyl)cyclohexyl)-N,N-dimethylsulfamide,
- cis-N'-(4-(3-(4-(2-bromo-5-chlorobenzyl)piperidin-1-yl)propyl)cyclohexyl)-N,N-dimethylsulfamide,
- cis-N'-(4-(3-(4-(2-bromo-5-fluorobenzyl)piperidin-1-yl)propyl)cyclohexyl)-N,N-dimethylsulfamide,
- cis-N'-(4-(2-(3-(2-bromo-5-methoxybenzyl)pyrrolidin-1-yl)ethyl)cyclohexyl)-N,N-dimethylsulfamide,
- trans-N-(4-(2-(4-(2-bromo-5-methoxybenzyl)piperidin-1-yl)ethyl)cyclohexyl)-N'-methylsulfamide,
- trans-N-(4-(2-(4-(2-bromo-5-isopropoxybenzyl)piperidin-1-yl)ethyl)cyclohexyl)-N'-methylsulfamide,
- trans-N-(4-(2-(4-(2-bromo-5-chlorobenzyl)piperidin-1-yl)ethyl)cyclohexyl)-N'-methylsulfamide,
- trans-N-(4-(2-(4-(2-bromo-5-fluorobenzyl)piperidin-1-yl)ethyl)cyclohexyl)-N'-methylsulfamide,
- trans-N-(4-(3-(4-(2-bromo-5-methoxybenzyl)piperidin-1-yl)propyl)cyclohexyl)-N'-methylsulfamide,
- trans-N-(4-(3-(4-(2-bromo-5-isopropoxybenzyl)piperidin-1-yl)propyl)cyclohexyl)-N'-methylsulfamide,
- trans-N-(4-(3-(4-(2-bromo-5-chlorobenzyl)piperidin-1-yl)propyl)cyclohexyl)-N'-methylsulfamide,
- trans-N-(4-(3-(4-(2-bromo-5-fluorobenzyl)piperidin-1-yl)propyl)cyclohexyl)-N'-methylsulfamide,
- trans-N-(4-(2-(3-(2-bromo-5-methoxybenzyl)pyrrolidin-1-yl)ethyl)cyclohexyl)-N'-methylsulfamide,
- trans-N-(4-(2-(4-(2-bromo-5-methoxybenzyl)piperidin-1-yl)ethyl)cyclohexyl)sulfamide,
- trans-N-(4-(2-(4-(2-bromo-5-isopropoxybenzyl)piperidin-1-yl)ethyl)cyclohexyl)sulfamide,
- trans-N-(4-(2-(4-(2-bromo-5-chlorobenzyl)piperidin-1-yl)ethyl)cyclohexyl)sulfamide,
- trans-N-(4-(2-(4-(2-bromo-5-fluorobenzyl)piperidin-1-yl)ethyl)cyclohexyl)sulfamide,
- trans-N-(4-(3-(4-(2-bromo-5-methoxybenzyl)piperidin-1-yl)propyl)cyclohexyl)sulfamide,
- trans-N-(4-(3-(4-(2-bromo-5-isopropoxybenzyl)piperidin-1-yl)propyl)cyclohexyl)sulfamide,
- trans-N-(4-(3-(4-(2-bromo-5-chlorobenzyl)piperidin-1-yl)propyl)cyclohexyl)sulfamide,

- trans-N-(4-(3-(4-(2-bromo-5-fluorobenzyl)piperidin-1-yl)propyl)cyclohexyl)sulfamide,
- trans-N-(4-(2-(3-(2-bromo-5-methoxybenzyl)pyrrolidin-1-yl)ethyl)cyclohexyl)sulfamide,
- trans-N-(4-(2-(4-(2-bromo-5-methoxybenzyl)piperidin-1-yl)ethyl)cyclohexyl)morpholine-4-sulfonamide,
- trans-N-(4-(2-(4-(2-bromo-5-isopropoxybenzyl)piperidin-1-yl)ethyl)cyclohexyl)morpholine-4-sulfonamide,
- trans-N-(4-(2-(4-(2-bromo-5-chlorobenzyl)piperidin-1-yl)ethyl)cyclohexyl)morpholine-4-sulfonamide,
- trans-N-(4-(2-(4-(2-bromo-5-fluorobenzyl)piperidin-1-yl)ethyl)cyclohexyl)morpholine-4-sulfonamide,
- trans-N-(4-(3-(4-(2-bromo-5-methoxybenzyl)piperidin-1-yl)propyl)cyclohexyl)morpholine-4-sulfonamide,
- trans-N-(4-(3-(4-(2-bromo-5-isopropoxybenzyl)piperidin-1-yl)propyl)cyclohexyl)morpholine-4-sulfonamide,
- trans-N-(4-(3-(4-(2-bromo-5-chlorobenzyl)piperidin-1-yl)propyl)cyclohexyl)morpholine-4-sulfonamide,
- trans-N-(4-(3-(4-(2-bromo-5-fluorobenzyl)piperidin-1-yl)propyl)cyclohexyl)morpholine-4-sulfonamide,
- trans-N-(4-(2-(3-(2-bromo-5-methoxybenzyl)pyrrolidin-1-yl)ethyl)cyclohexyl)morpholine-4-sulfonamide,
- trans-N-(4-(2-(4-(2-bromo-5-methoxybenzyl)piperidin-1-yl)ethyl)cyclohexyl)pyrrolidine-1-sulfonamide,
- trans-N-(4-(2-(4-(2-bromo-5-isopropoxybenzyl)piperidin-1-yl)ethyl)cyclohexyl)pyrrolidine-1-sulfonamide,
- trans-N-(4-(2-(4-(2-bromo-5-chlorobenzyl)piperidin-1-yl)ethyl)cyclohexyl)pyrrolidine-1-sulfonamide,
- trans-N-(4-(2-(4-(2-bromo-5-fluorobenzyl)piperidin-1-yl)ethyl)cyclohexyl)pyrrolidine-1-sulfonamide,
- trans-N-(4-(3-(4-(2-bromo-5-methoxybenzyl)piperidin-1-yl)propyl)cyclohexyl)pyrrolidine-1-sulfonamide,
- trans-N-(4-(3-(4-(2-bromo-5-isopropoxybenzyl)piperidin-1-yl)propyl)cyclohexyl)pyrrolidine-1-sulfonamide,
- trans-N-(4-(3-(4-(2-bromo-5-chlorobenzyl)piperidin-1-yl)propyl)cyclohexyl)pyrrolidine-1-sulfonamide,
- trans-N-(4-(3-(4-(2-bromo-5-fluorobenzyl)piperidin-1-yl)propyl)cyclohexyl)pyrrolidine-1-sulfonamide,
- trans-N-(4-(2-(3-(2-bromo-5-methoxybenzyl)pyrrolidin-1-yl)ethyl)cyclohexyl)pyrrolidine-1-sulfonamide,
- trans-N-(4-(2-(4-(2-bromo-5-methoxybenzyl)piperidin1-yl)ethyl)cyclohexyl)-N'-ethylurea,
- trans-N-(4-(2-(4-(2-bromo-5-isopropoxybenzyl)piperidin-1-yl)ethyl)cyclohexyl)-N'-ethylurea,
- trans-N-(4-(2-(4-(2-bromo-5-chlorobenzyl)piperidin-1-yl)ethyl)cyclohexyl)-N'-ethylurea,
- trans-N-(4-(2-(4-(2-bromo-5-fluorobenzyl)piperidin-1-yl)ethyl)cyclohexyl)-N'-ethylurea,
- trans-N-(4-(3-(4-(2-bromo-5-methoxybenzyl)piperidin-1-yl)propyl)cyclohexyl)-N'-ethylurea,
- trans-N-(4-(3-(4-(2-bromo-5-isopropoxybenzyl)piperidin-1-yl)propyl)cyclohexyl)-N'-ethylurea,
- trans-N-(4-(3-(4-(2-bromo-5-chlorobenzyl)piperidin-1-yl)propyl)cyclohexyl)-N'-ethylurea,
- trans-N-(4-(3-(4-(2-bromo-5-fluorobenzyl)piperidin-1-yl)propyl)cyclohexyl)-N'-ethylurea,
- trans-N-(4-(2-(3-(2-bromo-5-methoxybenzyl)pyrrolidin-1-yl)ethyl)cyclohexyl)-N'-ethylurea,
- trans-N-(4-(2-(4-(2-bromo-5-methoxybenzyl)piperidin-1-yl)ethyl)cyclohexyl)-N-ethylmethanesulfonamide,
- trans-N-(4-(2-(4-(2-bromo-5-isopropoxybenzyl)piperidin-1-yl)ethyl)cyclohexyl)-N-ethylmethanesulfonamide,
- trans-N-(4-(2-(4-(2-bromo-5-chlorobenzyl)piperidin-1-yl)ethyl)cyclohexyl)-N-ethylmethanesulfonamide,
- trans-N-(4-(2-(4-(2-bromo-5-fluorobenzyl)piperidin-1-yl)ethyl)cyclohexyl)-N-ethylmethanesulfonamide,
- trans-N-(4-(3-(4-(2-bromo-5-methoxybenzyl)piperidin-1-yl)propyl)cyclohexyl)-N-ethylmethanesulfonamide,
- trans-N-(4-(3-(4-(2-bromo-5-chlorobenzyl)piperidin-1-yl)propyl)cyclohexyl)-N-ethylmethanesulfonamide,
- trans-N-(4-(3-(4-(2-bromo-5-fluorobenzyl)piperidin-1-yl)propyl)cyclohexyl)-N-ethylmethanesulfonamide,
- trans-N-(4-(2-(3-(2-bromo-5-methoxybenzyl)pyrrolidin-1-yl)ethyl)cyclohexyl)-N-ethylmethanesulfonamide,
- trans-N-(4-(2-(4-(2-bromo-5-methoxybenzyl)piperidin-1-yl)ethyl)cyclohexyl)ethanesulfonamide,
- trans-N-(4-(2-(4-(2-bromo-5-isopropoxybenzyl)piperidin-1-yl)ethyl)cyclohexyl)ethanesulfonamide,
- trans-N-(4-(2-(4-(2-bromo-5-chlorobenzyl)piperidin-1-yl)ethyl)cyclohexyl)ethanesulfonamide,
- trans-N-(4-(2-(4-(2-bromo-5-fluorobenzyl)piperidin-1-yl)ethyl)cyclohexyl)ethanesulfonamide,
- trans-N-(4-(3-(4-(2-bromo-5-methoxybenzyl)piperidin-1-yl)propyl)cyclohexyl)ethanesulfonamide,
- trans-N-(4-(3-(4-(2-bromo-5-isopropoxybenzyl)piperidin-1-yl)propyl)cyclohexyl)ethanesulfonamide,
- tnans-N-(4-(3-(4-(2-bromo-5-chlorobenzyl)piperidin-1-yl)propyl)cyclohexyl)ethanesulfonamide,
- trans-N-(4-(3-(4-(2-bromo-5-fluorobenzyl)piperidin-1-yl)propyl)cyclohexyl)ethanesulfonamide,
- trans-N-(4-(2-(3-(2-bromo-5-methoxybenzyl)pyrrolidin-1-yl)ethyl)cyclohexyl)ethanesulfonamide,
- trans-N-(4-(2-(4-(2-bromo-5-methoxybenzyl)piperidin-1-yl)ethyl)cyclohexyl)-N-ethyl-N',N'-dimethylsulfamide,
- trans-N-(4-(2-(4-(2-bromo-5-isopropoxybenzyl)piperidin-1-yl)ethyl)cyclohexyl)-N-ethyl-N',N'-dimethylsulfamide,
- trans-N-(4-(2-(4-(2-bromo-5-chlorobenzyl)piperidin-1-yl)ethyl)cyclohexyl)-N-ethyl-N',N'-dimethylsulfamide,
- trans-N-(4-(2-(4-(2-bromo-5-fluorobenzyl)piperidin-1-yl)ethyl)cyclohexyl)-N-ethyl-N',N'-dimethylsulfamide,
- trans-N-(4-(3-(4-(2-bromo-5-methoxybenzyl)piperidin-1-yl)propyl)cyclohexyl)-N-ethyl-N',N'-dimethylsulfamide,
- trans-N-(4-(3-(4-(2-bromo-5-isopropoxybenzyl)piperidin-1-yl)propyl)cyclohexyl)-N-ethyl-N',N'-dimethylsulfamide,
- trans-N-(4-(3-(4-(2-bromo-5-chlorobenzyl)piperidin-1-yl)propyl)cyclohexyl)-N-ethyl-N',N'-dimethylsulfamide,
- trans-N-(4-(3-(4-(2-bromo-5-fluorobenzyl)piperidin-1-yl)propyl)cyclohexyl)-N-ethyl-N',N'-dimethylsulfamide,
- trans-N-(4-(2-(3-(2-bromo-5-methoxybenzyl)pyrrolidin-1-yl)ethyl)cyclohexyl)-N-ethyl-N',N'-dimethylsulfamide,
- trans-N-(4-(2-(4-(2-bromo-5-methoxybenzyl)piperidin-1-yl)ethyl)cyclohexyl)-N-(2-methoxyethyl)amine,
- trans-N-(4-(2-(4-(2-bromo-5-isopropoxybenzyl)piperidin-1-yl)ethyl)cyclohexyl)-N-(2-methoxyethyl)amine,
- trans-N-(4-(2-(4-(2-bromo-5-chlorobenzyl)piperidin-1-yl)ethyl)cyclohexyl)-N-(2-methoxyethyl)amine,

- trans-N-(4-(2-(4-(2-bromo-5-fluorobenzyl)piperidin-1-yl)ethyl)cyclohexyl)-N-(2-methoxyethyl)amine,
- trans-N-(4-(3-(4-(2-bromo-5-methoxybenzyl)piperidin-1-yl)propyl)cyclohexyl)-N-(2-methoxyethyl)amine,
- trans-N-(4-(3-(4-(2-bromo-5-isopropoxybenzyl)piperidin-1-yl)propyl)cyclohexyl)-N-(2-methoxyethyl)amine,
- trans-N-(4-(3-(4-(2-bromo-5-chlorobenzyl)piperidin-1-yl)propyl)cyclohexyl)-N-(2-methoxyethyl)amine,
- trans-N-(4-(3-(4-(2-bromo-5-fluorobenzyl)piperidin-1-yl)propyl)cyclohexyl)-N-(2-methoxyethyl)amine,
- trans-N-(4-(2-(3-(2-bromo-5-methoxybenzyl)pyrrolidin-1-yl)ethyl)cyclohexyl)-N-(2-methoxyethyl)amine,
- trans-N-(4-(2-(4-(2-bromo-5-methoxybenzyl)piperidin-1-yl)ethyl)cyclohexyl)-N-(2-methoxyethyl)acetamide,
- trans-N-(4-(2-(4-(2-bromo-5-isopropoxybenzyl)piperidin-1-yl)ethyl)cyclohexyl)-N-(2-methoxyethyl)acetamide,
- trans-N-(4-(2-(4-(2-bromo-5-chlorobenzyl)piperidin-1-yl)ethyl)cyclohexyl)-N-(2-methoxyethyl)acetamide,
- trans-N-(4-(2-(4-(2-bromo-5-fluorobenzyl)piperidin-1-yl)ethyl)cyclohexyl)-N-(2-methoxyethyl)acetamide,
- trans-N-(4-(3-(4-(2-bromo-5-methoxybenzyl)piperidin-1-yl)propyl)cyclohexyl)-N-(2-methoxyethyl)acetamide,
- trans-N-(4-(3-(4-(2-bromo-5-isopropoxybenzyl)piperidin-1-yl)propyl)cyclohexyl)-N-(2-methoxyethyl)acetamide,
- trans-N-(4-(3-(4-(2-bromo-5-chlorobenzyl)piperidin-1-yl)propyl)cyclohexyl)-N-(2-methoxyethyl)acetamide,
- trans-N-(4-(3-(4-(2-bromo-5-fluorobenzyl)piperidin-1-yl)propyl)cyclohexyl)-N-(2-methoxyethyl)acetamide,
- trans-N-(4-(2-(3-(2-bromo-5-methoxybenzyl)pyrrolidin-1-yl)ethyl)cyclohexyl)-N-(2-methoxyethyl)acetamide,
- trans-N-(4-(2-(4-(2-bromo-5-methoxybenzyl)piperidin-1-yl)ethyl)cyclohexyl)-N-ethyl-2-methoxyacetamide,
- trans-N-(4-(2-(4-(2-bromo-5-isopropoxybenzyl)piperidin-1-yl)ethyl)cyclohexyl)-N-ethyl-2-methoxyacetamide,
- trans-N-(4-(2-(4-(2-bromo-5-chlorobenzyl)piperidin-1-yl)ethyl)cyclohexyl)-N-ethyl-2-methoxyacetamide,
- trans-N-(4-(2-(4-(2-bromo-5-fluorobenzyl)piperidin-1-yl)ethyl)cyclohexyl)-N-ethyl-2-methoxyacetamide,
- trans-N-(4-(3-(4-(2-bromo-5-methoxybenzyl)piperidin-1-yl)propyl)cyclohexyl)-N-ethyl-2-methoxyacetamide,
- trans-N-(4-(3-(4-(2-bromo-5-isopropoxybenzyl)piperidin-1-yl)propyl)cyclohexyl)-N-ethyl-2-methoxyacetamide,
- trans-N-(4-(3-(4-(2-bromo-5-chlorobenzyl)piperidin-1-yl)propyl)cyclohexyl)-N-ethyl-2-methoxyacetamide,
- trans-N-(4-(3-(4-(2-bromo-5-fluorobenzyl)piperidin-1-yl)propyl)cyclohexyl)-N-ethyl-2-methoxyacetamide,
- trans-N-(4-(2-(3-(2-bromo-5-methoxybenzyl)pyrrolidin-1-yl)ethyl)cyclohexyl)-N-ethyl-2-methoxyacetamide,
- trans-N-(4-(2-(4-(2-bromo-5-methoxybenzyl)piperidin-1-yl)ethyl)cyclohexyl)-N-ethyl-2-hydroxyacetamide,
- trans-N-(4-(2-(4-(2-bromo-5-isopropoxybenzyl)piperidin-1-yl)ethyl)cyclohexyl)-N-ethyl-2-hydroxyacetamide,
- trans-N-(4-(2-(4-(2-bromo-5-chlorobenzyl)piperidin-1-yl)ethyl)cyclohexyl)-N-ethyl-2-hydroxyacetamide,
- trans-N-(4-(2-(4-(2-bromo-5-fluorobenzyl)piperidin-1-yl)ethyl)cyclohexyl)-N-ethyl-2-hydroxyacetamide,
- trans-N-(4-(3-(4-(2-bromo-5-methoxybenzyl)piperidin-1-yl)propyl)cyclohexyl)-N-ethyl-2-hydroxyacetamide,
- trans-N-(4-(3-(4-(2-bromo-5-isopropoxybenzyl)piperidin-1-yl)propyl)cyclohexyl)-N-ethyl-2-hydroxyacetamide,
- trans-N-(4-(3-(4-(2-bromo-5-chlorobenzyl)piperidin-1-yl)propyl)cyclohexyl)-N-ethyl-2-hydroxyacetamide,
- trans-N-(4-(3-(4-(2-bromo-5-fluorobenzyl)piperidin-1-yl)propyl)cyclohexyl)-N-ethyl-2-hydroxyacetamide,
- trans-N-(4-(2-(3-(2-bromo-5-methoxybenzyl)pyrrolidin-1-yl)ethyl)cyclohexyl)-N-ethyl-2-hydroxyacetamide,
- trans-methyl 4-(2-(4-(2-bromo-5-methoxybenzyl)piperidin-1-yl)ethyl)cyclohexylcarbamate,
- trans-methyl 4-(2-(4-(2-bromo-5-isopropoxybenzyl)piperidin-1-yl)ethyl)cyclohexylcarbamate,
- trans-methyl 4-(2-(4-(2-bromo-5-chlorobenzyl)piperidin-1-yl)ethyl)cyclohexylcarbamate,
- trans-methyl 4-(2-(4-(2-bromo-5-fluorobenzyl)piperidin-1-yl)ethyl)cyclohexylcarbamate,
- trans-methyl 4-(3-(4-(2-bromo-5-methoxybenzyl)piperidin-1-yl)propyl)cyclohexylcarbamate,
- trans-methyl 4-(3-(4-(2-bromo-5-isopropoxybenzyl)piperidin-1-yl)propyl)cyclohexylcarbamate,
- trans-methyl 4-(3-(4-(2-bromo-5-chlorobenzyl)piperidin-1-yl)propyl)cyclohexylcarbamate,
- trans-methyl 4-(3-(4-(2-bromo-5-fluorobenzyl)piperidin-1-yl)propyl)cyclohexylcarbamate,
- trans-methyl 4-(2-(3-(2-bromo-5-methoxybenzyl)pyrrolidin-1-yl)ethyl)cyclohexylcarbamate,
- 4-(2-(4-(2-bromo-5-methoxybenzyl)piperidin-1-yl)ethyl)cyclohexanol,
- 4-(2-(4-(2-bromo-5-isopropoxybenzyl)piperidin-1-yl)ethyl)cyclohexanol,
- 4-(2-(4-(2-bromo-5-chlorobenzyl)piperidin-1-yl)ethyl)cyclohexanol,
- 4-(2-(4-(2-bromo-5-fluorobenzyl)piperidin-1-yl)ethyl)cyclohexanol,
- 4-(3-(4-(2-bromo-5-methoxybenzyl)piperidin-1-yl)propyl)cyclohexanol,
- 4-(3-(4-(2-bromo-5-isopropoxybenzyl)piperidin-1-yl)propyl)cyclohexanol,
- 4-(3-(4-(2-bromo-5-chlorobenzyl)piperidin-1-yl)propyl)cyclohexanol,
- 4-(3-(4-(2-bromo-5-fluorobenzyl)piperidin-1-yl)propyl)cyclohexanol,
- 4-(2-(3-(2-bromo-5-methoxybenzyl)pyrrolidin-1-yl)ethyl)cyclohexanol,
- 4-(2-(4-(2-bromo-5-methoxybenzyl)piperidin-1-yl)ethyl)cyclohexyl dimethylcarbamate,
- 4-(2-(4-(2-bromo-5-isopropoxybenzyl)piperidin-1-yl)ethyl)cyclohexyl dimethylcarbamate,
- 4-(2-(4-(2-bromo-5-chlorobenzyl)piperidin-1-yl)ethyl)cyclohexyl dimethylcarbamate,
- 4-(2-(4-(2-bromo-5-fluorobenzyl)piperidin-1-yl)ethyl)cyclohexyl dimethylcarbamate,
- 4-(3-(4-(2-bromo-5-methoxybenzyl)piperidin-1-yl)propyl)cyclohexyl dimethylcarbamate,
- 4-(3-(4-(2-bromo-5-isopropoxybenzyl)piperidin-1-yl)propyl)cyclohexyl dimethylcarbamate,
- 4-(3-(4-(2-bromo-5-chlorobenzyl)piperidin-1-yl)propyl)cyclohexyl dimethylcarbamate,

- 4-(3-(4-(2-bromo-5-fluorobenzyl)piperidin-1-yl)propyl)cyclohexyl dimethylcarbamate,
- 4-(2-(3-(2-bromo-5-methoxybenzyl)pyrrolidin-1-yl)ethyl)cyclohexyl dimethylcarbamate,
- 4-(2-(4-(2-bromo-5-methoxybenzyl)piperidin-1-yl)ethyl)cyclohexyl carbamate,
- 4-(2-(4-(2-bromo-5-isopropoxybenzyl)piperidin-1-yl)ethyl)cyclohexyl carbamate,
- 4-(2-(4-(2-bromo-5-chlorobenzyl)piperidin-1-yl)ethyl)cyclohexyl carbamate,
- 4-(2-(4-(2-bromo-5-fluorobenzyl)piperidin-1-yl)ethyl)cyclohexyl carbamate,
- 4-(3-(4-(2-bromo-5-methoxybenzyl)piperidin-1-yl)propyl)cyclohexyl carbamate,
- 4-(3-(4-(2-bromo-5-isopropoxybenzyl)piperidin-1-yl)propyl)cyclohexyl carbamate,
- 4-(3-(4-(2-bromo-5-chlorobenzyl)piperidin-1-yl)propyl)cyclohexyl carbamate,
- 4-(3-(4-(2-bromo-5-fluorobenzyl)piperidin-1-yl)propyl)cyclohexyl carbamate,
- 4-(2-(3-(2-bromo-5-methoxybenzyl)pyrrolidin-1-yl)ethyl)cyclohexyl carbamate,
- 4-(2-bromo-5-methoxybenzyl)-1-(2-(4-methoxycyclohexyl)ethyl)piperidine,
- 4-(2-bromo-5-isopropoxybenzyl)-1-(2-(4-methoxycyclohexyl)ethyl)piperidine,
- 4-(2-bromo-5-chlorobenzyl)-1-(2-(4-methoxycyclohexyl)ethyl)piperidine,
- 4-(2-bromo-5-fluorobenzyl)-1-(2-(4-methoxycyclohexyl)ethyl)piperidine,
- 4-(2-bromo-5-methoxybenzyl)-1-(3-(4-methoxycyclohexyl)propyl)piperidine,
- 4-(2-bromo-5-isopropoxybenzyl)-1-(3-(4-methoxycyclohexyl)propyl)piperidine,
- 4-(2-bromo-5-chlorobenzyl)-1-(3-(4-methoxycyclohexyl)propyl)piperidine,
- 4-(2-bromo-5-fluorobenzyl)-1-(3-(4-methoxycyclohexyl)propyl)piperidine,
- 3-(2-bromo-5-methoxybenzyl)-1-(2-(4-methoxycyclohexyl)ethyl)pyrrolidine,
- (4-(2-(4-(2-bromo-5-methoxybenzyl)piperidin-1-yl)ethyl)cyclohexyl)methanol,
- (4-(2-(4-(2-bromo-5-isopropoxybenzyl)piperidin-1-yl)ethyl)cyclohexyl)methanol,
- (4-(2-(4-(2-bromo-5-chlorobenzyl)piperidin-1-yl)ethyl)cyclohexyl)methanol,
- (4-(2-(4-(2-bromo-5-fluorobenzyl)piperidin-1-yl)ethyl)cyclohexyl)methanol,
- (4-(3-(4-(2-bromo-5-methoxybenzyl)piperidin-1-yl)propyl)cyclohexyl)methanol,
- (4-(3-(4-(2-bromo-5-isopropoxybenzyl)piperidin-1-yl)propyl)cyclohexyl)methanol,
- (4-(3-(4-(2-bromo-5-chlorobenzyl)piperidin-1-yl)propyl)cyclohexyl)methanol,
- (4-(3-(4-(2-bromo-5-fluorobenzyl)piperidin-1-yl)propyl)cyclohexyl)methanol,
- (4-(2-(3-(2-bromo-5-methoxybenzyl)pyrrolidin-1-yl)ethyl)cyclohexyl)methanol,
- 2-(4-(2-(4-(2-bromo-5-isopropoxybenzyl)piperidin-1-yl)ethyl)cyclohexyl)ethanol,
- 2-(4-(2-(4-(2-bromo-5-chlorobenzyl)piperidin-1-yl)ethyl)cyclohexyl)ethanol,
- 2-(4-(2-(4-(2-bromo-5-fluorobenzyl)piperidin-1-yl)ethyl)cyclohexyl)ethanol,
- 2-(4-(3-(4-(2-bromo-5-methoxybenzyl)piperidin-1-yl)propyl)cyclohexyl)ethanol,
- 2-(4-(3-(4-(2-bromo-5-isopropoxybenzyl)piperidin-1-yl)propyl)cyclohexyl)ethanol,
- 2-(4-(3-(4-(2-bromo-5-chlorobenzyl)piperidin-1-yl)propyl)cyclohexyl)ethanol,
- 2-(4-(3-(4-(2-bromo-5-fluorobenzyl)piperidin-1-yl)propyl)cyclohexyl)ethanol,
- 2-(4-(2-(3-(2-bromo-5-methoxybenzyl)pyrrolidin-1-yl)ethyl)cyclohexyl)ethanol,
- 2-(1-(2-(4-(2-bromo-5-isopropoxybenzyl)piperidin-1-yl)ethyl)cyclohexyl)ethanol,
- 2-(1-(2-(4-(2-bromo-5-chlorobenzyl)piperidin-1-yl)ethyl)cyclohexyl)ethanol,
- 2-(1-(2-(4-(2-bromo-5-fluorobenzyl)piperidin-1-yl)ethyl)cyclohexyl)ethanol,
- 2-(1-(3-(4-(2-bromo-5-methoxybenzyl)piperidin-1-yl)propyl)cyclohexyl)ethanol,
- 2-(1-(3-(4-(2-bromo-5-isopropoxybenzyl)piperidin-1-yl)propyl)cyclohexyl)ethanol,
- 2-(1-(3-(4-(2-bromo-5-chlorobenzyl)piperidin-1-yl)propyl)cyclohexyl)ethanol,
- 2-(1-(3-(4-(2-bromo-5-fluorobenzyl)piperidin-1-yl)propyl)cyclohexyl)ethanol and
- 2-(1-(2-(3-(2-bromo-5-methoxybenzyl)pyrrolidin-1-yl)ethyl)cyclohexyl)ethanol.

The following compounds may be produced in the same manner as in Example 65, 70, 72 or 73:

- 2-(2-(4-(2-bromo-5-methoxybenzyl)piperidin-1-yl)ethyl)-1,2,3,4-tetrahydroquinoline,
- 2-(2-(4-(2-bromo-5-isopropoxybenzyl)piperidin-1-yl)ethyl)-1,2,3,4-tetrahydroquinoline,
- 2-(2-(4-(2-bromo-5-ethoxybenzyl)piperidin-1-yl)ethyl)-1,2,3,4-tetrahydroquinoline,
- 2-(2-(4-(2-bromo-5-chlorobenzyl)piperidin-1-yl)ethyl)-1,2,3,4-tetrahydroquinoline,
- 2-(2-(4-(2-bromo-5-fluorobenzyl)piperidin-1-yl)ethyl)-1,2,3,4-tetrahydroquinoline,
- 2-(2-(3-(2-bromo-5-methoxybenzyl)pyrrolidin-1-yl)ethyl)-1,2,3,4-tetrahydroquinoline,
- 2-(2-(4-(2-chloro-5-methoxybenzyl)piperidin-1-yl)ethyl)-1,2,3,4-tetrahydroquinoline,
- 2-(2-(4-(2-chloro-5-isopropoxybenzyl)piperidin-1-yl)ethyl)-1,2,3,4-tetrahydroquinoline,
- 2-(2-(4-(2-chloro-5-ethoxybenzyl)piperidin-1-yl)ethyl)-1,2,3,4-tetrahydroquinoline,
- 2-(2-(4-(2,5-dichlorobenzyl)piperidin-1-yl)ethyl)-1,2,3,4-tetrahydroquinoline,

- 2-(2-(4-(2-chloro-5-fluorobenzyl)piperidin-1-yl)ethyl)-1,2,3,4-tetrahydroquinoline,
- 2-((4-(2-chloro-5-methoxybenzyl)piperidin-1-yl)methyl)-1,2,3,4-tetrahydroquinoline,
- 2-((4-(2-chloro-5-isopropoxybenzyl)piperidin-1-yl)methyl)-1,2,3,4-tetrahydroquinoline,
- 2-((4-(2-bromo-5-methoxybenzyl)piperidin-1-yl)methyl)-1-methyl-1,2,3,4-tetrahydroquinoline,
- 2-((4-(2-bromo-5-isopropoxybenzyl)piperidin-1-yl)methyl)-1-methyl-1,2,3,4-tetrahydroquinoline,
- 2-(2-(4-(2-bromo-5-methoxybenzyl)piperidin-1-yl)ethyl)-1-methyl-1,2,3,4-tetrahydroquinoline,
- 2-(2-(4-(2-bromo-5-isopropoxybenzyl)piperidin-1-yl)ethyl)-1-methyl-1,2,3,4-tetrahydroquinoline,
- 3-((4-(2-bromo-5-methoxybenzyl)piperidin-1-yl)methyl)-1-methyl-1,2,3,4-tetrahydroquinoline,
- 3-((4-(2-bromo-5-isopropoxybenzyl)piperidin-1-yl)methyl)-1-methyl-1,2,3,4-tetrahydroquinoline,
- 3-(2-(4-(2-bromo-5-methoxybenzyl)piperidin-1-yl)ethyl)-1,2,3,4-tetrahydroquinoline,
- 3-(2-(4-(2-bromo-5-isopropoxybenzyl)piperidin-1-yl)ethyl)-1,2,3,4-tetrahydroquinoline,
- 3-((4-(2-chloro-5-methoxybenzyl)piperidin-1-yl)methyl)-1,2,3,4-tetrahydroquinoline,
- 3-((4-(2-chloro-5-isopropoxybenzyl)piperidin-1-yl)methyl)-1,2,3,4-tetrahydroquinoline,
- 3-((4-(2-chloro-5-methoxybenzyl)piperidin-1-yl)methyl)-1-methyl-1,2,3,4-tetrahydroquinoline,
- 3-((4-(2-chloro-5-isopropoxybenzyl)piperidin-1-yl)methyl)-1-methyl-1,2,3,4-tetrahydroquinoline,
- 4-((4-(2-bromo-5-methoxybenzyl)piperidin-1-yl)methyl)-1,2,3,4-tetrahydroquinoline,
- 4-((4-(2-bromo-5-isopropoxybenzyl)piperidin-1-yl)methyl)-1,2,3,4-tetrahydroquinoline,
- 4-((4-(2-bromo-5-methoxybenzyl)piperidin-1-yl)methyl)-1-methyl-1,2,3,4-tetrahydroquinoline,
- 4-((4-(2-bromo-5-isopropoxybenzyl)piperidin-1-yl)methyl)-1-methyl-1,2,3,4-tetrahydroquinoline,
- 4-(2-(4-(2-bromo-5-methoxybenzyl)piperidin-1-yl)ethyl)-1,2,3,4-tetrahydroquinoline,
- 4-(2-(4-(2-bromo-5-isopropoxybenzyl)piperidin-1-yl)ethyl)-1,2,3,4-tetrahydroquinoline,
- 4-((4-(2-chloro-5-methoxybenzyl)piperidin-1-yl)methyl)-1,2,3,4-tetrahydroquinoline,
- 4-((4-(2-chloro-5-isopropoxybenzyl)piperidin-1-yl)methyl)-1,2,3,4-tetrahydroquinoline,
- 4-((4-(2-chloro-5-methoxybenzyl)piperidin-1-yl)methyl)-1-methyl-1,2,3,4-tetrahydroquinoline,
- 4-((4-(2-chloro-5-isopropoxybenzyl)piperidin-1-yl)methyl)-1-methyl-1,2,3,4-tetrahydroquinoline,
- 4-((4-(2-bromo-5-methoxybenzyl)piperidin-1-yl)methyl)-3,4-dihydroquinolin-2(1H)-one,
- 4-((4-(2-bromo-5-isopropoxybenzyl)piperidin-1-yl)methyl)-3,4-dihydroquinolin-2(1H)-one,
- 4-((4-(2-bromo-5-ethoxybenzyl)piperidin-1-yl)methyl)-3,4-dihydroquinolin-2(1H)-one,
- 4-((4-(2-bromo-5-chlorobenzyl)piperidin-1-yl)methyl)-3,4-dihydroquinolin-2(1H)-one,
- 4-((4-(2-bromo-5-fluorobenzyl)piperidin-1-yl)methyl)-3,4-dihydroquinolin-2(1H)-one,
- 4-((3-(2-bromo-5-methoxybenzyl)pyrrolidin-1-yl)methyl)-3,4-dihydroquinolin-2(1H)-one,
- 4-((4-(2-bromo-5-methoxybenzyl)piperidin-1-yl)methyl)-1-methyl-3,4-dihydroquinolin-2(1H)-one,
- 4-((4-(2-bromo-5-isopropoxybenzyl)piperidin-1-yl)methyl)-1-methyl-3,4-dihydroquinolin-2(1H)-one,
- 4-(2-(4-(2-bromo-5-methoxybenzyl)piperidin-1-yl)ethyl)-3,4-dihydroquinolin-2(1H)-one,
- 4-(2-(4-(2-bromo-5-isopropoxybenzyl)piperidin-1-yl)ethyl)-3,4-dihydroquinolin-2(1H)-one,
- 4-((4-(2-chloro-5-methoxybenzyl)piperidin-1-yl)methyl)-3,4-dihydroquinolin-2(1H)-one,
- 4-((4-(2-chloro-5-isopropoxybenzyl)piperidin-1-yl)methyl)-3,4-dihydroquinolin-2(1H)-one,
- 4-((4-(2-chloro-5-methoxybenzyl)piperidin-1-yl)methyl)-1-methyl-3,4-dihydroquinolin-2(1H)-one,
- 4-((4-(2-chloro-5-isopropoxybenzyl)piperidin-1-yl)methyl)-1-methyl-3,4-dihydroquinolin-2(1H)-one,
- 3-((4-(2-bromo-5-methoxybenzyl)piperidin-1-yl)methyl)-3,4-dihydroquinolin-2(1H)-one,
- 3-((4-(2-bromo-5-isopropoxybenzyl)piperidin-1-yl)methyl)-3,4-dihydroquinolin-2(1H)-one,
- 3-((4-(2-bromo-5-methoxybenzyl)piperidin-1-yl)methyl)-1-methyl-3,4-dihydroquinolin-2(1H)-one,
- 3-((4-(2-bromo-5-isopropoxybenzyl)piperidin-1-yl)methyl)-1-methyl-3,4-dihydroquinolin-2(1H)-one,
- 3-(2-(4-(2-bromo-5-methoxybenzyl)piperidin-1-yl)ethyl)-3,4-dihydroquinolin-2(1H)-one,
- 3-(2-(4-(2-bromo-5-isopropoxybenzyl)piperidin-1-yl)ethyl)-3,4-dihydroquinolin-2(1H)-one,
- 3-((4-(2-chloro-5-methoxybenzyl)piperidin-1-yl)methyl)-3,4-dihydroquinolin-2(1H)-one,
- 3-((4-(2-chloro-5-isopropoxybenzyl)piperidin-1-yl)methyl)-3,4-dihydroquinolin-2(1H)-one,
- 3-((4-(2-chloro-5-methoxybenzyl)piperidin-1-yl)methyl)-1-methyl-3,4-dihydroquinolin-2(1H)-one,
- 3-((4-(2-chloro-5-isopropoxybenzyl)piperidin-1-yl)methyl)-1-methyl-3,4-dihydroquinolin-2(1H)-one,
- 3-((4-(2-bromo-5-methoxybenzyl)piperidin-1-yl)methyl)-3,4-dihydro-2H-1,4-benzoxazine,
- 3-((4-(2-bromo-5-isopropoxybenzyl)piperidin-1-yl)methyl)-3,4-dihydro-2H-1,4-benzoxazine,
- 3-((4-(2-bromo-5-ethoxybenzyl)piperidin-1-yl)methyl)-3,4-dihydro-2H-1,4-benzoxazine,
- 3-((4-(2-bromo-5-chlorobenzyl)piperidin-1-yl)methyl)-3,4-dihydro-2H-1,4-benzoxazine,
- 3-((4-(2-bromo-5-fluorobenzyl)piperidin-1-yl)methyl)-3,4-dihydro-2H-1,4-benzoxazine,
- 3-((3-(2-bromo-5-methoxybenzyl)pyrrolidin-1-yl)methyl)-3,4-dihydro-2H-1,4-benzoxazine,
- 3-((3-(2-bromo-5-methoxyethoxybenzyl)pyrrolidin-1-yl)methyl)-3,4-dihydro-2H-1,4-benzoxazine,
- 3-((4-(2-bromo-5-methoxybenzyl)piperidin-1-yl)methyl)-4-methyl-3,4-dihydro-2H-1,4-benzoxazine,
- 3-((4-(2-bromo-5-isopropoxybenzyl)piperidin-1-yl)methyl)-4-methyl-3,4-dihydro-2H-1,4-benzoxazine,

- 3-(2-(4-(2-bromo-5-methoxybenzyl)piperidin-1-yl)ethyl)-3,4-dihydro-2H-1,4-benzoxazine,
- 3-(2-(4-(2-bromo-5-isopropoxybenzyl)piperidin-1-yl)ethyl)-3,4-dihydro-2H-1,4-benzoxazine,
- 3-((4-(2-chloro-5-methoxybenzyl)piperidin-1-yl)methyl)-3,4-dihydro-2H-1,4-benzoxazine,
- 3-((4-(2-chloro-5-isopropoxybenzyl)piperidin-1-yl)methyl)-3,4-dihydro-2H-1,4-benzoxazine,
- 3-((4-(2-chloro-5-methoxybenzyl)piperidin-1-yl)methyl)-4-methyl-3,4-dihydro-2H-1,4-benzoxazine,
- 3-((4-(2-chloro-5-isopropoxybenzyl)piperidin-1-yl)methyl)-4-methyl-3,4-dihydro-2H-1,4-benzoxazine,
- 2-((4-(2-bromo-5-methoxybenzyl)piperidin-1-yl)methyl)-3,4-dihydro-2H-1,4-benzoxazine,
- 2-((4-(2-bromo-5-isopropoxybenzyl)piperidin-1-yl)methyl)-3,4-dihydro-2H-1,4-benzoxazine,
- 2-((4-(2-bromo-5-methoxybenzyl)piperidin-1-yl)methyl)-4-methyl-3,4-dihydro-2H-1,4-benzoxazine,
- 2-((4-(2-bromo-5-isopropoxybenzyl)piperidin-1-yl)methyl)-4-methyl-3,4-dihydro-2H-1,4-benzoxazine,
- 2-(2-(4-(2-bromo-5-methoxybenzyl)piperidin-1-yl)ethyl)-3,4-dihydro-2H-1,4-benzoxazine,
- 2-(2-(4-(2-bromo-5-isopropoxybenzyl)piperidin-1-yl)ethyl)-3,4-dihydro-2H-1,4-benzoxazine,
- 2-((4-(2-chloro-5-methoxybenzyl)piperidin-1-yl)methyl)-3,4-dihydro-2H-1,4-benzoxazine,
- 2-((4-(2-chloro-5-isopropoxybenzyl)piperidin-1-yl)methyl)-3,4-dihydro-2H-1,4-benzoxazine,
- 2-((4-(2-bromo-5-methoxybenzyl)piperidin-1-yl)methyl)-2H-1,4-benzoxazin-3(4H)-one,
- 2-((4-(2-bromo-5-isopropoxybenzyl)piperidin-1-yl)methyl)-2H-1,4-benzoxazin-3(4H)-one,
- 2-((4-(2-bromo-5-ethoxybenzyl)piperidin-1-yl)methyl)-2H-1,4-benzoxazin-3(4H)-one,
- 2-((4-(2-bromo-5-chlorobenzyl)piperidin-1-yl)methyl)-2H-1,4-benzoxazin-3(4H)-one,
- 2-((4-(2-bromo-5-fluorobenzyl)piperidin-1-yl)methyl)-2H-1,4-benzoxazin-3(4H)-one,
- 2-((3-(2-bromo-5-methoxybenzyl)pyrrolidin-1-yl)methyl)-2H-1,4-benzoxazin-3(4H)-one,
- 2-((3-(2-bromo-5-methoxyethoxybenzyl)pyrrolidin-1-yl)methyl)-2H-1,4-benzoxazin-3(4H)-one,
- 2-((4-(2-bromo-5-methoxybenzyl)piperidin-1-yl)methyl)-4-methyl-2H-1,4-benzoxazin-3(4H)-one,
- 2-((4-(2-bromo-5-isopropoxybenzyl)piperidin-1-yl)methyl)-4-methyl-2H-1,4-benzoxazin-3(4H)-one,
- 2-(2-(4-(2-bromo-5-methoxybenzyl)piperidin-1-yl)ethyl)-2H-1,4-benzoxazin-3(4H)-one,
- 2-(2-(4-(2-bromo-5-isopropoxybenzyl)piperidin-1-yl)ethyl)-2H-1,4-benzoxazin-3(4H)-one,
- 2-((4-(2-chloro-5-methoxybenzyl)piperidin-1-yl)methyl)-2H-1,4-benzoxazin-3(4H)-one,
- 2-((4-(2-chloro-5-isopropoxybenzyl)piperidin-1-yl)methyl)-2H-1,4-benzoxazin-3(4H)-one,
- 2-((4-(2-chloro-5-methoxybenzyl)piperidin-1-yl)methyl)-4-methyl-2H-1,4-benzoxazin-3(4H)-one,
- 2-((4-(2-chloro-5-isopropoxybenzyl)piperidin-1-yl)methyl)-4-methyl-2H-1,4-benzoxazin-3(4H)-one,
- 3-((4-(2-bromo-5-methoxybenzyl)piperidin-1-yl)methyl)-1,3-dihydro-2H-indol-2-one,
- 3-((4-(2-bromo-5-isopropoxybenzyl)piperidin-1-yl)methyl)-1,3-dihydro-2H-indol-2-one,
- 3-((4-(2-bromo-5-ethoxybenzyl)piperidin-1-yl)methyl)-1,3-dihydro-2H-indol-2-one,
- 3-((4-(2-bromo-5-chlorobenzyl)piperidin-1-yl)methyl)-1,3-dihydro-2H-indol-2-one,
- 3-((4-(2-bromo-5-fluorobenzyl)piperidin-1-yl)methyl)-1,3-dihydro-2H-indol-2-one,
- 3-((3-(2-bromo-5-methoxybenzyl)pyrrolidin-1-yl)methyl)-1,3-dihydro-2H-indol-2-one,
- 3-((3-(2-bromo-5-methoxyethoxybenzyl)pyrrolidin-1-yl)methyl)-1,3-dihydro-2H-indol-2-one,
- 3-((4-(2-bromo-5-methoxybenzyl)piperidin-1-yl)methyl)-1-methyl-1,3-dihydro-2H-indol-2-one,
- 3-((4-(2-bromo-5-isopropoxybenzyl)piperidin-1-yl)methyl)-1-methyl-1,3-dihydro-2H-indol-2-one,
- 3-(2-(4-(2-bromo-5-methoxybenzyl)piperidin-1-yl)ethyl)-1,3-dihydro-2H-indol-2-one,
- 3-(2-(4-(2-bromo-5-isopropoxybenzyl)piperidin-1-yl)ethyl)-1,3-dihydro-2H-indol-2-one,
- 3-((4-(2-chloro-5-methoxybenzyl)piperidin-1-yl)methyl)-1,3-dihydro-2H-indol-2-one,
- 3-((4-(2-chloro-5-isopropoxybenzyl)piperidin-1-yl)methyl)-1,3-dihydro-2H-indol-2-one,
- 3-((4-(2-chloro-5-methoxybenzyl)piperidin-1-yl)methyl)-1-methyl-1,3-dihydro-2H-indol-2-one,
- 3-((4-(2-chloro-5-isopropoxybenzyl)piperidin-1-yl)methyl)-1-methyl-1,3-dihydro-2H-indol-2-one,
- 3-((4-(2-bromo-5-methoxybenzyl)piperidin-1-yl)methyl)indoline,
- 3-((4-(2-bromo-5-isopropoxybenzyl)piperidin-1-yl)methyl)indoline,
- 3-((4-(2-bromo-5-ethoxybenzyl)piperidin-1-yl)methyl)indoline,
- 3-((4-(2-bromo-5-chlorobenzyl)piperidin-1-yl)methyl)indoline,
- 3-((4-(2-bromo-5-fluorobenzyl)piperidin-1-yl)methyl)indoline,
- 3-((3-(2-bromo-5-methoxybenzyl)pyrrolidin-1-yl)methyl)indoline,
- 3-((3-(2-bromo-5-methoxyethoxybenzyl)pyrrolidin-1-yl)methyl)indoline,
- 3-((4-(2-bromo-5-methoxybenzyl)piperidin-1-yl)methyl)-1-methylindoline,
- 3-((4-(2-bromo-5-isopropoxybenzyl)piperidin-1-yl)methyl)-1-methylindoline,
- 3-((4-(2-bromo-5-ethoxybenzyl)piperidin-1-yl)methyl)-1-methylindoline,
- 3-((4-(2-bromo-5-chlorobenzyl)piperidin-1-yl)methyl)-1-methylindoline,
- 3-((4-(2-bromo-5-fluorobenzyl)piperidin-1-yl)methyl)-1-methylindoline,
- 3-((3-(2-bromo-5-methoxybenzyl)pyrrolidin-1-yl)methyl)-1-methylindoline,
- 3-((3-(2-bromo-5-methoxyethoxybenzyl)pyrrolidin-1-yl)methyl)-1-methylindoline,

- 3-(2-(4-(2-bromo-5-methoxybenzyl)piperidin-1-yl)ethyl)indoline,
- 3-(2-(4-(2-bromo-5-isopropoxybenzyl)piperidin-1-yl)ethyl)indoline,
- 3-((4-(2-chloro-5-methoxybenzyl)piperidin-1-yl)methyl)indoline,
- 3-((4-(2-chloro-5-isopropoxybenzyl)piperidin-1-yl)methyl)indoline,
- 3-((4-(2-chloro-5-methoxybenzyl)piperidin-1-yl)methyl)-1-methylindoline,
- 3-((4-(2-chloro-5-isopropoxybenzyl)piperidin-1-yl)methyl)-1-methylindoline,
- 2-((4-(2-bromo-5-methoxybenzyl)piperidin-1-yl)methyl)indoline,
- 2-((4-(2-bromo-5-isopropoxybenzyl)piperidin-1-yl)methyl)indoline,
- 2-((4-(2-bromo-5-methoxybenzyl)piperidin-1-yl)methyl)-1-methylindoline,
- 2-((4-(2-bromo-5-isopropoxybenzyl)piperidin-1-yl)methyl)-1-methylindoline,
- 2-(2-(4-(2-bromo-5-methoxybenzyl)piperidin-1-yl)ethyl)indoline,
- 2-(2-(4-(2-bromo-5-isopropoxybenzyl)piperidin-1-yl)ethyl)indoline,
- 2-((4-(2-chloro-5-methoxybenzyl)piperidin-1-yl)methyl)indoline,
- 2-((4-(2-chloro-5-isopropoxybenzyl)piperidin-1-yl)methyl)indoline,
- 2-((4-(2-chloro-5-methoxybenzyl)piperidin-1-yl)methyl)-1-methylindoline,
- 2-((4-(2-chloro-5-isopropoxybenzyl)piperidin-1-yl)methyl)-1-methylindoline,
- 5-((4-(2-bromo-5-methoxybenzyl)piperidin-1-yl)methyl)-3-phenyl-1,3-oxazolidin-2-one,
- 5-((4-(2-bromo-5-isopropoxybenzyl)piperidin-1-yl)methyl)-3-phenyl-1,3-oxazolidin-2-one
- 5-((4-(2-bromo-5-ethoxybenzyl)piperidin-1-yl)methyl)-3-phenyl-1,3-oxazolidin-2-one,
- 5-((4-(2-bromo-5-chlorobenzyl)piperidin-1-yl)methyl)-3-phenyl-1,3-oxazolidin-2-one,
- 5-((4-(2-bromo-5-fluorobenzyl)piperidin-1-yl)methyl)-3-phenyl-1,3-oxazolidin-2-one,
- 5-((3-(2-bromo-5-methoxybenzyl)pyrrolidin-1-yl)methyl)-3-phenyl-1,3-oxazolidin-2-one,
- 5-((3-(2-bromo-5-methoxyethoxybenzyl)pyrrolidin-1-yl)methyl)-3-phenyl-1,3-oxazolidin-2-one,
- 5-(2-(4-(2-bromo-5-methoxybenzyl)piperidin-1-yl)ethyl)-3-phenyl-1,3-oxazolidin-2-one,
- 5-(2-(4-(2-bromo-5-isopropoxybenzyl)piperidin-1-yl)ethyl)-3-phenyl-1,3-oxazolidin-2-one,
- 5-((4-(2-chloro-5-methoxybenzyl)piperidin-1-yl)methyl)-3-phenyl-1,3-oxazolidin-2-one,
- 5-((4-(2-chloro-5-isopropoxybenzyl)piperidin-1-yl)methyl)-3-phenyl-1,3-oxazolidin-2-one,
- 5-((4-(2-chloro-5-ethoxybenzyl)piperidin-1-yl)methyl)-3-phenyl-1,3-oxazolidin-2-one,
- 5-((4-(2,5-dichlorobenzyl)piperidin-1-yl)methyl)-3-phenyl-1,3-oxazolidin-2-one,
- 5-((4-(2-chloro-5-fluorobenzyl)piperidin-1-yl)methyl)-3-phenyl-1,3-oxazolidin-2-one,
- 5-(2-(3-(2-bromo-5-methoxybenzyl)pyrrolidin-1-yl)ethyl)-3-phenyl-1,3-oxazolidin-2-one,
- 5-(2-(3-(2-bromo-5-methoxyethoxybenzyl)pyrrolidin-1-yl)ethyl)-3-phenyl-1,3-oxazolidin-2-one,
- 5-((4-(2-bromo-5-isopropoxybenzyl)piperidin-1-yl)methyl)-3-(4-methoxyphenyl)-1,3-oxazolidin-2-one,
- 5-((4-(2-bromo-5-ethoxybenzyl)piperidin-1-yl)methyl)-3-(4-methoxyphenyl)-1,3-oxazolidin-2-one,
- 5-((4-(2-bromo-5-chlorobenzyl)piperidin-1-yl)methyl)-3-(4-methoxyphenyl)-1,3-oxazolidin-2-one,
- 5-((4-(2-bromo-5-fluorobenzyl)piperidin-1-yl)methyl)-3-(4-methoxyphenyl)-1,3-oxazolidin-2-one,
- 5-((3-(2-bromo-5-methoxybenzyl)pyrrolidin-1-yl)methyl)-3-(4-methoxyphenyl)-1,3-oxazolidin-2-one,
- 5-((3-(2-bromo-5-methoxyethoxybenzyl)pyrrolidin-1-yl)methyl)-3-(4-methoxyphenyl)-1,3-oxazolidin-2-one,
- 5-(2-(4-(2-bromo-5-methoxybenzyl)piperidin-1-yl)ethyl)-3-(4-methoxyphenyl)-1,3-oxazolidin-2-one,
- 5-(2-(4-(2-bromo-5-isopropoxybenzyl)piperidin-1-yl)ethyl)-3-(4-methoxyphenyl)-1,3-oxazolidin-2-one,
- 5-(2-(4-(2-bromo-5-ethoxybenzyl)piperidin-1-yl)ethyl)-3-(4-methoxyphenyl)-1,3-oxazolidin-2-one,
- 5-(2-(4-(2-bromo-5-chlorobenzyl)piperidin-1-yl)ethyl)-3-(4-methoxyphenyl)-1,3-oxazolidin-2-one,
- 5-(2-(4-(2-bromo-5-fluorobenzyl)piperidin-1-yl)ethyl)-3-(4-methoxyphenyl)-1,3-oxazolidin-2-one,
- 5-(2-(3-(2-bromo-5-methoxybenzyl)pyrrolidin-1-yl)ethyl)-3-(4-methoxyphenyl)-1,3-oxazolidin-2-one,
- 5-(2-(3-(2-bromo-5-methoxyethoxybenzyl)pyrrolidin-1-yl)ethyl)-3-(4-methoxyphenyl)-1,3-oxazolidin-2-one,
- 5-((4-(2-chloro-5-methoxybenzyl)piperidin-1-yl)methyl)-3-(4-methoxyphenyl)-1,3-oxazolidin-2-one,
- 5-((4-(2-chloro-5-isopropoxybenzyl)piperidin-1-yl)methyl)-3-(4-methoxyphenyl)-1,3-oxazolidin-2-one,
- 5-((4-(2-chloro-5-ethoxybenzyl)piperidin-1-yl)methyl)-3-(4-methoxyphenyl)-1,3-oxazolidin-2-one,
- 5-((4-(2,5-dichlorobenzyl)piperidin-1-yl)methyl)-3-(4-methoxyphenyl)-1,3-oxazolidin-2-one,
- 5-((4-(2-chloro-5-fluorobenzyl)piperidin-1-yl)methyl)-3-(4-methoxyphenyl)-1,3-oxazolidin-2-one,
- 5-((4-(2-bromo-5-methoxybenzyl)piperidin-2-yl)methyl)-3-(4-chlorophenyl)-1,3-oxazolidin-2-one,
- 5-((4-(2-bromo-5-isopropoxybenzyl)piperidin-1-yl)methyl)-3-(4-chlorophenyl)-1,3-oxazolidin-2-one,
- 5-((4-(2-bromo-5-ethoxybenzyl)piperidin-1-yl)methyl)-3-(4-chlorophenyl)-1,3-oxazolidin-2-one,
- 5-((4-(2-bromo-5-chlorobenzyl)piperidin-1-yl)methyl)-3-(4-chlorophenyl)-1,3-oxazolidin-2-one,
- 5-((4-(2-bromo-5-fluorobenzyl)piperidin-1-yl)methyl)-3-(4-chlorophenyl)-1,3-oxazolidin-2-one,
- 5-((3-(2-bromo-5-methoxybenzyl)pyrrolidin-1-yl)methyl)-3-(4-chlorophenyl)-1,3-oxazolidin-2-one,
- 5-((3-(2-bromo-5-methoxyethoxybenzyl)pyrrolidin-1-yl)methyl)-3-(4-chlorophenyl)-1,3-oxazolidin-2-one,
- 5-(2-(4-(2-bromo-5-methoxybenzyl)piperidin-1-yl)ethyl)-3-(4-chlorophenyl)-1,3-oxazolidin-2-one,

- 5-(2-(4-(2-bromo-5-isopropoxybenzyl)piperidin-1-yl)ethyl)-3-(4-chlorophenyl)-1,3-oxazolidin-2-one,
- 5-(2-(4-(2-bromo-5-ethoxybenzyl)piperidin-1-yl)ethyl)-3-(4-chlorophenyl)-1,3-oxazolidin-2-one,
- 5-(2-(4-(2-bromo-5-chlorobenzyl)piperidin-1-yl)ethyl)-3-(4-chlorophenyl)-1,3-oxazolidin-2-one,
- 5-(2-(4-(2-bromo-5-fluorobenzyl)piperidin-1-yl)ethyl)-3-(4-chlorophenyl)-1,3-oxazolidin-2-one,
- 5-(2-(3-(2-bromo-5-methoxybenzyl)pyrrolidin-1-yl)ethyl)-3-(4-chlorophenyl)-1,3-oxazolidin-2-one,
- 5-(2-(3-(2-bromo-5-methoxyethoxybenzyl)pyrrolidin-1-yl)ethyl)-3-(4-chlorophenyl)-1,3-oxazolidin-2-one,
- 5-((4-(2-chloro-5-methoxybenzyl)piperidin-1-yl)methyl)-3-(4-chlorophenyl)-1,3-oxazolidin-2-one,
- 5-((4-(2-chloro-5-isopropoxybenzyl)piperidin-1-yl)methyl)-3-(4-chlorophenyl)-1,3-oxazolidin-2-one,
- 5-((4-(2-chloro-5-ethoxybenzyl)piperidin-1-yl)methyl)-3-(4-chlorophenyl)-1,3-oxazolidin-2-one,
- 5-((4-(2,5-dichlorobenzyl)piperidin-1-yl)methyl)-3-(4-chlorophenyl)-1,3-oxazolidin-2-one,
- 5-((4-(2-chloro-5-fluorobenzyl)piperidin-1-yl)methyl)-3-(4-chlorophenyl)-1,3-oxazolidin-2-one,
- 5-((4-(2-bromo-5-isopropoxybenzyl)piperidin-1-yl)methyl)-3-cyclohexyl-1,3-oxazolidin-2-one,
- 5-((4-(2-bromo-5-ethoxybenzyl)piperidin-1-yl)methyl)-3-cyclohexyl-1,3-oxazolidin-2-one,
- 5-((4-(2-bromo-5-chlorobenzyl)piperidin-1-yl)methyl)-3-cyclohexyl-1,3-oxazolidin-2-one,
- 5-((4-(2-bromo-5-fluorobenzyl)piperidin-1-yl)methyl)-3-cyclohexyl-1,3-oxazolidin-2-one,
- 5-((3-(2-bromo-5-methoxybenzyl)pyrrolidin-1-yl)methyl)-3-cyclohexyl-1,3-oxazolidin-2-one,
- 5-((3-(2-bromo-5-methoxyethoxybenzyl)pyrrolidin-1-yl)methyl)-3-cyclohexyl-1,3-oxazolidin-2-one,
- 5-(2-(4-(2-bromo-5-methoxybenzyl)piperidin-1-yl)ethyl)-3-cyclohexyl-1,3-oxazolidin-2-one,
- 5-(2-(4-(2-bromo-5-isopropoxybenzyl)piperidin-1-yl)ethyl)-3-cyclohexyl-1,3-oxazolidin-2-one,
- 5-(2-(4-(2-bromo-5-ethoxybenzyl)piperidin-1-yl)ethyl)-3-cyclohexyl-1,3-oxazolidin-2-one,
- 5-(2-(4-(2-bromo-5-chlorobenzyl)piperidin-1-yl)ethyl)-3-cyclohexyl-1,3-oxazolidin-2-one,
- 5-(2-(4-(2-bromo-5-fluorobenzyl)piperidin-1-yl)ethyl)-3-cyclohexyl-1,3-oxazolidin-2-one,
- 5-(2-(3-(2-bromo-5-methoxybenzyl)pyrrolidin-1-yl)ethyl)-3-cyclohexyl-1,3-oxazolidin-2-one,
- 5-(2-(3-(2-bromo-5-methoxyethoxybenzyl)pyrrolidin-1-yl)ethyl)-3-cyclohexyl-1,3-oxazolidin-2-one,
- 5-((4-(2-chloro-5-methoxybenzyl)piperidin-1-yl)methyl)-3-cyclohexyl-1,3-oxazolidin-2-one,
- 5-((4-(2-chloro-5-isopropoxybenzyl)piperidin-1-yl)methyl)-3-cyclohexyl-1,3-oxazolidin-2-one,
- 5-((4-(2-chloro-5-ethoxybenzyl)piperidin-1-yl)methyl)-3-cyclohexyl-1,3-oxazolidin-2-one,
- 5-((4-(2,5-dichlorobenzyl)piperidin-1-yl)methyl)-3-cyclohexyl-1,3-oxazolidin-2-one,
- 5-((4-(2-chloro-5-fluorobenzyl)piperidin-1-yl)methyl)-3-cyclohexyl-1,3-oxazolidin-2-one,
- 5-((4-(2-bromo-5-methoxybenzyl)piperidin-1-yl)methyl)-3-isopropyl-1,3-oxazolidin-2-one,
- 5-((4-(2-bromo-5-isopropoxybenzyl)piperidin-1-yl)methyl)-3-isopropyl-1,3-oxazolidin-2-one,
- 5-((4-(2-bromo-5-ethoxybenzyl)piperidin-1-yl)methyl)-3-isopropyl-1,3-oxazolidin-2-one,
- 5-((4-(2-bromo-5-chlorobenzyl)piperidin-1-yl)methyl)-3-isopropyl-1,3-oxazolidin-2-one,
- 5-((4-(2-bromo-5-fluorobenzyl)piperidin-1-yl)methyl)-3-isopropyl-1,3-oxazolidin-2-one,
- 5-((3-(2-bromo-5-methoxybenzyl)pyrrolidin-1-yl)methyl)-3-isopropyl-1,3-oxazolidin-2-one,
- 5-((3-(2-bromo-5-methoxyethoxybenzyl)pyrrolidin-1-yl)methyl)-3-isopropyl-1,3-oxazolidin-2-one,
- 5-(2-(4-(2-bromo-5-methoxybenzyl)piperidin-1-yl)ethyl)-3-isopropyl-1,3-oxazolidin-2-one,
- 5-(2-(4-(2-bromo-5-isopropoxybenzyl)piperidin-1-yl)ethyl)-3-isopropyl-1,3-oxazolidin-2-one,
- 5-(2-(4-(2-bromo-5-ethoxybenzyl)piperidin-1-yl)ethyl)-3-isopropyl-1,3-oxazolidin-2-one,
- 5-(2-(4-(2-bromo-5-chlorobenzyl)piperidin-1-yl)ethyl)-3-isopropyl-1,3-oxazolidin-2-one,
- 5-(2-(4-(2-bromo-5-fluorobenzyl)piperidin-1-yl)ethyl)-3-isopropyl-1,3-oxazolidin-2-one,
- 5-(2-(3-(2-bromo-5-methoxybenzyl)pyrrolidin-1-yl)ethyl)-3-isopropyl-1,3-oxazolidin-2-one,
- 5-(2-(3-(2-bromo-5-methoxyethoxybenzyl)pyrrolidin-1-yl)ethyl)-3-isopropyl-1,3-oxazolidin-2-one,
- 5-((4-(2-chloro-5-methoxybenzyl)piperidin-1-yl)methyl)-3-isopropyl-1,3-oxazolidin-2-one,
- 5-((4-(2-chloro-5-isopropoxybenzyl)piperidin-1-yl)methyl)-3-isopropyl-1,3-oxazolidin-2-one,
- 5-((4-(2-chloro-5-ethoxybenzyl)piperidin-1-yl)methyl)-3-isopropyl-1,3-oxazolidin-2-one,
- 5-((4-(2,5-dichlorobenzyl)piperidin-1-yl)methyl)-3-isopropyl-1,3-oxazolidin-2-one,
- 5-((4-(2-chloro-5-fluorobenzyl)piperidin-1-yl)methyl)-3-isopropyl-1,3-oxazolidin-2-one,
- 5-((4-(2-bromo-5-methoxybenzyl)piperidin-1-yl)methyl)-3-propyl-1,3-oxazolidin-2-one,
- 5-((4-(2-bromo-5-isopropoxybenzyl)piperidin-1-yl)methyl)-3-propyl-1,3-oxazolidin-2-one,
- 5-((4-(2-bromo-5-ethoxybenzyl)piperidin-1-yl)methyl)-3-propyl-1,3-oxazolidin-2-one,
- 5-((4-(2-bromo-5-chlorobenzyl)piperidin-1-yl)methyl)-3-propyl-1,3-oxazolidin-2-one,
- 5-((4-(2-bromo-5-fluorobenzyl)piperidin-1-yl)methyl)-3-propyl-1,3-oxazolidin-2-one,
- 5-((3-(2-bromo-5-methoxybenzyl)pyrrolidin-1-yl)methyl)-3-propyl-1,3-oxazolidin-2-one,
- 5-((3-(2-bromo-5-methoxyethoxybenzyl)pyrrolidin-1-yl)methyl)-3-propyl-1,3-oxazolidin-2-one,
- 5-(2-(4-(2-bromo-5-methoxybenzyl)piperidin-1-yl)ethyl)-3-propyl-1,3-oxazolidin-2-one,
- 5-(2-(4-(2-bromo-5-isopropoxybenzyl)piperidin-1-yl)ethyl)-3-propyl-1,3-oxazolidin-2-one,
- 5-(2-(4-(2-bromo-5-ethoxybenzyl)piperidin-1-yl)ethyl)-3-propyl-1,3-oxazolidin-2-one,

- 5-(2-(4-(2-bromo-5-chlorobenzyl)piperidin-1-yl)ethyl)-3-propyl-1,3-oxazolidin-2-one,
- 5-(2-(4-(2-bromo-5-fluorobenzyl)piperidin-1-yl)ethyl)-3-propyl-1,3-oxazolidin-2-one,
- 5-(2-(3-(2-bromo-5-methoxybenzyl)pyrrolidin-1-yl)ethyl)-3-propyl-1,3-oxazolidin-2-one,
- 5-(2-(3-(2-bromo-5-methoxyethoxybenzyl)pyrrolidin-1-yl)ethyl)-3-propyl-1,3-oxazolidin-2-one,
- 5-((4-(2-chloro-5-methoxybenzyl)piperidin-1-yl)methyl)-3-propyl-1,3-oxazolidin-2-one,
- 5-((4-(2-chloro-5-isopropoxybenzyl)piperidin-1-yl)methyl)-3-propyl-1,3-oxazolidin-2-one,
- 5-((4-(2-chloro-5-ethoxybenzyl)piperidin-1-yl)methyl)-3-propyl-1,3-oxazolidin-2-one,
- 5-((4-(2,5-dichlorobenzyl)piperidin-1-yl)methyl)-3-propyl-1,3-oxazolidin-2-one,
- 5-((4-(2-chloro-5-fluorobenzyl)piperidin-1-yl)methyl)-3-propyl-1,3-oxazolidin-2-one,
- 5-((4-(2-bromo-5-methoxybenzyl)piperidin-1-yl)methyl)-3-benzyl-1,3-oxazolidin-2-one,
- 5-((4-(2-bromo-5-isopropoxybenzyl)piperidin-1-yl)methyl)-3-benzyl-1,3-oxazolidin-2-one,
- 5-((4-(2-bromo-5-ethoxybenzyl)piperidin-1-yl)methyl)-3-benzyl-1,3-oxazolidin-2-one,
- 5-((4-(2-bromo-5-chlorobenzyl)piperidin-1-yl)methyl)-3-benzyl-1,3-oxazolidin-2-one,
- 5-((4-(2-bromo-5-fluorobenzyl)piperidin-1-yl)methyl)-3-benzyl-1,3-oxazolidin-2-one,
- 5-((3-(2-bromo-5-methoxybenzyl)pyrrolidin-1-yl)methyl)-3-benzyl-1,3-oxazolidin-2-one,
- 5-((3-(2-bromo-5-methoxyethoxybenzyl)pyrrolidin-1-yl)methyl)-3-benzyl-1,3-oxazolidin-2-one,
- 5-(2-(4-(2-bromo-5-methoxybenzyl)piperidin-1-yl)ethyl)-3-benzyl-1,3-oxazolidin-2-one,
- 5-(2-(4-(2-bromo-5-isopropoxybenzyl)piperidin-1-yl)ethyl)-3-benzyl-1,3-oxazolidin-2-one,
- 5-(2-(4-(2-bromo-5-ethoxybenzyl)piperidin-1-yl)ethyl)-3-benzyl-1,3-oxazolidin-2-one,
- 5-(2-(4-(2-bromo-5-chlorobenzyl)piperidin-1-yl)ethyl)-3-benzyl-1,3-oxazolidin-2-one,
- 5-(2-(4-(2-bromo-5-fluorobenzyl)piperidin-1-yl)ethyl)-3-benzyl-1,3-oxazolidin-2-one,
- 5-(2-(3-(2-bromo-5-methoxybenzyl)pyrrolidin-1-yl)ethyl)-3-benzyl-1,3-oxazolidin-2-one,
- 5-(2-(3-(2-bromo-5-methoxyethoxybenzyl)pyrrolidin-1-yl)ethyl)-3-benzyl-1,3-oxazolidin-2-one,
- 5-((4-(2-chloro-5-methoxybenzyl)piperidin-1-yl)methyl)-3-benzyl-1,3-oxazolidin-2-one,
- 5-((4-(2-chloro-5-isopropoxybenzyl)piperidin-1-yl)methyl)-3-benzyl-1,3-oxazolidin-2-one,
- 5-((4-(2-chloro-5-ethoxybenzyl)piperidin-1-yl)methyl)-3-benzyl-1,3-oxazolidin-2-one,
- 5-((4-(2,5-dichlorobenzyl)piperidin-1-yl)methyl)-3-benzyl-1,3-oxazolidin-2-one,
- 5-((4-(2-chloro-5-fluorobenzyl)piperidin-1-yl)methyl)-3-benzyl-1,3-oxazolidin-2-one,
- 5-((4-(2-bromo-5-methoxybenzyl)piperidin-1-yl)methyl)-3-cyclopropylmethyl-1,3-oxazolidin-2-one,
- 5-((4-(2-bromo-5-isopropoxybenzyl)piperidin-1-yl)methyl)-3-cyclopropylmethyl-1,3-oxazolidin-2-one,
- 5-((4-(2-bromo-5-ethoxybenzyl)piperidin-1-yl)methyl)-3-cyclopropylmethyl-1,3-oxazolidin-2-one,
- 5-((4-(2-bromo-5-chlorobenzyl)piperidin-1-yl)methyl)-3-cyclopropylmethyl-1,3-oxazolidin-2-one,
- 5-((4-(2-bromo-5-fluorobenzyl)piperidin-1-yl)methyl)-3-cyclopropylmethyl-1,3-oxazolidin-2-one,
- 5-((3-(2-bromo-5-methoxybenzyl)pyrrolidin-1-yl)methyl)-3-cyclopropylmethyl-1,3-oxazolidin-2-one,
- 5-((3-(2-bromo-5-methoxyethoxybenzyl)pyrrolidin-1-yl)methyl)-3-cyclopropylmethyl-1,3-oxazolidin-2-one,
- 5-(2-(4-(2-bromo-5-methoxybenzyl)piperidin-1-yl)ethyl)-3-cyclopropylmethyl-1,3-oxazolidin-2-one,
- 5-(2-(4-(2-bromo-5-isopropoxybenzyl)piperidin-1-yl)ethyl)-3-cyclopropylmethyl-1,3-oxazolidin-2-one,
- 5-(2-(4-(2-bromo-5-ethoxybenzyl)piperidin-1-yl)ethyl)-3-cyclopropylmethyl-1,3-oxazolidin-2-one,
- 5-(2-(4-(2-bromo-5-chlorobenzyl)piperidin-1-yl)ethyl)-3-cyclopropylmethyl-1,3-oxazolidin-2-one,
- 5-(2-(4-(2-bromo-5-fluorobenzyl)piperidin-1-yl)ethyl)-3-cyclopropylmethyl-1,3-oxazolidin-2-one,
- 5-(2-(3-(2-bromo-5-methoxybenzyl)pyrrolidin-1-yl)ethyl)-3-cyclopropylmethyl-1,3-oxazolidin-2-one,
- 5-(2-(3-(2-bromo-5-methoxyethoxybenzyl)pyrrolidin-1-yl)ethyl)-3-cyclopropylmethyl-1,3-oxazolidin-2-one,
- 5-((4-(2-chloro-5-methoxybenzyl)piperidin-1-yl)methyl)-3-cyclopropylmethyl-1,3-oxazolidin-2-one,
- 5-((4-(2-chloro-5-isopropoxybenzyl)piperidin-1-yl)methyl)-3-cyclopropylmethyl-1,3-oxazolidin-2-one,
- 5-((4-(2-chloro-5-ethoxybenzyl)piperidin-1-yl)methyl)-3-cyclopropylmethyl-1,3-oxazolidin-2-one,
- 5-((4-(2,5-dichlorobenzyl)piperidin-1-yl)methyl)-3-cyclopropylmethyl-1,3-oxazolidin-2-one,
- 5-((4-(2-chloro-5-fluorobenzyl)piperidin-1-yl)methyl)-3-cyclopropylmethyl-1,3-oxazolidin-2-one,
- 5-((4-(2-bromo-5-methoxybenzyl)piperidin-1-yl)methyl)-3-phenyl-1,3-thiazolidin-2-one,
- 5-((4-(2-bromo-5-isopropoxybenzyl)piperidin-1-yl)methyl)-3-phenyl-1,3-thiazolidin-2-one,
- 5-((4-(2-bromo-5-ethoxybenzyl)piperidin-1-yl)methyl)-3-phenyl-1,3-thiazolidin-2-one,
- 5-((4-(2-bromo-5-chlorobenzyl)piperidin-1-yl)methyl)-3-phenyl-1,3-thiazolidin-2-one,
- 5-((4-(2-bromo-5-fluorobenzyl)piperidin-1-yl)methyl)-3-phenyl-1,3-thiazolidin-2-one,
- 5-((3-(2-bromo-5-methoxybenzyl)pyrrolidin-1-yl)methyl)-3-phenyl-1,3-thiazolidin-2-one,
- 5-((3-(2-bromo-5-methoxyethoxybenzyl)pyrrolidin-1-yl)methyl)-3-phenyl-1,3-thiazolidin-2-one,
- 5-(2-(4-(2-bromo-5-methoxybenzyl)piperidin-1-yl)ethyl)-3-phenyl-1,3-thiazolidin-2-one,
- 5-(2-(4-(2-bromo-5-isopropoxybenzyl)piperidin-1-yl)ethyl)-3-phenyl-1,3-thiazolidin-2-one,
- 5-(2-(4-(2-bromo-5-ethoxybenzyl)piperidin-1-yl)ethyl)-3-phenyl-1,3-thiazolidin-2-one,
- 5-(2-(4-(2-bromo-5-chlorobenzyl)piperidin-1-yl)ethyl)-3-phenyl-1,3-thiazolidin-2-one,

- 5-(2-(4-(2-bromo-5-fluorobenzyl)piperidin-1-yl)ethyl)-3-phenyl-1,3-thiazolidin-2-one,
- 5-((4-(2-chloro-5-methoxybenzyl)piperidin-1-yl)methyl)-3-phenyl-1,3-thiazolidin-2-one,
- 5-((4-(2-chloro-5-isopropoxybenzyl)piperidin-1-yl)methyl)-3-phenyl-1,3-thiazolidin-2-one,
- 5-((4-(2-chloro-5-ethoxybenzyl)piperidin-1-yl)methyl)-3-phenyl-1,3-thiazolidin-2-one,
- 5-((4-(2,5-dichlorobenzyl)piperidin-1-yl)methyl)-3-phenyl-1,3-thiazolidin-2-one,
- 5-((4-(2-chloro-5-fluorobenzyl)piperidin-1-yl)methyl)-3-phenyl-1,3-thiazolidin-2-one,
- 5-((4-(2-bromo-5-methoxybenzyl)piperidin-1-yl)methyl)-3-(4-methoxyphenyl)-1,3-thiazolidin-2-one,
- 5-((4-(2-bromo-5-isopropoxybenzyl)piperidin-1-yl)methyl)-3-(4-methoxyphenyl)-1,3-thiazolidin-2-one,
- 5-((4-(2-bromo-5-ethoxybenzyl)piperidin-1-yl)methyl)-3-(4-methoxyphenyl)-1,3-thiazolidin-2-one,
- 5-((4-(2-bromo-5-chlorobenzyl)piperidin-1-yl)methyl)-3-(4-methoxyphenyl)-1,3-thiazolidin-2-one,
- 5-((4-(2-bromo-5-fluorobenzyl)piperidin-1-yl)methyl)-3-(4-methoxyphenyl)-1,3-thiazolidin-2-one,
- 5-(2-(4-(2-bromo-5-methoxybenzyl)piperidin-1-yl)ethyl)-3-(4-methoxyphenyl)-1,3-thiazolidin-2-one,
- 5-(2-(4-(2-bromo-5-isopropoxybenzyl)piperidin-1-yl)ethyl)-3-(4-methoxyphenyl)-1,3-thiazolidin-2-one,
- 5-(2-(4-(2-bromo-5-ethoxybenzyl)piperidin-1-yl)ethyl)-3-(4-methoxyphenyl)-1,3-thiazolidin-2-one,
- 5-(2-(4-(2-bromo-5-chlorobenzyl)piperidin-1-yl)ethyl)-3-(4-methoxyphenyl)-1,3-thiazolidin-2-one,
- 5-(2-(4-(2-bromo-5-fluorobenzyl)piperidin-1-yl)ethyl)-3-(4-methoxyphenyl)-1,3-thiazolidin-2-one,
- 5-((4-(2-chloro-5-methoxybenzyl)piperidin-1-yl)methyl)-3-(4-methoxyphenyl)-1,3-thiazolidin-2-one,
- 5-((4-(2-chloro-5-isopropoxybenzyl)piperidin-1-yl)methyl)-3-(4-methoxyphenyl)-1,3-thiazolidin-2-one,
- 5-((4-(2-chloro-5-ethoxybenzyl)piperidin-1-yl)methyl)-3-(4-methoxyphenyl)-1,3-thiazolidin-2-one,
- 5-((4-(2,5-dichlorobenzyl)piperidin-1-yl)methyl)-3-(4-methoxyphenyl)-1,3-thiazolidin-2-one,
- 5-((4-(2-chloro-5-fluorobenzyl)piperidin-1-yl)methyl)-3-(4-methoxyphenyl)-1,3-thiazolidin-2-one,
- 5-((4-(2-bromo-5-methoxybenzyl)piperidin-1-yl)methyl)-3-(4-chlorophenyl)-1,3-thiazolidin-2-one,
- 5-((4-(2-bromo-5-isopropoxybenzyl)piperidin-1-yl)methyl)-3-(4-chlorophenyl)-1,3-thiazolidin-2-one,
- 5-((4-(2-bromo-5-ethoxybenzyl)piperidin-1-yl)methyl)-3-(4-chlorophenyl)-1,3-thiazolidin-2-one,
- 5-((4-(2-bromo-5-chlorobenzyl)piperidin-1-yl)methyl)-3-(4-chlorophenyl)-1,3-thiazolidin-2-one,
- 5-((4-(2-bromo-5-fluorobenzyl)piperidin-1-yl)methyl)-3-(4-chlorophenyl)-1,3-thiazolidin-2-one,
- 5-(2-(4-(2-bromo-5-methoxybenzyl)piperidin-1-yl)ethyl)-3-(4-chlorophenyl)-1,3-thiazolidin-2-one,
- 5-(2-(4-(2-bromo-5-isopropoxybenzyl)piperidin-1-yl)ethyl)-3-(4-chlorophenyl)-1,3-thiazolidin-2-one,
- 5-(2-(4-(2-bromo-5-ethoxybenzyl)piperidin-1-yl)ethyl)-3-(4-chlorophenyl)-1,3-thiazolidin-2-one,
- 5-(2-(4-(2-bromo-5-chlorobenzyl)piperidin-1-yl)ethyl)-3-(4-chlorophenyl)-1,3-thiazolidin-2-one,
- 5-(2-(4-(2-bromo-5-fluorobenzyl)piperidin-1-yl)ethyl)-3-(4-chlorophenyl)-1,3-thiazolidin-2-one,
- 5-((4-(2-chloro-5-methoxybenzyl)piperidin-1-yl)methyl)-3-(4-chlorophenyl)-1,3-thiazolidin-2-one,
- 5-((4-(2-chloro-5-isopropoxybenzyl)piperidin-1-yl)methyl)-3-(4-chlorophenyl)-1,3-thiazolidin-2-one,
- 5-((4-(2-chloro-5-ethoxybenzyl)piperidin-1-yl)methyl)-3-(4-chlorophenyl)-1,3-thiazolidin-2-one,
- 5-((4-(2,5-dichlorobenzyl)piperidin-1-yl)methyl)-3-(4-chlorophenyl)-1,3-thiazolidin-2-one,
- 5-((4-(2-chloro-5-fluorobenzyl)piperidin-1-yl)methyl)-3-(4-chlorophenyl)-1,3-thiazolidin-2-one,
- 5-((4-(2-bromo-5-methoxybenzyl)piperidin-1-yl)methyl)-3-cyclohexyl-1,3-thiazolidin-2-one,
- 5-((4-(2-bromo-5-isopropoxybenzyl)piperidin-1-yl)methyl)-3-cyclohexyl-1,3-thiazolidin-2-one,
- 5-((4-(2-bromo-5-ethoxybenzyl)piperidin-1-yl)methyl)-3-cyclohexyl-1,3-thiazolidin-2-one,
- 5-((4-(2-bromo-5-chlorobenzyl)piperidin-1-yl)methyl)-3-cyclohexyl-1,3-thiazolidin-2-one,
- 5-((4-(2-bromo-5-fluorobenzyl)piperidin-1-yl)methyl)-3-cyclohexyl-1,3-thiazolidin-2-one,
- 5-(2-(4-(2-bromo-5-methoxybenzyl)piperidin-1-yl)ethyl)-3-cyclohexyl-1,3-thiazolidin-2-one,
- 5-(2-(4-(2-bromo-5-isopropoxybenzyl)piperidin-1-yl)ethyl)-3-cyclohexyl-1,3-thiazolidin-2-one,
- 5-(2-(4-(2-bromo-5-ethoxybenzyl)piperidin-1-yl)ethyl)-3-cyclohexyl-1,3-thiazolidin-2-one,
- 5-(2-(4-(2-bromo-5-chlorobenzyl)piperidin-1-yl)ethyl)-3-cyclohexyl-1,3-thiazolidin-2-one,
- 5-(2-(4-(2-bromo-5-fluorobenzyl)piperidin-1-yl)ethyl)-3-cyclohexyl-1,3-thiazolidin-2-one,
- 5-((4-(2-chloro-5-methoxybenzyl)piperidin-1-yl)methyl)-3-cyclohexyl-1,3-thiazolidin-2-one,
- 5-((4-(2-chloro-5-isopropoxybenzyl)piperidin-1-yl)methyl)-3-cyclohexyl-1,3-thiazolidin-2-one,
- 5-((4-(2-chloro-5-ethoxybenzyl)piperidin-1-yl)methyl)-3-cyclohexyl-1,3-thiazolidin-2-one,
- 5-((4-(2,5-dichlorobenzyl)piperidin-1-yl)methyl)-3-cyclohexyl-1,3-thiazolidin-2-one,
- 5-((4-(2-chloro-5-fluorobenzyl)piperidin-1-yl)methyl)-3-cyclohexyl-1,3-thiazolidin-2-one,
- 5-((4-(2-bromo-5-methoxybenzyl)piperidin-1-yl)methyl)-3-isopropyl-1,3-thiazolidin-2-one,
- 5-((4-(2-bromo-5-isopropoxybenzyl)piperidin-1-yl)methyl)-3-isopropyl-1,3-thiazolidin-2-one,
- 5-((4-(2-bromo-5-ethoxybenzyl)piperidin-1-yl)methyl)-3-isopropyl-1,3-thiazolidin-2-one,
- 5-((4-(2-bromo-5-chlorobenzyl)piperidin-1-yl)methyl)-3-isopropyl-1,3-thiazolidin-2-one,
- 5-((4-(2-bromo-5-fluorobenzyl)piperidin-1-yl)methyl)-3-isopropyl-1,3-thiazolidin-2-one,
- 5-(2-(4-(2-bromo-5-methoxybenzyl)piperidin-1-yl)ethyl)-3-isopropyl-1,3-thiazolidin-2-one,
- 5-(2-(4-(2-bromo-5-isopropoxybenzyl)piperidin-1-yl)ethyl)-3-isopropyl-1,3-thiazolidin-2-one,

- 5-(2-(4-(2-bromo-5-ethoxybenzyl)piperidin-1-yl)ethyl)-3-isopropyl-1,3-thiazolidin-2-one,
- 5-(2-(4-(2-bromo-5-chlorobenzyl)piperidin-1-yl)ethyl)-3-isopropyl-1,3-thiazolidin-2-one,
- 5-(2-(4-(2-bromo-5-fluorobenzyl)piperidin-1-yl)ethyl)-3-isopropyl-1,3-thiazolidin-2-one,
- 5-((4-(2-chloro-5-methoxybenzyl)piperidin-1-yl)methyl)-3-isopropyl-1,3-thiazolidin-2-one,
- 5-((4-(2-chloro-5-isopropoxybenzyl)piperidin-1-yl)methyl)-3-isopropyl-1,3-thiazolidin-2-one,
- 5-((4-(2-chloro-5-ethaxybenzyl)piperidin-1-yl)methyl)-3-isopropyl-1,3-thiazolidin-2-one,
- 5-((4-(2,5-dichlorobenzyl)piperidin-1-yl)methyl)-3-isopropyl-1,3-thiazolidin-2-one,
- 5-((4-(2-chloro-5-fluorobenzyl)piperidin-1-yl)methyl)-3-isopropyl-1,3-thiazolidin-2-one,
- 5-((4-(2-bromo-5-methoxybenzyl)piperidin-1-yl)methyl)-3-propyl-1,3-thiazolidin-2-one,
- 5-((4-(2-bromo-5-isopropoxybenzyl)piperidin-1-yl)methyl)-3-propyl-1,3-thiazolidin-2-one,
- 5-((4-(2-bromo-5-ethoxybenzyl)piperidin-1-yl)methyl)-3-propyl-1,3-thiazolidin-2-one,
- 5-((4-(2-bromo-5-chlorobenzyl)piperidin-1-yl)methyl)-3-propyl-1,3-thiazolidin-2-one,
- 5-((4-(2-bromo-5-fluorobenzyl)piperidin-1-yl)methyl)-3-propyl-1,3-thiazolidin-2-one,
- 5-(2-(4-(2-bromo-5-methoxybenzyl)piperidin-1-yl)ethyl)-3-propyl-1,3-thiazolidin-2-one,
- 5-(2-(4-(2-bromo-5-isopropoxybenzyl)piperidin-1-yl)ethyl)-3-propyl-1,3-thiazolidin-2-one,
- 5-(2-(4-(2-bromo-5-ethoxybenzyl)piperidin-1-yl)ethyl)-3-propyl-1,3-thiazolidin-2-one,
- 5-(2-(4-(2-bromo-5-chlorobenzyl)piperidin-1-yl)ethyl)-3-propyl-1,3-thiazolidin-2-one,
- 5-(2-(4-(2-bromo-5-fluorobenzyl)piperidin-1-yl)ethyl)-3-propyl-1,3-thiazolidin-2-one,
- 5-((4-(2-chloro-5-methoxybenzyl)piperidin-1-yl)methyl)-3-propyl-1,3-thiazolidin-2-one,
- 5-((4-(2-chloro-5-isopropoxybenzyl)piperidin-1-yl)methyl)-3-propyl-1,3-thiazolidin-2-one,
- 5-((4-(2-chloro-5-ethoxybenzyl)piperidin-1-yl)methyl)-3-propyl-1,3-thiazolidin-2-one,
- 5-((4-(2,5-dichlorobenzyl)piperidin-1-yl)methyl)-3-propyl-1,3-thiazolidin-2-one,
- 5-((4-(2-chloro-5-fluorobenzyl)piperidin-1-yl)methyl)-3-propyl-1,3-thiazolidin-2-one,
- 5-((4-(2-bromo-5-methoxybenzyl)piperidin-1-yl)methyl)-3-benzyl-1,3-thiazolidin-2-one,
- 5-((4-(2-bromo-5-isopropoxybenzyl)piperidin-1-yl)methyl)-3-benzyl-1,3-thiazolidin-2-one,
- 5-((4-(2-bromo-5-ethoxybenzyl)piperidin-1-yl)methyl)-3-benzyl-1,3-thiazolidin-2-one,
- 5-((4-(2-bromo-5-chlorobenzyl)piperidin-1-yl)methyl)-3-benzyl-1,3-thiazolidin-2-one,
- 5-((4-(2-bromo-5-fluorobenzyl)piperidin-1-yl)methyl)-3-benzyl-1,3-thiazolidin-2-one,
- 5-(2-(4-(2-bromo-5-methoxybenzyl)piperidin-1-yl)ethyl)-3-benzyl-1,3-thiazolidin-2-one,
- 5-(2-(4-(2-bromo-5-isopropoxybenzyl)piperidin-1-yl)ethyl)-3-benzyl-1,3-thiazolidin-2-one,
- 5-(2-(4-(2-bromo-5-ethoxybenzyl)piperidin-1-yl)ethyl)-3-benzyl-1,3-thiazolidin-2-one,
- 5-(2-(4-(2-bromo-5-chlorobenzyl)piperidin-1-yl)ethyl)-3-benzyl-1,3-thiazolidin-2-one,
- 5-(2-(4-(2-bromo-5-fluorobenzyl)piperidin-1-yl)ethyl)-3-benzyl-1,3-thiazolidin-2-one,
- 5-((4-(2-chloro-5-methoxybenzyl)piperidin-1-yl)methyl)-3-benzyl-1,3-thiazolidin-2-one,
- 5-((4-(2-chloro-5-isopropoxybenzyl)piperidin-1-yl)methyl)-3-benzyl-1,3-thiazolidin-2-one,
- 5-((4-(2-chloro-5-ethoxybenzyl)piperidin-1-yl)methyl)-3-benzyl-1,3-thiazolidin-2-one,
- 5-((4-(2,5-dichlorobenzyl)piperidin-1-yl)methyl)-3-benzyl-1,3-thiazolidin-2-one,
- 5-((4-(2-chloro-5-fluorobenzyl)piperidin-1-yl)methyl)-3-benzyl-1,3-thiazolidin-2-one,
- 5-((4-(2-bromo-5-methoxybenzyl)piperidin-1-yl)methyl)-3-cyclopropylmethyl-1,3-thiazolidin-2-one,
- 5-((4-(2-bromo-5-isopropoxybenzyl)piperidin-1-yl)methyl)-3-cyclopropylmethyl-1,3-thiazolidin-2-one,
- 5-((4-(2-bromo-5-ethoxybenzyl)piperidin-1-yl)methyl)-3-cyclopropylmethyl-1,3-thiazolidin-2-one,
- 5-((4-(2-bromo-5-chlorobenzyl)piperidin-1-yl)methyl)-3-cyclopropylmethyl-1,3-thiazolidin-2-one,
- 5-((4-(2-bromo-5-fluorobenzyl)piperidin-1-yl)methyl)-3-cyclopropylmethyl-1,3-thiazolidin-2-one,
- 5-(2-(4-(2-bromo-5-methoxybenzyl)piperidin-1-yl)ethyl)-3-cyclopropylmethyl-1,3-thiazolidin-2-one,
- 5-(2-(4-(2-bromo-5-isopropoxybenzyl)piperidin-1-yl)ethyl)-3-cyclopropylmethyl-1,3-thiazolidin-2-one,
- 5-(2-(4-(2-bromo-5-ethoxybenzyl)piperidin-1-yl)ethyl)-3-cyclopropylmethyl-1,3-thiazolidin-2-one,
- 5-(2-(4-(2-bromo-5-chlorobenzyl)piperidin-1-yl)ethyl)-3-cyclopropylmethyl-1,3-thiazolidin-2-one,
- 5-(2-(4-(2-bromo-5-fluorobenzyl)piperidin-1-yl)ethyl)-3-cyclopropylmethyl-1,3-thiazolidin-2-one,
- 5-((4-(2-chloro-5-methoxybenzyl)piperidin-1-yl)methyl)-3-cyclopropylmethyl-1,3-thiazolidin-2-one,
- 5-((4-(2-chloro-5-isopropoxybenzyl)piperidin-1-yl)methyl)-3-cyclopropylmethyl-1,3-thiazolidin-2-one,
- 5-((4-(2-chloro-5-ethoxybenzyl)piperidin-1-yl)methyl)-3-cyclopropylmethyl-1,3-thiazolidin-2-one,
- 5-((4-(2,5-dichlorobenzyl)piperidin-1-yl)methyl)-3-cyclopropylmethyl-1,3-thiazolidin-2-one,
- 5-((4-(2-chloro-5-fluorobenzyl)piperidin-1-yl)methyl)-3-cyclopropylmethyl-1,3-thiazolidin-2-one,
- 5-((4-(2-bromo-5-methoxybenzyl)piperidin-1-yl)methyl)-3-phenyl-1,3-oxazinan-2-one,
- 5-((4-(2-bromo-5-isopropoxybenzyl)piperidin-1-yl)methyl)-3-phenyl-1,3-oxazinan-2-one,
- 5-((4-(2-bromo-5-ethoxybenzyl)piperidin-1-yl)methyl)-3-phenyl-1,3-oxazinan-2-one,
- 5-((4-(2-bromo-5-chlorobenzyl)piperidin-1-yl)methyl)-3-phenyl-1,3-oxazinan-2-one,
- 5-((4-(2-bromo-5-fluorobenzyl)piperidin-1-yl)methyl)-3-phenyl-1,3-oxazinan-2-one,

- 5-((3-(2-bromo-5-methoxybenzyl)pyrrolidin-1-yl)methyl)-3-phenyl-1,3-oxazinan-2-one,
- 5-((3-(2-bromo-5-methoxyethoxybenzyl)pyrrolidin-1-yl)methyl)-3-phenyl-1,3-oxazinan-2-one,
- 5-(2-(4-(2-bromo-5-methoxybenzyl)piperidin-1-yl)ethyl)-3-phenyl-1,3-oxazinan-2-one,
- 5-(2-(4-(2-bromo-5-isopropoxybenzyl)piperidin-1-yl)ethyl)-3-phenyl-1,3-oxazinan-2-one,
- 5-(2-(4-(2-bromo-5-ethoxybenzyl)piperidin-1-yl)ethyl)-3-phenyl-1,3-oxazinan-2-one,
- 5-(2-(4-(2-bromo-5-chlorobenzyl)piperidin-1-yl)ethyl)-3-phenyl-1,3-oxazinan-2-one,
- 5-(2-(4-(2-bromo-5-fluorobenzyl)piperidin-1-yl)ethyl)-3-phenyl-1,3-oxazinan-2-one,
- 5-((4-(2-chloro-5-methoxybenzyl)piperidin-1-yl)methyl)-3-phenyl-1,3-oxazinan-2-one,
- 5-((4-(2-chloro-5-isopropoxybenzyl)piperidin-1-yl)methyl)-3-phenyl-1,3-oxazinan-2-one,
- 5-((4-(2-chloro-5-ethoxybenzyl)piperidin-1-yl)methyl)-3-phenyl-1,3-oxazinan-2-one,
- 5-((4-(2,5-dichlorobenzyl)piperidin-1-yl)methyl)-3-phenyl-1,3-oxazinan-2-one,
- 5-((4-(2-chloro-5-fluorobenzyl)piperidin-1-yl)methyl)-3-phenyl-1,3-oxazinan-2-one,
- 5-((4-(2-bromo-5-methoxybenzyl)piperidin-1-yl)methyl)-3-(4-methoxyphenyl)-1,3-oxazinan-2-one,
- 5-((4-(2-bromo-5-isopropoxybenzyl)piperidin-1-yl)methyl)-3-(4-methoxyphenyl)-1,3-oxazinan-2-one,
- 5-((4-(2-bromo-5-ethoxybenzyl)piperidin-1-yl)methyl)-3-(4-methoxyphenyl)-1,3-oxazinan-2-one,
- 5-((4-(2-bromo-5-chlorobenzyl)piperidin-1-yl)methyl)-3-(4-methoxyphenyl)-1,3-oxazinan-2-one,
- 5-((4-(2-bromo-5-fluorobenzyl)piperidin-1-yl)methyl)-3-(4-methoxyphenyl)-1,3-oxazinan-2-one,
- 5-(2-(4-(2-bromo-5-methoxybenzyl)piperidin-1-yl)ethyl)-3-(4-methoxyphenyl)-1,3-oxazinan-2-one,
- 5-(2-(4-(2-bromo-5-isopropoxybenzyl)piperidin-1-yl)ethyl)-3-(4-methoxyphenyl)-1,3-oxazinan-2-one,
- 5-(2-(4-(2-bromo-5-ethoxybenzyl)piperidin-1-yl)ethyl)-3-(4-methoxyphenyl)-1,3-oxazinan-2-one,
- 5-(2-(4-(2-bromo-5-chlorobenzyl)piperidin-1-yl)ethyl)-3-(4-methoxyphenyl)-1,3-oxazinan-2-one,
- 5-(2-(4-(2-bromo-5-fluorobenzyl)piperidin-1-yl)ethyl)-3-(4-methoxyphenyl)-1,3-oxazinan-2-one,
- 5-((4-(2-chloro-5-methoxybenzyl)piperidin-1-yl)methyl)-3-(4-methoxyphenyl)-1,3-oxazinan-2-one,
- 5-((4-(2-chloro-5-isopropoxybenzyl)piperidin-1-yl)methyl)-3-(4-methoxyphenyl)-1,3-oxazinan-2-one,
- 5-((4-(2-chloro-5-ethoxybenzyl)piperidin-1-yl)methyl)-3-(4-methoxyphenyl)-1,3-oxazinan-2-one,
- 5-((4-(2,5-dichlorobenzyl)piperidin-1-yl)methyl)-3-(4-methoxyphenyl)-1,3-oxazinan-2-one,
- 5-((4-(2-chloro-5-fluorobenzyl)piperidin-1-yl)methyl)-3-(4-methoxyphenyl)-1,3-oxazinan-2-one,
- 5-((4-(2-bromo-5-methoxybenzyl)piperidin-1-yl)methyl)-3-(4-chlorophenyl)-1,3-oxazinan-2-one,
- 5-((4-(2-bromo-5-isopropoxybenzyl)piperidin-1-yl)methyl)-3-(4-chlorophenyl)-1,3-oxazinan-2-one,
- 5-((4-(2-bromo-5-ethoxybenzyl)piperidin-1-yl)methyl)-3-(4-chlorophenyl)-1,3-oxazinan-2-one,
- 5-((4-(2-bromo-5-chlorobenzyl)piperidin-1-yl)methyl)-3-(4-chlorophenyl)-1,3-oxazinan-2-one,
- 5-((4-(2-bromo-5-fluorobenzyl)piperidin-1-yl)methyl)-3-(4-chlorophenyl)-1,3-oxazinan-2-one,
- 5-(2-(4-(2-bromo-5-methoxybenzyl)piperidin-1-yl)ethyl)-3-(4-chlorophenyl)-1,3-oxazinan-2-one,
- 5-(2-(4-(2-bromo-5-isopropoxybenzyl)piperidin-1-yl)ethyl)-3-(4-chlorophenyl)-1,3-oxazinan-2-one,
- 5-(2-(4-(2-bromo-5-ethoxybenzyl)piperidin-1-yl)ethyl)-3-(4-chlorophenyl)-1,3-oxazinan-2-one,
- 5-(2-(4-(2-bromo-5-chlorobenzyl)piperidin-1-yl)ethyl)-3-(4-chlorophenyl)-1,3-oxazinan-2-one,
- 5-(2-(4-(2-bromo-5-fluorobenzyl)piperidin-1-yl)ethyl)-3-(4-chlorophenyl)-1,3-oxazinan-2-one,
- 5-((4-(2-chloro-5-methoxybenzyl)piperidin-1-yl)methyl)-3-(4-chlorophenyl)-1,3-oxazinan-2-one,
- 5-((4-(2-chloro-5-isopropoxybenzyl)piperidin-1-yl)methyl)-3-(4-chlorophenyl)-1,3-oxazinan-2-one,
- 5-((4-(2-chloro-5-ethoxybenzyl)piperidin-1-yl)methyl)-3-(4-chlorophenyl)-1,3-oxazinan-2-one,
- 5-((4-(2,5-dichlorobenzyl)piperidin-1-yl)methyl)-3-(4-chlorophenyl)-1,3-oxazinan-2-one,
- 5-((4-(2-chloro-5-fluorobenzyl)piperidin-1-yl)methyl)-3-(4-chlorophenyl)-1,3-oxazinan-2-one,
- 5-((4-(2-bromo-5-methoxybenzyl)piperidin-1-yl)methyl)-3-cyclohexyl-1,3-oxazinan-2-one,
- 5-((4-(2-bromo-5-isopropoxybenzyl)piperidin-1-yl)methyl)-3-cyclohexyl-1,3-oxazinan-2-one,
- 5-((4-(2-bromo-5-ethoxybenzyl)piperidin-1-yl)methyl)-3-cyclohexyl-1,3-oxazinan-2-one,
- 5-((4-(2-bromo-5-chlorobenzyl)piperidin-1-yl)methyl)-3-cyclohexyl-1,3-oxazinan-2-one,
- 5-((4-(2-bromo-5-fluorobenzyl)piperidin-1-yl)methyl)-3-cyclohexyl-1,3-oxazinan-2-one,
- 5-(2-(4-(2-bromo-5-methoxybenzyl)piperidin-1-yl)ethyl)-3-cyclohexyl-1,3-oxazinan-2-one,
- 5-(2-(4-(2-bromo-5-isopropoxybenzyl)piperidin-1-yl)ethyl)-3-cyclohexyl-1,3-oxazinan-2-one,
- 5-(2-(4-(2-bromo-5-ethoxybenzyl)piperidin-1-yl)ethyl)-3-cyclohexyl-1,3-oxazinan-2-one,
- 5-(2-(4-(2-bromo-5-chlorobenzyl)piperidin-1-yl)ethyl)-3-cyclohexyl-1,3-oxazinan-2-one,
- 5-(2-(4-(2-bromo-5-fluorobenzyl)piperidin-1-yl)ethyl)-3-cyclohexyl-1,3-oxazinan-2-one,
- 5-((4-(2-chloro-5-methoxybenzyl)piperidin-1-yl)methyl)-3-cyclohexyl-1,3-oxazinan-2-one,
- 5-((4-(2-chloro-5-isopropoxybenzyl)piperidin-1-yl)methyl)-3-cyclohexyl-1,3-oxazinan-2-one,
- 5-((4-(2-chloro-5-ethoxybenzyl)piperidin-1-yl)methyl)-3-cyclohexyl-1,3-oxazinan-2-one,
- 5-((4-(2,5-dichlorobenzyl)piperidin-1-yl)methyl)-3-cyclohexyl-1,3-oxazinan-2-one,
- 5-((4-(2-chloro-5-fluorobenzyl)piperidin-1-yl)methyl)-3-cyclohexyl-1,3-oxazinan-2-one,
- 5-((4-(2-bromo-5-methoxybenzyl)piperidin-1-yl)methyl)-3-isopropyl-1,3-oxazinan-2-one,

- 5-((4-(2-bromo-5-isopropoxybenzyl)piperidin-1-yl)methyl)-3-isopropyl-1,3-oxazinan-2-one,
- 5-((4-(2-bromo-5-ethoxybenzyl)piperidin-1-yl)methyl)-3-isopropyl-1,3-oxazinan-2-one,
- 5-((4-(2-bromo-5-chlorobenzyl)piperidin-1-yl)methyl)-3-isopropyl-1,3-oxazinan-2-one,
- 5-((4-(2-bromo-5-fluorobenzyl)piperidin-1-yl)methyl)-3-isopropyl-1,3-oxazinan-2-one,
- 5-(2-(4-(2-bromo-5-methoxybenzyl)piperidin-1-yl)ethyl)-3-isopropyl-1,3-oxazinan-2-one,
- 5-(2-(4-(2-bromo-5-isopropoxybenzyl)piperidin-1-yl)ethyl)-3-isopropyl-1,3-oxazinan-2-one,
- 5-(2-(4-(2-bromo-5-ethoxybenzyl)piperidin-1-yl)ethyl)-3-isopropyl-1,3-oxazinan-2-one,
- 5-(2-(4-(2-bromo-5-chlorobenzyl)piperidin-1-yl)ethyl)-3-isopropyl-1,3-oxazinan-2-one,
- 5-(2-(4-(2-bromo-5-fluorobenzyl)piperidin-1-yl)ethyl)-3-isopropyl-1,3-oxazinan-2-one,
- 5-((4-(2-chloro-5-methoxybenzyl)piperidin-1-yl)methyl)-3-isopropyl-1,3-oxazinan-2-one,
- 5-((4-(2-chloro-5-isopropoxybenzyl)piperidin-1-yl)methyl)-3-isopropyl-1,3-oxazinan-2-one,
- 5-((4-(2-chloro-5-ethoxybenzyl)piperidin-1-yl)methyl)-3-isopropyl-1,3-oxazinan-2-one,
- 5-((4-(2,5-dichlorobenzyl)piperidin-1-yl)methyl)-3-isopropyl-1,3-oxazinan-2-one,
- 5-((4-(2-chloro-5-fluorobenzyl)piperidin-1-yl)methyl)-3-isopropyl-1,3-oxazinan-2-one,
- 5-((4-(2-bromo-5-methoxybenzyl)piperidin-1-yl)methyl)-3-propyl-1,3-oxazinan-2-one,
- 5-((4-(2-bromo-5-isopropoxybenzyl)piperidin-1-yl)methyl)-3-propyl-1,3-oxazinan-2-one,
- 5-((4-(2-bromo-5-ethoxybenzyl)piperidin-1-yl)methyl)-3-propyl-1,3-oxazinan-2-one,
- 5-((4-(2-bromo-5-chlorobenzyl)piperidin-1-yl)methyl)-3-propyl-1,3-oxazinan-2-one,
- 5-((4-(2-bromo-5-fluorobenzyl)piperidin-1-yl)methyl)-3-propyl-1,3-oxazinan-2-one,
- 5-(2-(4-(2-bromo-5-methoxybenzyl)piperidin-1-yl)ethyl)-3-propyl-1,3-oxazinan-2-one,
- 5-(2-(4-(2-bromo-5-isopropoxybenzyl)piperidin-1-yl)ethyl)-3-propyl-1,3-oxazinan-2-one,
- 5-(2-(4-(2-bromo-5-ethoxybenzyl)piperidin-1-yl)ethyl)-3-propyl-1,3-oxazinan-2-one,
- 5-(2-(4-(2-bromo-5-chlorobenzyl)piperidin-1-yl)ethyl)-3-propyl-1,3-oxazinan-2-one,
- 5-(2-(4-(2-bromo-5-fluorobenzyl)piperidin-1-yl)ethyl)-3-propyl-1,3-oxazinan-2-one,
- 5-((4-(2-chloro-5-methoxybenzyl)piperidin-1-yl)methyl)-3-propyl-1,3-oxazinan-2-one,
- 5-((4-(2-chloro-5-isopropoxybenzyl)piperidin-1-yl)methyl)-3-propyl-1,3-oxazinan-2-one,
- 5-((4-(2-chloro-5-ethoxybenzyl)piperidin-1-yl)methyl)-3-propyl-1,3-oxazinan-2-one,
- 5-((4-(2,5-dichlorobenzyl)piperidin-1-yl)methyl)-3-propyl-1,3-oxazinan-2-one,
- 5-((4-(2-chloro-5-fluorobenzyl)piperidin-1-yl)methyl)-3-propyl-1,3-oxazinan-2-one,
- 5-((4-(2-bromo-5-methoxybenzyl)piperidin-1-yl)methyl)-3-benzyl-1,3-oxazinan-2-one,
- 5-((4-(2-bromo-5-isopropoxybenzyl)piperidin-1-yl)methyl)-3-benzyl-1,3-oxazinan-2-one,
- 5-((4-(2-bromo-5-ethoxybenzyl)piperidin-1-yl)methyl)-3-benzyl-1,3-oxazinan-2-one,
- 5-((4-(2-bromo-5-chlorobenzyl)piperidin-1-yl)methyl)-3-benzyl-1,3-oxazinan-2-one,
- 5-((4-(2-bromo-5-fluorobenzyl)piperidin-1-yl)methyl)-3-benzyl-1,3-oxazinan-2-one,
- 5-(2-(4-(2-bromo-5-methoxybenzyl)piperidin-1-yl)ethyl)-3-benzyl-1,3-oxazinan-2-one,
- 5-(2-(4-(2-bromo-5-isopropoxybenzyl)piperidin-1-yl)ethyl)-3-benzyl-1,3-oxazinan-2-one,
- 5-(2-(4-(2-bromo-5-ethoxybenzyl)piperidin-1-yl)ethyl)-3-benzyl-1,3-oxazinan-2-one,
- 5-(2-(4-(2-bromo-5-chlorobenzyl)piperidin-1-yl)ethyl)-3-benzyl-1,3-oxazinan-2-one,
- 5-(2-(4-(2-bromo-5-fluorobenzyl)piperidin-1-yl)ethyl)-3-benzyl-1,3-oxazinan-2-one,
- 5-((4-(2-chloro-5-methoxybenzyl)piperidin-1-yl)methyl)-3-benzyl-1,3-oxazinan-2-one,
- 5-((4-(2-chloro-5-isopropoxybenzyl)piperidin-1-yl)methyl)-3-benzyl-1,3-oxazinan-2-one,
- 5-((4-(2-chloro-5-ethoxybenzyl)piperidin-1-yl)methyl)-3-benzyl-1,3-oxazinan-2-one,
- 5-((4-(2,5-dichlorobenzyl)piperidin-1-yl)methyl)-3-benzyl-1,3-oxazinan-2-one,
- 5-((4-(2-chloro-5-fluorobenzyl)piperidin-1-yl)methyl)-3-benzyl-1,3-oxazinan-2-one,
- 5-((4-(2-bromo-5-methoxybenzyl)piperidin-1-yl)methyl)-3-cyclopropylmethyl-1,3-oxazinan-2-one,
- 5-((4-(2-bromo-5-isopropoxybenzyl)piperidin-1-yl)methyl)-3-cyclopropylmethyl-1,3-oxazinan-2-one,
- 5-((4-(2-bromo-5-ethoxybenzyl)piperidin-1-yl)methyl)-3-cyclopropylmethyl-1,3-oxazinan-2-one,
- 5-((4-(2-bromo-5-chlorobenzyl)piperidin-1-yl)methyl)-3-cyclopropylmethyl-1,3-oxazinan-2-one,
- 5-((4-(2-bromo-5-fluoroobenzyl)piperidin-1-yl)methyl)-3-cyclopropylmethyl-1,3-oxazinan-2-one,
- 5-(2-(4-(2-bromo-5-methoxybenzyl)piperidin-1-yl)ethyl)-3-cyclopropylmethyl-1,3-oxazinan-2-one,
- 5-(2-(9-(2-bromo-5-isopropoxybenzyl)piperidin-1-yl)ethyl)-3-cyclopropylmethyl-1,3-oxazinan-2-one,
- 5-(2-(4-(2-bromo-5-ethoxybenzyl)piperidin-1-yl)ethyl)-3-cyclopropylmethyl-1,3-oxazinan-2-one,
- 5-(2-(4-(2-bromo-5-chlorobenzyl)piperidin-1-yl)ethyl)-3-cyclopropylmethyl-1,3-oxazinan-2-one,
- 5-(2-(4-(2-bromo-5-fluorobenzyl)piperidin-1-yl)ethyl)-3-cyclopropylmethyl-1,3-oxazinan-2-one,
- 5-((4-(2-chloro-5-methoxybenzyl)piperidin-1-yl)methyl)-3-cyclopropylmethyl-1,3-oxazinan-2-one,
- 5-((4-(2-chloro-5-isopropoxybenzyl)piperidin-1-yl)methyl)-3-cyclopropylmethyl-1,3-oxazinan-2-one,
- 5-((4-(2-chloro-5-ethoxybenzyl)piperidin-1-yl)methyl)-3-cyclopropylmethyl-1,3-oxazinan-2-one,
- 5-((4-(2,5-dichlorobenzyl)piperidin-1-yl)methyl)-3-cyclopropylmethyl-1,3-oxazinan-2-one,

- 5-((4-(2-chloro-5-fluorobenzyl)piperidin-1-yl)methyl)-3-cyclopropylmethyl-1,3-oxazinan-2-one,
- 4-((4-(2-bromo-5-methoxybenzyl)piperidin-1-yl)methyl)-1-phenylimidazolidin-2-one,
- 4-((4-(2-bromo-5-isopropoxybenzyl)piperidin-1-yl)methyl)-1-phenylimidazolidin-2-one,
- 4-((4-(2-bromo-5-ethoxybenzyl)piperidin-1-yl)methyl)-1-phenylimidazolidin-2-one,
- 4-((4-(2-bromo-5-chlorobenzyl)piperidin-1-yl)methyl)-1-phenylimidazolidin-2-one,
- 4-((4-(2-bromo-5-fluorobenzyl)piperidin-1-yl)methyl)-1-phenylimidazolidin-2-one,
- 4-((3-(2-bromo-5-methoxybenzyl)pyrrolidin-1-yl)methyl)-1-phenylimidazolidin-2-one,
- 4-((3-(2-bromo-5-methoxyethoxybenzyl)pyrrolidin-1-yl)methyl)-1-phenylimidazolidin-2-one,
- 4-((4-(2-bromo-5-methoxybenzyl)piperidin-1-yl)methyl)-3-methyl-1-phenylimidazolidin-2-one,
- 4-((4-(2-bromo-5-isopropoxybenzyl)piperidin-1-yl)methyl)-3-methyl-1-phenylimidazolidin-2-one,
- 4-((4-(2-bromo-5-ethoxybenzyl)piperidin-1-yl)methyl)-3-methyl-1-phenylimidazolidin-2-one,
- 4-((4-(2-bromo-5-chlorobenzyl)piperidin-1-yl)methyl)-3-methyl-1-phenylimidazolidin-2-one,
- 4-((4-(2-bromo-5-fluorobenzyl)piperidin-1-yl)methyl)-3-methyl-1-phenylimidazolidin-2-one,
- 4-(2-(4-(2-bromo-5-methoxybenzyl)piperidin-1-yl)ethyl)-1-phenylimidazolidin-2-one,
- 4-(2-(4-(2-bromo-5-isopropoxybenzyl)piperidin-1-yl)ethyl)-1-phenylimidazolidin-2-one,
- 4-(2-(4-(2-bromo-5-ethoxybenzyl)piperidin-1-yl)ethyl)-1-phenylimidazolidin-2-one,
- 4-(2-(4-(2-bromo-5-chlorobenzyl)piperidin-1-yl)ethyl)-1-phenylimidazolidin-2-one,
- 4-(2-(4-(2-bromo-5-fluorobenzyl)piperidin-1-yl)ethyl)-1-phenylimidazolidin-2-one,
- 4-((4-(2-chloro-5-methoxybenzyl)piperidin-1-yl)methyl)-1-phenylimidazolidin-2-one,
- 4-((4-(2-chloro-5-isopropoxybenzyl)piperidin-1-yl)methyl)-1-phenylimidazolidin-2-one,
- 4-((4-(2-chloro-5-ethoxybenzyl)piperidin-1-yl)methyl)-1-phenylimidazolidin-2-one,
- 4-((4-(2,5-dichlorobenzyl)piperidin-1-yl)methyl)-1-phenylimidazolidin-2-one,
- 4-((4-(2-chloro-5-fluorobenzyl)piperidin-1-yl)methyl)-1-phenylimidazolidin-2-one,
- 4-((4-(2-chloro-5-methoxybenzyl)piperidin-1-yl)methyl)-3-methyl-1-phenylimidazolidin-2-one,
- 4-((4-(2-chloro-5-isopropoxybenzyl)piperidin-1-yl)methyl)-3-methyl-1-phenylimidazolidin-2-one,
- 4-((4-(2-chloro-5-ethoxybenzyl)piperidin-1-yl)methyl)-3-methyl-1-phenylimidazolidin-2-one,
- 4-((4-(2,5-dichlorobenzyl)piperidin-1-yl)methyl)-3-methyl-1-phenylimidazolidin-2-one,
- 4-((4-(2-chloro-5-fluorobenzyl)piperidin-1-yl)methyl)-3-methyl-1-phenylimidazolidin-2-one,
- 4-((4-(2-bromo-5-methoxybenzyl)piperidin-1-yl)methyl)-1-benzylimidazolidin-2-one,
- 4-((4-(2-bromo-5-isopropoxybenzyl)piperidin-1-yl)methyl)-1-benzylimidazolidin-2-one,
- 4-((4-(2-bromo-5-ethoxybenzyl)piperidin-1-yl)methyl)-1-benzylimidazolidin-2-one,
- 4-((4-(2-bromo-5-chlorobenzyl)piperidin-1-yl)methyl)-1-benzylimidazolidin-2-one,
- 4-((4-(2-bromo-5-fluorobenzyl)piperidin-1-yl)methyl)-1-benzylimidazolidin-2-one,
- 4-((3-(2-bromo-5-methoxybenzyl)pyrrolidin-1-yl)methyl)-1-benzylimidazolidin-2-one,
- 4-((3-(2-bromo-5-methoxyethoxybenzyl)pyrrolidin-1-yl)methyl)-1-benzylimidazolidin-2-one,
- 4-((4-(2-bromo-5-methoxybenzyl)piperidin-1-yl)methyl)-3-methyl-1-benzylimidazolidin-2-one,
- 4-((4-(2-bromo-5-isopropoxybenzyl)piperidin-1-yl)methyl)-3-methyl-1-benzylimidazolidin-2-one,
- 4-((4-(2-bromo-5-ethoxybenzyl)piperidin-1-yl)methyl)-3-methyl-1-benzylimidazolidin-2-one,
- 4-((4-(2-bromo-5-chlorobenzyl)piperidin-1-yl)methyl)-3-methyl-1-benzylimidazolidin-2-one,
- 4-((4-(2-bromo-5-fluorobenzyl)piperidin-1-yl)methyl)-3-methyl-1-benzylimidazolidin-2-one,
- 4-(2-(4-(2-bromo-5-methoxybenzyl)piperidin-1-yl)ethyl)-1-benzylimidazolidin-2-one,
- 4-(2-(4-(2-bromo-5-isopropoxybenzyl)piperidin-1-yl)ethyl)-1-benzylimidazolidin-2-one,
- 4-(2-(4-(2-bromo-5-ethoxybenzyl)piperidin-1-yl)ethyl)-1-benzylimidazolidin-2-one,
- 4-(2-(4-(2-bromo-5-chlorobenzyl)piperidin-1-yl)ethyl)-1-benzylimidazolidin-2-one,
- 4-(2-(4-(2-bromo-5-fluorobenzyl)piperidin-1-yl)ethyl)-1-benzylimidazolidin-2-one,
- 4-((4-(2-chloro-5-methoxybenzyl)piperidin-1-yl)methyl)-1-benzylimidazolidin-2-one,
- 4-((4-(2-chloro-5-isopropoxybenzyl)piperidin-1-yl)methyl)-1-benzylimidazolidin-2-one,
- 4-((4-(2-chloro-5-ethoxybenzyl)piperidin-1-yl)methyl)-1-benzylimidazolidin-2-one,
- 4-((4-(2,5-dichlorobenzyl)piperidin-1-yl)methyl)-1-benzylimidazolidin-2-one,
- 4-((4-(2-chloro-5-fluorobenzyl)piperidin-1-yl)methyl)-1-benzylimidazolidin-2-one,
- 4-((4-(2-chloro-5-methoxybenzyl)piperidin-1-yl)methyl)-3-methyl-1-benzylimidazolidin-2-one,
- 4-((4-(2-chloro-5-isopropoxybenzyl)piperidin-1-yl)methyl)-3-methyl-1-benzylimidazolidin-2-one,
- 4-((4-(2-chloro-5-ethoxybenzyl)piperidin-1-yl)methyl)-3-methyl-1-benzylimidazolidin-2-one,
- 4-((4-(2,5-dichlorobenzyl)piperidin-1-yl)methyl)-3-methyl-1-benzylimidazolidin-2-one,
- 4-((4-(2-chloro-5-fluorobenzyl)piperidin-1-yl)methyl)-3-methyl-1-benzylimidazolidin-2-one,
- 4-((4-(2-bromo-5-methoxybenzyl)piperidin-1-yl)methyl)-1-cyclohexylimidazolidin-2-one,
- 4-((4-(2-bromo-5-isopropoxybenzyl)piperidin-1-yl)methyl)-1-cyclohexylimidazolidin-2-one,
- 4-((4-(2-bromo-5-ethoxybenzyl)piperidin-1-yl)methyl)-1-cyclohexylimidazolidin-2-one,

- 4-((4-(2-bromo-5-chlorobenzyl)piperidin-1-yl)methyl)-1-cyclohexylimidazolidin-2-one,
- 4-((4-(2-bromo-5-fluorobenzyl)piperidin-1-yl)methyl)-1-cyclohexylimidazolidin-2-one,
- 4-((3-(2-bromo-5-methoxybenzyl)pyrrolidin-1-yl)methyl)-1-cyclohexylimidazolidin-2-one,
- 4-((3-(2-bromo-5-methoxyethoxybenzyl)pyrrolidin-1-yl)methyl)-1-cyclohexylimidazolidin-2-one,
- 4-((4-(2-bromo-5-methoxybenzyl)piperidin-1-yl)methyl)-3-methyl-1-cyclohexylimidazolidin-2-one,
- 4-((4-(2-bromo-5-isopropoxybenzyl)piperidin-1-yl)methyl)-3-methyl-1-cyclohexylimidazolidin-2-one,
- 4-((4-(2-bromo-5-ethoxybenzyl)piperidin-1-yl)methyl)-3-methyl-1-cyclohexylimidazolidin-2-one,
- 4-((4-(2-bromo-5-chlorobenzyl)piperidin-1-yl)methyl)-3-methyl-1-cyclohexylimidazolidin-2-one,
- 4-((4-(2-bromo-5-fluorobenzyl)piperidin-1-yl)methyl)-3-methyl-1-cyclohexylimidazolidin-2-one,
- 4-(2-(4-(2-bromo-5-methoxybenzyl)piperidin-1-yl)ethyl)-1-cyclohexylimidazolidin-2-one,
- 4-(2-(4-(2-bromo-5-isopropoxybenzyl)piperidin-1-yl)ethyl)-1-cyclohexylimidazolidin-2-one,
- 4-(2-(4-(2-bromo-5-ethoxybenzyl)piperidin-1-yl)ethyl)-1-cyclohexylimidazolidin-2-one,
- 4-(2-(4-(2-bromo-5-chlorobenzyl)piperidin-1-yl)ethyl)-1-cyclohexylimidazolidin-2-one,
- 4-(2-(4-(2-bromo-5-fluorobenzyl)piperidin-1-yl)ethyl)-1-cyclohexylimidazolidin-2-one,
- 4-((4-(2-chloro-5-methoxybenzyl)piperidin-1-yl)methyl)-1-cyclohexylimidazolidin-2-one,
- 4-((4-(2-chloro-5-isopropoxybenzyl)piperidin-1-yl)methyl)-1-cyclohexylimidazolidin-2-one,
- 4-((4-(2-chloro-5-ethoxybenzyl)piperidin-1-yl)methyl)-1-cyclohexylimidazolidin-2-one,
- 4-((4-(2,5-dichlorobenzyl)piperidin-1-yl)methyl)-1-cyclohexylimidazolidin-2-one,
- 4-((4-(2-chloro-5-fluorobenzyl)piperidin-1-yl)methyl)-1-cyclohexylimidazolidin-2-one,
- 4-((4-(2-chloro-5-methoxybenzyl)piperidin-1-yl)methyl)-3-methyl-1-cyclohexylimidazolidin-2-one,
- 4-((4-(2-chloro-5-isopropoxybenzyl)piperidin-1-yl)methyl)-3-methyl-1-cyclohexylimidazolidin-2-one,
- 4-((4-(2-chloro-5-ethoxybenzyl)piperidin-1-yl)methyl)-3-methyl-1-cyclohexylimidazolidin-2-one,
- 4-((4-(2,5-dichlorobenzyl)piperidin-1-yl)methyl)-3-methyl-1-cyclohexylimidazolidin-2-one,
- 4-((4-(2-chloro-5-fluorobenzyl)piperidin-1-yl)methyl)-3-methyl-1-cyclohexylimidazolidin-2-one,
- 4-((4-(2-bromo-5-methoxybenzyl)piperidin-1-yl)methyl)-1-phenyltetrahydropyrimidin-2(1H)-one,
- 4-((4-(2-bromo-5-isopropoxybenzyl)piperidin-1-yl)methyl)-1-phenyltetrahydropyrimidin-2(1H)-one,
- 4-((4-(2-bromo-5-ethoxybenzyl)piperidin-1-yl)methyl)-1-phenyltetrahydropyrimidin-2(1H)-one,
- 4-((4-(2-bromo-5-chlorobenzyl)piperidin-1-yl)methyl)-1-phenyltetrahydropyrimidin-2(1H)-one,
- 4-((4-(2-bromo-5-fluorobenzyl)piperidin-1-yl)methyl)-1-phenyltetrahydropyrimidin-2(1H)-one,
- 4-((3-(2-bromo-5-methoxybenzyl)pyrrolidin-1-yl)methyl)-1-phenyltetrahydropyrimidin-2(1H)-one,
- 4-((3-(2-bromo-5-methoxyethoxybenzyl)pyrrolidin-1-yl)methyl)-1-phenyltetrahydropyrimidin-2(1H)-one,
- 4-((4-(2-bromo-5-methoxybenzyl)piperidin-1-yl)methyl)-3-methyl-1-phenyltetrahydropyrimidin-2(1H)-one,
- 4-((4-(2-bromo-5-isopropoxybenzyl)piperidin-1-yl)methyl)-3-methyl-1-phenyltetrahydropyrimidin-2(1H)-one,
- 4-((4-(2-bromo-5-ethoxybenzyl)piperidin-1-yl)methyl)-3-methyl-1-phenyltetrahydropyrimidin-2(1H)-one,
- 4-((4-(2-bromo-5-chlorobenzyl)piperidin-1-yl)methyl)-3-methyl-1-phenyltetrahydropyrimidin-2(1H)-one,
- 4-((4-(2-bromo-5-fluorobenzyl)piperidin-1-yl)methyl)-3-methyl-1-phenyltetrahydropyrimidin-2(1H)-one,
- 4-(2-(4-(2-bromo-5-methoxybenzyl)piperidin-1-yl)ethyl)-1-phenyltetrahydropyrimidin-2(1H)-one,
- 4-(2-(4-(2-bromo-5-isopropoxybenzyl)piperidin-1-yl)ethyl)-1-phenyltetrahydropyrimidin-2(1H)-one,
- 4-(2-(4-(2-bromo-5-ethoxybenzyl)piperidin-1-yl)ethyl)-1-phenyltetrahydropyrimidin-2(1H)-one,
- 4-(2-(4-(2-bromo-5-chlorobenzyl)piperidin-1-yl)ethyl)-1-phenyltetrahydropyrimidin-2(1H)-one,
- 4-(2-(4-(2-bromo-5-fluorobenzyl)piperidin-1-yl)ethyl)-1-phenyltetrahydropyrimidin-2(1H)-one,
- 4-((4-(2-chloro-5-methoxybenzyl)piperidin-1-yl)methyl)-1-phenyltetrahydropyrimidin-2(1H)-one,
- 4-((4-(2-chloro-5-isopropoxybenzyl)piperidin-1-yl)methyl)-1-phenyltetrahydropyrimidin-2(1H)-one,
- 4-((4-(2-chloro-5-ethoxybenzyl)piperidin-1-yl)methyl)-1-phenyltetrahydropyrimidin-2(1H)-one,
- 4-((4-(2,5-dichlorobenzyl)piperidin-1-yl)methyl)-1-phenyltetrahydropyrimidin-2(1H)-one,
- 4-((4-(2-chloro-5-fluorobenzyl)piperidin-1-yl)methyl)-1-phenyltetrahydropyrimidin-2(1H)-one,
- 4-((4-(2-chloro-5-methoxybenzyl)piperidin-1-yl)methyl)-3-methyl-1-phenyltetrahydropyrimidin-2(1H)-one,
- 4-((4-(2-chloro-5-isopropoxybenzyl)piperidin-1-yl)methyl)-3-methyl-1-phenyltetrahydropyrimidin-2(1H)-one,
- 4-((4-(2-chloro-5-ethoxybenzyl)piperidin-1-yl)methyl)-3-methyl-1-phenyltetrahydropyrimidin-2(1H)-one,
- 4-((4-(2,5-dichlorobenzyl)piperidin-1-yl)methyl)-3-methyl-1-phenyltetrahydropyrimidin-2(1H)-one,
- 4-((4-(2-chloro-5-fluorobenzyl)piperidin-1-yl)methyl)-3-methyl-1-phenyltetrahydropyrimidin-2(1H)-one,
- 4-((4-(2-bromo-5-methoxybenzyl)piperidin-1-yl)methyl)-1-benzyltetrahydropyrimidin-2(1H)-one,
- 4-((4-(2-bromo-5-isopropoxybenzyl)piperidin-1-yl)methyl)-1-benzyltetrahydropyrimidin-2(1H)-one,
- 4-((4-(2-bromo-5-ethoxybenzyl)piperidin-1-yl)methyl)-1-benzyltetrahydropyrimidin-2(1H)-one,
- 4-((4-(2-bromo-5-chlorobenzyl)piperidin-1-yl)methyl)-1-benzyltetrahydropyrimidin-2(1H)-one,
- 4-((4-(2-bromo-5-fluorobenzyl)piperidin-1-yl)methyl)-1-benzyltetrahydropyrimidin-2(1H)-one,
- 4-((3-(2-bromo-5-methoxybenzyl)pyrrolidin-1-yl)methyl)-1-benzyltetrahydropyrimidin-2(1H)-one,
- 4-((3-(2-bromo-5-methoxyethoxybenzyl)pyrrolidin-1-yl)methyl)-1-benzyltetrahydropyrimidin-2(1H)-one,

- 4-((4-(2-bromo-5-methoxybenzyl)piperidin-1-yl)methyl)-3-methyl-1-benzyltetrahydropyrimidin-2(1H)-one,
- 4-((4-(2-bromo-5-isopropoxybenzyl)piperidin-1-yl)methyl)-3-methyl-1-benzyltetrahydropyrimidin-2(1H)-one,
- 4-((4-(2-bromo-5-ethoxybenzyl)piperidin-1-yl)methyl)-3-methyl-1-benzyltetrahydropyrimidin-2(1H)-one,
- 4-((4-(2-bromo-5-chlorobenzyl)piperidin-1-yl)methyl)-3-methyl-1-benzyltetrahydropyrimidin-2(1H)-one,
- 4-((4-(2-bromo-5-fluorobenzyl)piperidin-1-yl)methyl)-3-methyl-1-benzyltetrahydropyrimidin-2(1H)-one,
- 4-(2-(4-(2-bromo-5-methoxybenzyl)piperidin-1-yl)ethyl)-1-benzyltetrahydropyrimidin-2(1H)-one,
- 4-(2-(4-(2-bromo-5-isopropoxybenzyl)piperidin-1-yl)ethyl)-1-benzyltetrahydropyrimidin-2(1H)-one,
- 4-(2-(4-(2-bromo-5-ethoxybenzyl)piperidin-1-yl)ethyl)-1-benzyltetrahydropyrimidin-2(1H)-one,
- 4-(2-(4-(2-bromo-5-chlorobenzyl)piperidin-1-yl)ethyl)-1-benzyltetrahydropyrimidin-2(1H)-one,
- 4-(2-(4-(2-bromo-5-fluorobenzyl)piperidin-1-yl)ethyl)-1-benzyltetrahydropyrimidin-2(1H)-one,
- 4-((4-(2-chloro-5-methoxybenzyl)piperidin-1-yl)methyl)-1-benzyltetrahydropyrimidin-2(1H)-one,
- 4-((4-(2-chloro-5-isopropoxybenzyl)piperidin-1-yl)methyl)-1-benzyltetrahydropyrimidin-2(1H)-one,
- 4-((4-(2-chloro-5-ethoxybenzyl)piperidin-1-yl)methyl)-1-benzyltetrahydropyrimidin-2(1H)-one,
- 4-((4-(2,5-dichlorobenzyl)piperidin-1-yl)methyl)-1-benzyltetrahydropyrimidin-2(1H)-one,
- 4-((4-(2-chloro-5-fluorobenzyl)piperidin-1-yl)methyl)-1-benzyltetrahydropyrimidin-2(1H)-one,
- 4-((4-(2-chloro-5-methoxybenzyl)piperidin-1-yl)methyl)-3-methyl-1-benzyltetrahydropyrimidin-2(1H)-one,
- 4-((4-(2-chloro-5-isopropoxybenzyl)piperidin-1-yl)methyl)-3-methyl-1-benzyltetrahydropyrimidin-2(1H)-one,
- 4-((4-(2-chloro-5-ethoxybenzyl)piperidin-1-yl)methyl)-3-methyl-1-benzyltetrahydropyrimidin-2(1H)-one,
- 4-((4-(2,5-dichlorobenzyl)piperidin-1-yl)methyl)-3-methyl-1-benzyltetrahydropyrimidin-2(1H)-one,
- 4-((4-(2-chloro-5-fluorobenzyl)piperidin-1-yl)methyl)-3-methyl-1-benzyltetrahydropyrimidin-2(1H)-one,
- 4-((4-(2-bromo-5-methoxybenzyl)piperidin-1-yl)methyl)-1-cyclohexyltetrahydropyrimidin-2(1H)-one,
- 4-((4-(2-bromo-5-isopropoxybenzyl)piperidin-1-yl)methyl)-1-cyclohexyltetrahydropyrimidin-2(1H)-one,
- 4-((4-(2-bromo-5-ethoxybenzyl)piperidin-1-yl)methyl)-1-cyclohexyltetrahydropyrimidin-2(1H)-one,
- 4-((4-(2-bromo-5-chlorobenzyl)piperidin-1-yl)methyl)-1-cyclohexyltetrahydropyrimidin-2(1H)-one,
- 4-((4-(2-bromo-5-fluorobenzyl)piperidin-1-yl)methyl)-1-cyclohexyltetrahydropyrimidin-2(1H)-one,
- 4-((3-(2-bromo-5-methoxybenzyl)pyrrolidin-1-yl)methyl)-1-cyclohexyltetrahydropyrimidin-2(1H)-one,
- 4-((3-(2-bromo-5-methoxyethoxybenzyl)pyrrolidin-1-yl)methyl)-1-cyclohexyltetrahydropyrimidin-2(1H)-one,
- 4-((4-(2-bromo-5-methoxybenzyl)piperidin-1-yl)methyl)-3-methyl-1-cyclohexyltetrahydropyrimidin-2(1H)-one,
- 4-((4-(2-bromo-5-isopropoxybenzyl)piperidin-1-yl)methyl)-3-methyl-1-cyclohexyltetrahydropyrimidin-2(1H)-one,
- 4-((4-(2-bromo-5-ethoxybenzyl)piperidin-1-yl)methyl)-3-methyl-1-cyclohexyltetrahydropyrimidin-2(1H)-one,
- 4-((4-(2-bromo-5-chlorobenzyl)piperidin-1-yl)methyl)-3-methyl-1-cyclohexyltetrahydropyrimidin-2(1H)-one,
- 4-((4-(2-bromo-5-fluorobenzyl)piperidin-1-yl)methyl)-3-methyl-1-cyclohexyltetrahydropyrimidin-2(1H)-one,
- 4-(2-(4-(2-bromo-5-methoxybenzyl)piperidin-1-yl)ethyl)-1-cyclohexyltetrahydropyrimidin-2(1H)-one,
- 4-(2-(4-(2-bromo-5-isopropoxybenzyl)piperidin-1-yl)ethyl)-1-cyclohexyltetrahydropyrimidin-2(1H)-one,
- 4-(2-(4-(2-bromo-5-ethoxybenzyl)piperidin-1-yl)ethyl)-1-cyclohexyltetrahydropyrimidin-2(1H)-one,
- 4-(2-(4-(2-bromo-5-chlorobenzyl)piperidin-1-yl)ethyl)-1-cyclohexyltetrahydropyrimidin-2(1H)-one,
- 4-(2-(4-(2-bromo-5-fluorobenzyl)piperidin-1-yl)ethyl)-1-cyclohexyltetrahydropyrimidin-2(1H)-one,
- 4-((4-(2-chloro-5-methoxybenzyl)piperidin-1-yl)methyl)-1-cyclohexyltetrahydropyrimidin-2(1H)-one,
- 4-((4-(2-chloro-5-isopropoxybenzyl)piperidin-1-yl)methyl)-1-cyclohexyltetrahydropyrimidin-2(1H)-one,
- 4-((4-(2-chloro-5-ethoxybenzyl)piperidin-1-yl)methyl)-1-cyclohexyltetrahydropyrimidin-2(1H)-one,
- 4-((4-(2,5-dichlorobenzyl)piperidin-1-yl)methyl)-1-cyclohexyltetrahydropyrimidin-2(1H)-one,
- 4-((4-(2-chloro-5-fluorobenzyl)piperidin-1-yl)methyl)-1-cyclohexyltetrahydropyrimidin-2(1H)-one,
- 4-((4-(2-chloro-5-methoxybenzyl)piperidin-1-yl)methyl)-3-methyl-1-cyclohexyltetrahydropyrimidin-2(1H)-one,
- 4-((4-(2-chloro-5-isopropoxybenzyl)piperidin-1-yl)methyl)-3-methyl-1-cyclohexyltetrahydropyrimidin-2(1H)-one,
- 4-((4-(2-chloro-5-ethoxybenzyl)piperidin-1-yl)methyl)-3-methyl-1-cyclohexyltetrahydropyrimidin-2(1H)-one,
- 4-((4-(2,5-dichlorobenzyl)piperidin-1-yl)methyl)-3-methyl-1-cyclohexyltetrahydropyrimidin-2(1H)-one,
- 4-((4-(2-chloro-5-fluorobenzyl)piperidin-1-yl)methyl)-3-methyl-1-cyclohexyltetrahydropyrimidin-2(1H)-one,
- 6-((4-(2-bromo-5-methoxybenzyl)piperidin-1-yl)methyl)-3-phenyldihydropyrimidine-2,4(1H,3H)-dione,
- 6-((4-(2-bromo-5-isopropoxybenzyl)piperidin-1-yl)methyl)-3-phenyldihydropyrimidine-2,4(1H,3H)-dione,
- 6-((4-(2-bromo-5-ethoxybenzyl)piperidin-1-yl)methyl)-3-phenyldihydropyrimidine-2,4(1H,3H)-dione,
- 6-((4-(2-bromo-5-chlorobenzyl)piperidin-1-yl)methyl)-3-phenyldihydropyrimidine-2,4(1H,3H)-dione,
- 6-((4-(2-bromo-5-fluorobenzyl)piperidin-1-yl)methyl)-3-phenyldihydropyrimidine-2,4(1H,3H)-dione,
- 6-((3-(2-bromo-5-methoxybenzyl)pyrrolidin-1-yl)methyl)-3-phenyldihydropyrimidine-2,4(1H,3H)-dione,
- 6-((3-(2-bromo-5-methoxyethoxybenzyl)pyrrolidin-1-yl)methyl)-3-phenyldihydropyrimidine-2,4(1H,3H)-dione,
- 6-((4-(2-bromo-5-methoxybenzyl)piperidin-1-yl)methyl)-1-methyl-3-phenyldihydropyrimidine-2,4(1H,3H)-dione,
- 6-((4-(2-bromo-5-isopropoxybenzyl)piperidin-1-yl)methyl)-1-methyl-3-phenyldihydropyrimidine-2,4(1H,3H)-dione,
- 6-((4-(2-bromo-5-ethoxybenzyl)piperidin-1-yl)methyl)-1-methyl-3-phenyldihydropyrimidine-2,4(1H,3H)-dione,

- 6-((4-(2-bromo-5-chlorobenzyl)piperidin-1-yl)methyl)-1-methyl-3-phenyldihydropyrimidine-2,4(1H,3H)-dione,
- 6-((4-(2-bromo-5-fluorobenzyl)piperidin-1-yl)methyl)-1-methyl-3-phenyldihydropyrimidine-2,4(1H,3H)-dione,
- 6-(2-(4-(2-bromo-5-methoxybenzyl)piperidin-1-yl)ethyl)-3-phenyldihydropyrimidine-2,4(1H,3H)-dione,
- 6-(2-(4-(2-bromo-5-isopropoxybenzyl)piperidin-1-yl)ethyl)-3-phenyldihydropyrimidine-2,4(1H,3H)-dione,
- 6-(2-(4-(2-bromo-5-ethoxybenzyl)piperidin-1-yl)ethyl)-3-phenyldihydropyrimidine-2,4(1H,3H)-dione,
- 6-(2-(4-(2-bromo-5-chlorobenzyl)piperidin-1-yl)ethyl)-3-phenyldihydropyrimidine-2,4(1H,3H)-dione,
- 6-(2-(4-(2-bromo-5-fluorobenzyl)piperidin-1-yl)ethyl)-3-phenyldihydropyrimidine-2,4(1H,3H)-dione,
- 6-((4-(2-chloro-5-methoxybenzyl)piperidin-1-yl)methyl)-3-phenyldihydropyrimidine-2,4(1H,3H)-dione,
- 6-((4-(2-chloro-5-isopropoxybenzyl)piperidin-1-yl)methyl)-3-phenyldihydropyrimidine-2,4(1H,3H)-dione,
- 6-((4-(2-chloro-5-ethoxybenzyl)piperidin-1-yl)methyl)-3-phenyldihydropyrimidine-2,4(1H,3H)-dione,
- 6-((4-(2,5-dichlorobenzyl)piperidin-1-yl)methyl)-3-phenyldihydropyrimidine-2,4(1H,3H)-dione,
- 6-((4-(2-chloro-5-fluorobenzyl)piperidin-1-yl)methyl)-3-phenyldihydropyrimidine-2,4(1H,3H)-dione,
- 6-((4-(2-chloro-5-methoxybenzyl)piperidin-1-yl)methyl)-1-methyl-3-phenyldihydropyrimidine-2,4(1H,3H)-dione,
- 6-((4-(2-chloro-5-isopropoxybenzyl)piperidin-1-yl)methyl)-1-methyl-3-phenyldihydropyrimidine-2,4(1H,3H)-dione,
- 6-((4-(2-chloro-5-ethoxybenzyl)piperidin-1-yl)methyl)-1-methyl-3-phenyldihydropyrimidine-2,4(1H,3H)-dione,
- 6-((4-(2,5-dichlorobenzyl)piperidin-1-yl)methyl)-1-methyl-3-phenyldihydropyrimidine-2,4(1H,3H)-dione,
- 6-((4-(2-chloro-5-fluorobenzyl)piperidin-1-yl)methyl)-1-methyl-3-phenyldihydropyrimidine-2,4(1H,3H)-dione,
- 6-((4-(2-bromo-5-methoxybenzyl)piperidin-1-yl)methyl)-3-benzyldihydropyrimidine-2,4(1H,3H)-dione,
- 6-((4-(2-bromo-5-isopropoxybenzyl)piperidin-1-yl)methyl)-3-benzyldihydropyrimidine-2,4(1H,3H)-dione,
- 6-((4-(2-bromo-5-ethoxybenzyl)piperidin-1-yl)methyl)-3-benzyldihydropyrimidine-2,4(1H,3H)-dione,
- 6-((4-(2-bromo-5-chlorobenzyl)piperidin-1-yl)methyl)-3-benzyldihydropyrimidine-2,4(1H,3H)-dione,
- 6-((4-(2-bromo-5-fluorobenzyl)piperidin-1-yl)methyl)-3-benzyldihydropyrimidine-2,4(1H,3H)-dione,
- 6-((3-(2-bromo-5-methoxybenzyl)pyrrolidin-1-yl)methyl)-3-benzyldihydropyrimidine-2,4(1H,3H)-dione,
- 6-((3-(2-bromo-5-methoxyethoxybenzyl)pyrrolidin-1-yl)methyl)-3-benzyldihydropyrimidine-2,4(1H,3H)-dione,
- 6-((4-(2-bromo-5-methoxybenzyl)piperidin-1-yl)methyl)-1-methyl-3-benzyldihydropyrimidine-2,4(1H,3H)-dione,
- 6-((4-(2-bromo-5-isopropoxybenzyl)piperidin-1-yl)methyl)-1-methyl-3-benzyldihydropyrimidine-2,4(1H,3H)-dione,
- 6-((4-(2-bromo-5-ethoxybenzyl)piperidin-1-yl)methyl)-1-methyl-3-benzyldihydropyrimidine-2,4(1H,3H)-dione,
- 6-((4-(2-bromo-5-chlorobenzyl)piperidin-1-yl)methyl)-1-methyl-3-benzyldihydropyrimidine-2,4(1H,3H)-dione,
- 6-((4-(2-bromo-5-fluorobenzyl)piperidin-1-yl)methyl)-1-methyl-3-benzyldihydropyrimidine-2,4(1H,3H)-dione,
- 6-(2-(4-(2-bromo-5-methoxybenzyl)piperidin-1-yl)ethyl)-3-benzyldihydropyrimidine-2,4(1H,3H)-dione,
- 6-(2-(4-(2-bromo-5-isopropoxybenzyl)piperidin-1-yl)ethyl)-3-benzyldihydropyrimidine-2,4(1H,3H)-dione,
- 6-(2-(4-(2-bromo-5-ethoxybenzyl)piperidin-1-yl)ethyl)-3-benzyldihydropyrimidine-2,4(1H,3H)-dione,
- 6-(2-(4-(2-bromo-5-chlorobenzyl)piperidin-1-yl)ethyl)-3-benzyldihydropyrimidine-2,4(1H,3H)-dione,
- 6-(2-(4-(2-bromo-5-fluorobenzyl)piperidin-1-yl)ethyl)-3-benzyldihydropyrimidine-2,4(1H,3H)-dione,
- 6-((4-(2-chloro-5-methoxybenzyl)piperidin-1-yl)methyl)-3-benzyldihydropyrimidine-2,4(1H,3H)-dione,
- 6-((4-(2-chloro-5-isopropoxybenzyl)piperidin-1-yl)methyl)-3-benzyldihydropyrimidine-2,4(1H,3H)-dione,
- 6-((4-(2-chloro-5-ethoxybenzyl)piperidin-1-yl)methyl)-3-benzyldihydropyrimidine-2,4(1H,3H)-dione,
- 6-((4-(2,5-dichlorobenzyl)piperidin-1-yl)methyl)-3-benzyldihydropyrimidine-2,4(1H,3H)-dione,
- 6-((4-(2-chloro-5-fluorobenzyl)piperidin-1-yl)methyl)-3-benzyldihydropyrimidine-2,4(1H,3H)-dione,
- 6-((4-(2-chloro-5-methoxybenzyl)piperidin-1-yl)methyl)-1-methyl-3-benzyldihydropyrimidine-2,4(1H,3H)-dione,
- 6-((4-(2-chloro-5-isopropoxybenzyl)piperidin-1-yl)methyl)-1-methyl-3-benzyldihydropyrimidine-2,4(1H,3H)-dione,
- 6-((4-(2-chloro-5-ethoxybenzyl)piperidin-1-yl)methyl)-1-methyl-3-benzyldihydropyrimidine-2,4(1H,3H)-dione,
- 6-((4-(2,5-dichlorobenzyl)piperidin-1-yl)methyl)-1-methyl-3-benzyldihydropyrimidine-2,4(1H,3H)-dione,
- 6-((4-(2-chloro-5-fluorobenzyl)piperidin-1-yl)methyl)-1-methyl-3-benzyldihydropyrimidine-2,4(1H,3H)-dione,
- 6-((4-(2-bromo-5-methoxybenzyl)piperidin-1-yl)methyl)-3-cyclohexyldihydropyrimidine-2,4(1H,3H)-dione,
- 6-((4-(2-bromo-5-isopropoxybenzyl)piperidin-1-yl)methyl)-3-cyclohexyldihydropyrimidine-2,4(1H,3H)-dione,
- 6-((4-(2-bromo-5-ethoxybenzyl)piperidin-1-yl)methyl)-3-cyclohexyldihydropyrimidine-2,4(1H,3H)-dione,
- 6-((4-(2-bromo-5-chlorobenzyl)piperidin-1-yl)methyl)-3-cyclohexyldihydropyrimidine-2,4(1H,3H)-dione,
- 6-((4-(2-bromo-5-fluorobenzyl)piperidin-1-yl)methyl)-3-cyclohexyldihydropyrimidine-2,4(1H,3H)-dione,
- 6-((3-(2-bromo-5-methoxybenzyl)pyrrolidin-1-yl)methyl)-3-cyclohexyldihydropyrimidine-2,4(1H,3H)-dione,
- 6-((3-(2-bromo-5-methoxyethoxybenzyl)pyrrolidin-1-yl)methyl)-3-cyclohexyldihydropyrimidine-2,4(1H,3H)-dione,
- 6-((4-(2-bromo-5-methoxybenzyl)piperidin-1-yl)methyl)-1-methyl-3-cyclohexyldihydropyrimidine-2,4(1H,3H)-dione,
- 6-((4-(2-bromo-5-isopropoxybenzyl)piperidin-1-yl)methyl)-1-methyl-3-cyclohexyldihydropyrimidine-2,4(1H,3H)-dione,
- 6-((4-(2-bromo-5-ethoxybenzyl)piperidin-1-yl)methyl)-1-methyl-3-cyclohexyldihydropyrimidine-2,4(1H,3H)-dione,
- 6-((4-(2-bromo-5-chlorobenzyl)piperidin-1-yl)methyl)-1-methyl-3-cyclohexyldihydropyrimidine-2,4(1H,3H)-dione,

- 6-((4-(2-bromo-5-fluorobenzyl)piperidin-1-yl)methyl)-1-methyl-3-cyclohexyldihydropyrimidine-2,4(1H,3H)-dione,
- 6-(2-(4-(2-bromo-5-methoxybenzyl)piperidin-1-yl)ethyl)-3-cyclohexyldihydropyrimidine-2,4(1H,3H)-dione,
- 6-(2-(4-(2-bromo-5-isopropoxybenzyl)piperidin-1-yl)ethyl)-3-cyclohexyldihydropyrimidine-2,4(1H,3H)-dione,
- 6-(2-(4-(2-bromo-5-ethoxybenzyl)piperidin-1-yl)ethyl)-3-cyclohexyldihydropyrimidine-2,4(1H,3H)-dione,
- 6-(2-(4-(2-bromo-5-chlorobenzyl)piperidin-1-yl)ethyl)-3-cyclohexyldihydropyrimidine-2,4(1H,3H)-dione,
- 6-(2-(4-(2-bromo-5-fluorobenzyl)piperidin-1-yl)ethyl)-3-cyclohexyldihydropyrimidine-2,4(1H,3H)-dione,
- 6-((4-(2-chloro-5-methoxybenzyl)piperidin-1-yl)methyl)-3-cyclohexyldihydropyrimidine-2,4(1H,3H)-dione,
- 6-((4-(2-chloro-5-isopropoxybenzyl)piperidin-1-yl)methyl)-3-cyclohexyldihydropyrimidine-2,4(1H,3H)-dione,
- 6-((4-(2-chloro-5-ethoxybenzyl)piperidin-1-yl)methyl)-3-cyclohexyldihydropyrimidine-2,4(1H,3H)-dione,
- 6-((4-(2,5-dichlorobenzyl)piperidin-1-yl)methyl)-3-cyclohexyldihydropyrimidine-2,4(1H,3H)-dione,
- 6-((4-(2-chloro-5-fluorobenzyl)piperidin-1-yl)methyl)-3-cyclohexyldihydropyrimidine-2,4(1H,3H)-dione,
- 6-((4-(2-chloro-5-methoxybenzyl)piperidin-1-yl)methyl)-1-methyl-3-cyclohexyldihydropyrimidine-2,4(1H,3H)-dione,
- 6-((4-(2-chloro-5-isopropoxybenzyl)piperidin-1-yl)methyl)-1-methyl-3-cyclohexyldihydropyrimidine-2,4(1H,3H)-dione,
- 6-((4-(2-chloro-5-ethoxybenzyl)piperidin-1-yl)methyl)-1-methyl-3-cyclohexyldihydropyrimidine-2,4(1H,3H)-dione,
- 6-((4-(2,5-dichlorobenzyl)piperidin-1-yl)methyl)-1-methyl-3-cyclohexyldihydropyrimidine-2,4(1H,3H)-dione and
- 6-((4-(2-chloro-5-fluorobenzyl)piperidin-1-yl)methyl)-1-methyl-3-cyclohexyldihydropyrimidine-2,4(1H,3H)-dione.

INDUSTRIAL APPLICABILITY

The present invention makes it possible to provide a selective serotonin reuptake inhibitor having affinity for serotonin 1A receptors which is useful as a therapeutic agent for human or animal diseases such as mood disorders including depression, seasonal affective disorder and dysthymia; anxiety disorders including generalized anxiety disorder, obsessive-compulsive disorder and panic disorder; agoraphobia and avoidant personality disorder; social phobia; compulsive reaction; post-traumatic stress disorder; psychosomatic disorder; memory impairments including dementia, forgetfulness and memory impairment associated with aging; eating disorders including anorexia nervosa and bulimia nervosa; obesity; somnipathy; schizophrenia; chemical dependency due to alcohol, tobacco, nicotine or the like; cluster headache; migraine; pains; Alzheimer's disease; chronic paroxysmal migraine; headache associated with vasculopathy; Parkinson's disease including dementia, depression and anxiety caused by Parkinson's disease, Parkinsonism induced by a neuroleptic agent, and tardive dyskinesia; dysendocrinism such as hyperprolactinemia; vasospasm (in particular, cerebrovascular spasm); hypertension; kinetic gastrointestinal troubles and gastrointestinal troubles in which a secretion change participates; sexual dysfunction including premature ejaculation; drug dependence; and the like. Furthermore, the present invention makes it possible to provide novel non-aromatic heterocyclic derivatives having a 4-benzylpiperidinoalkyl group as a substituent on their carbon atom which include compounds having such effects and are useful as intermediates of medicines and agrochemicals, and a production process of the derivatives.

## Claims

**1.** A serotonin reuptake inhibitor comprising a cyclic amine represented by the formula:

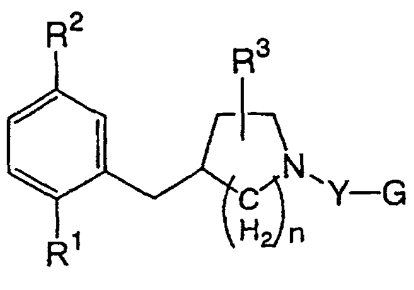

(1)

wherein G represents a formula (2):

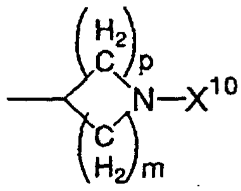

(a) 、 $-Z^2-X^{20}$ (b) 、 or $-Z^3$ (c) (2)

wherein

$R^1$ is a halogen atom or an alkyl group;
$R^2$ is a hydrogen atom, a halogen atom, a hydroxyl group, an alkyl group, a substituted alkyl group, an alkoxy group, a substituted alkoxy group, an alkylthio group or a substituted alkylthio group;
$R^3$ is a hydrogen atom or a lower alkyl group;
Y is a substituted or unsubstituted alkylene group;
n is an integer of 1, 2 or 3;
m is an integer of 0, 1, 2 or 3;
p is an integer of 1, 2, 3 or 4;
$X^{10}$ is a hydrogen atom, an alkyl group, a substituted alkyl group, a cycloalkyl group, an alkanoyl group, a substituted alkanoyl group, an alkanesulfonyl group, a substituted alkanesulfonyl group, a carbamoyl group, an alkylcarbamoyl group, a substituted alkylcarbamoyl group, an alkylsulfamoyl group, a substituted alkylsulfamoyl group, an alkoxycarbonyl group, a substituted alkoxycarbonyl group, an amidino group or a substituted amidino group;
$X^{20}$ is an alkyl group, a substituted alkyl group, an amino group, a substituted amino group, a hydroxyl group, an alkoxy group, a substituted alkoxy group, a carbamoyloxy group, an alkylcarbamoyloxy group or a substituted alkylcarbamoyloxy group;
$Z^2$ is a cycloalkane ring;
$Z^3$ represents a formula:

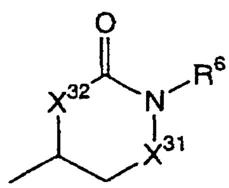

(A)

wherein

$X^{31}$ is a bond, a methylene group or a carbonyl group;
$X^{32}$ is an oxygen atom, a sulfur atom or a group: $-N(R^7)-$;
$R^6$ is a hydrogen atom, an alkyl group, a substituted alkyl group, a cycloalkyl group, a substituted cycloalkyl group, an aryl group, a substituted aryl group, a heteroaryl group or a substituted heteroaryl group;
$R^7$ is a hydrogen atom or an alkyl group, or a formula:

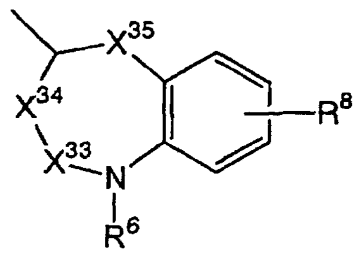

(B)

wherein

$X^{33}$ is a bond, a methylene group or a carbonyl group;
$X^{34}$ is a bond or a methylene group, and $X^{35}$ is a bond, a methylene group, an oxygen atom, a sulfur atom or a group: -N($R^7$)-, provided that $X^{34}$ and $X^{35}$ are not bonds at the same time;
$R^6$ and $R^7$ are as defined above; and
$R^8$ is a hydrogen atom, a halogen atom, an alkyl group, a hydroxyl group, an alkoxy group, a substituted alkoxy group, a carbamoyloxy group, an alkylcarbamoyloxy group or a substituted alkylcarbamoyloxy group,

a prodrug of said cyclic amine, or a pharmaceutically acceptable salt of said cyclic amine or prodrug, as an active ingredient.

**2.** A serotonin reuptake inhibitor according to claim 1, wherein G represents a formula:

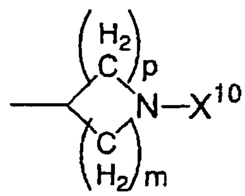

**3.** A serotonin reuptake inhibitor according to claim 2, wherein the alkylene group for Y is an ethylene group or a trimethylene group.

**4.** A serotonin reuptake inhibitor according to claim 2 or 3, wherein n is 1 or 2.

**5.** A serotonin reuptake inhibitor according to claim 2, 3 or 4, wherein each of p and m is 2.

**6.** A serotonin reuptake inhibitor according to claim 2, 3 or 4, wherein one of p and m is 1 and the other is 3.

**7.** A serotonin reuptake inhibitor according to any one of claims 2 to 6, wherein $X^{10}$ is a hydrogen atom, a lower alkyl group, a hydroxyalkyl group of 4 or less carbon atoms, an alkanoyl group, a hydroxy-substituted alkanoyl group, an alkanesulfonyl group or an alkylsulfamoyl group.

**8.** A cyclic amine represented by the formula:

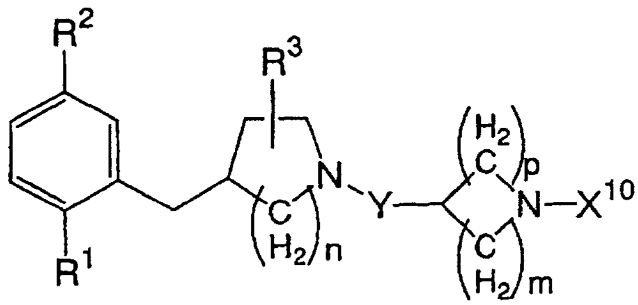

wherein

$R^1$ is a halogen atom or an alkyl group;
$R^2$ is a hydrogen atom, a halogen atom, a hydroxyl group, an alkyl group, a substituted alkyl group, an alkoxy group, a substituted alkoxy group, an alkylthio group or a substituted alkylthio group;
$R^3$ is a hydrogen atom or a lower alkyl group;
Y is a substituted or unsubstituted alkylene group;
$X^{10}$ is a hydrogen atom, an alkyl group, a substituted alkyl group, a cycloalkyl group, an alkanoyl group, a substituted alkanoyl group, an alkanesulfonyl group, a substituted alkanesulfonyl group, a carbamoyl group, an alkylcarbamoyl group, a substituted alkylcarbamoyl group, an alkylsulfamoyl group, a substituted alkylsulfamoyl group, an alkoxycarbonyl group, a substituted alkoxycarbonyl group, an amidino group or a substituted amidino group;
n is an integer of 1, 2 or 3;
m is an integer of 0, 1, 2 or 3; and
p is an integer of 1, 2, 3 or 4,

a prodrug of said cyclic amine, or a pharmaceutically acceptable salt or solvate of said cyclic amine or prodrug.

**9.** A compound according to claim 8, wherein $R^2$ is a hydroxyl group, a lower alkoxy group, a halogen-substituted alkoxy group or a halogen atom, or $R^2$ is a hydrogen atom only in the case of $R^1$ being a bromine atom; and Y is a substituted or unsubstituted alkylene group of 2 or 3 carbon atoms.

**10.** A compound according to claim 8 or 9, wherein n is 1 or 2.

**11.** A compound according to any one of claims 8 to 10, wherein Y is an unsubstituted alkylene group.

**12.** A compound according to any one of claims 8 to 11, wherein each of p and m is 2.

**13.** A compound according to any one of claims 8 to 12, wherein $R^1$ is a bromine atom, a chlorine atom, a methyl group or an ethyl group.

**14.** A compound according to any one of claims 8 to 13, wherein $R^2$ is a lower alkoxy group or a halogen atom.

**15.** A compound according to any one of claims 8 to 14, wherein $X^{10}$ is a hydrogen atom, a lower alkyl group, a hydroxyalkyl group of 4 or less carbon atoms, an alkanoyl group, a hydroxy-substituted alkanoyl group, an alkanesulfonyl group or an alkylsulfamoyl group.

**16.** A serotonin reuptake inhibitor according to claim 1, wherein G represents a formula:

$$-Z^2-X^{20}$$

**17.** A serotonin reuptake inhibitor according to claim 16, wherein the alkylene group for Y is an ethylene group or a trimethylene group.

**18.** A serotonin reuptake inhibitor according to claim 16 or 17, wherein n is 1 or 2.

**19.** A serotonin reuptake inhibitor according to claim 16, 17 or 18, wherein the cycloalkane ring is a cyclohexane ring.

**20.** A serotonin reuptake inhibitor according to claim 19, wherein the substitution positions of the cyclohexane ring are the 1-position and the 4-position.

**21.** A serotonin reuptake inhibitor according to any one of claims 16 to 20, wherein X is an amino group, a substituted amino group, a hydroxyl group, an alkoxy group or a carbamoyloxy group, and the substituent of the aforesaid substituted amino group is a lower alkyl group, a hydroxyalkyl group of 4 or less carbon atoms, an alkanoyl group, a hydroxyalkanoyl group, an alkanesulfonyl group or an alkylsulfamoyl group.

**22.** A cyclic amine represented by the formula:

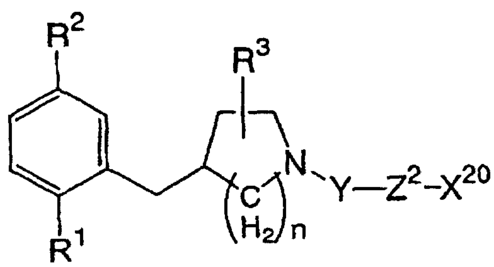

wherein

$R^1$ is a halogen atom or an alkyl group;
$R^2$ is a hydrogen atom, a halogen atom, a hydroxyl group, an alkoxy group, a substituted alkoxy group, an alkylthio group or a substituted alkylthio group;
$R^3$ is a hydrogen atom or a lower alkyl group;
$Z^2$ is a cycloalkane ring;
$X^{20}$ is an alkyl group, a substituted alkyl group, an amino group, a substituted amino group, a hydroxyl group, an alkoxy group, a substituted alkoxy group, a carbamoyloxy group, an alkylcarbamoyloxy group or a substituted alkylcarbamoyloxy group;
Y is a substituted or unsubstituted alkylene group; and
n is an integer of 1, 2 or 3,

a prodrug of said cyclic amine, or a pharmaceutically acceptable salt of said cyclic amine or prodrug.

**23.** A compound according to claim 22, wherein $R^2$ is a hydroxyl group, a lower alkoxy group, a halogen-substituted alkoxy group or a halogen atom, or $R^2$ is a hydrogen atom only in the case of $R^1$ being a bromine atom; and Y is a substituted or unsubstituted alkylene group of 2 or 3 carbon atoms.

**24.** A compound according to claim 22 or 23, wherein n is 1 or 2.

**25.** A compound according to any one of claims 22 to 24, wherein Y is an unsubstituted alkylene group.

**26.** A compound according to any one of claims 22 to 25, wherein Z is a cyclohexane ring.

**27.** A compound according to claim 26, wherein the substitution positions of the cyclohexane ring are the 1-position and the 4-position.

**28.** A compound according to any one of claims 22 to 27, wherein $R^1$ is a bromine atom, a chlorine atom, a methyl group or an ethyl group.

**29.** A compound according to any one of claims 22 to 28, wherein $R^2$ is a lower alkoxy group or a halogen atom.

**30.** A compound according to any one of claims 22 to 29, wherein X is an amino group, a substituted amino group, a hydroxyl group, an alkoxy group or a carbamoyloxy group, and the substituent of the aforesaid substituted amino group is a lower alkyl group, a hydroxyalkyl group of 4 or less carbon atoms, an alkanoyl group, a hydroxyalkanoyl group, an alkanesulfonyl group, an alkylsulfamoyl group or a substituted alkylsulfamoyl group.

**31.** A serotonin reuptake inhibitor according to claim 1, wherein G represents a formula:

$$-Z^3$$

**32.** A serotonin reuptake inhibitor according to claim 31, wherein the alkylene group for Y is a methylene group, an ethylene group or a trimethylene group.

**33.** A serotonin reuptake inhibitor according to claim 31 or 32, wherein n is 1 or 2.

**34.** A serotonin reuptake inhibitor according to claim 31, 32 or 33, wherein $R^2$ is a halogen atom or an alkoxy group.

**35.** A cyclic amine represented by the formula:

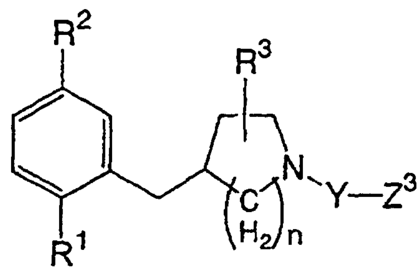

wherein

$R^1$ is a halogen atom or an alkyl group;
$R^2$ is a hydrogen atom, a halogen atom, a hydroxyl group, an alkyl group, a substituted alkyl group, an alkoxy group, a substituted alkoxy group, an alkylthio group or a substituted alkylthio group;
$R^3$ is a hydrogen atom or a lower alkyl group;
Y is a substituted or unsubstituted alkylene group;
$Z^3$ represents a formula:

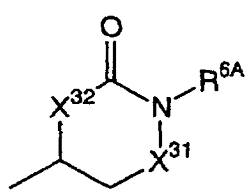

(A')

wherein

$X^{31}$ is a bond, a methylene group or a carbonyl group;
$X^{32}$ is an oxygen atom, a sulfur atom or a group: $-N(R^7)-$;
$R^{6A}$ is a hydrogen atom, an alkyl group, a substituted alkyl group, a cycloalkyl group, a substituted cycloalkyl group, a heteroaryl group or a substituted heteroaryl group;
$R^7$ is a hydrogen atom or an alkyl group, or a formula:

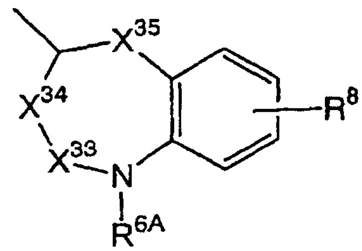

(B')

wherein

$X^{33}$ is a bond, a methylene group or a carbonyl group;
$X^{34}$ is a bond or a methylene group, and $X^{35}$ is a bond, a methylene group, an oxygen atom, a sulfur atom or a group: -N($R^7$)-, provided that $X^{34}$ and $X^{35}$ are not bonds at the same time;
$R^{6A}$ and $R^7$ are as defined above;
$R^8$ is a hydrogen atom, a halogen atom, an alkyl group, a hydroxyl group, an alkoxy group, a substituted alkoxy group, a carbamoyloxy group, an alkylcarbamoyloxy group or a substituted alkylcarbamoyloxy group; and
n is an integer of 1, 2 or 3,

a prodrug of said cyclic amine, or a pharmaceutically acceptable salt of said cyclic amine or prodrug.

**36.** A compound according to claim 35, wherein the alkylene group for Y is a methylene group, an ethylene group or a trimethylene group.

**37.** A compound according to claim 35 or 36, wherein n is 1 or 2.

**38.** A compound according to claim 35, 36 or 37, wherein $R^2$ is a halogen atom or an alkoxy group.

## INTERNATIONAL SEARCH REPORT

International application No.
PCT/JP02/13043

**A. CLASSIFICATION OF SUBJECT MATTER**

Int.Cl$^7$ C07D211/26, 211/22, 401/06, 413/06, A61K31/445, 31/4545, 31/4709, 31/5355, A61P1/00, 3/04, 5/00, 9/12, 15/00, 25/06, 25/16, 25/22, 25/24, 25/28, 25/30, 25/32, 25/34, 43/00

According to International Patent Classification (IPC) or to both national classification and IPC

**B. FIELDS SEARCHED**

Minimum documentation searched (classification system followed by classification symbols)

Int.Cl$^7$ C07D211/26, 211/22, 401/06, 413/06, A61K31/445, 31/4545, 31/4709, 31/5355, A61P1/00, 3/04, 5/00, 9/12, 15/00, 25/06, 25/16, 25/22, 25/24, 25/28, 25/30, 25/32, 25/34, 43/00

Documentation searched other than minimum documentation to the extent that such documents are included in the fields searched

Electronic data base consulted during the international search (name of data base and, where practicable, search terms used)

REGISTRY(STN), CAPLUS(STN), CAOLD(STN)

**C. DOCUMENTS CONSIDERED TO BE RELEVANT**

| Category* | Citation of document, with indication, where appropriate, of the relevant passages | Relevant to claim No. |
|---|---|---|
| X | WO 98/08816 A1 (Meiji Seika Kaisha, Ltd.), 05 March, 1998 (05.03.98), Full text (Family: none) | 35-38 |
| A | JP 2001-131149 A (Sumitomo Pharmaceuticals Co., Ltd.), 15 May, 2001 (15.05.01), (Family: none) | 1-38 |
| A | WO 00/44376 A1 (BRISTOL-MYERS SQUIBB CO.), 03 August, 2000 (03.08.00), & US 6225324 B1 & AU 2712200 A & BR 9916618 A | 1-38 |

[X] Further documents are listed in the continuation of Box C.  [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 March, 2003 (03.03.03) | 18 March, 2003 (18.03.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/13043

C (Continuation). DOCUMENTS CONSIDERED TO BE RELEVANT

| Category* | Citation of document, with indication, where appropriate, of the relevant passages | Relevant to claim No. |
|---|---|---|
| P,A | WO 02/06231 A1 (Sumitomo Pharmaceuticals Co., Ltd.), 24 January, 2002 (24.01.02), (Family: none) | 1-38 |

Form PCT/ISA/210 (continuation of second sheet) (July 1998)